# EUROPEAN PATENT APPLICATION

(11) **EP 1 566 444 A2**
(43) Date of publication of application: **24.08.2005**
(21) Application number: 05007595.1
(22) Date of filing: 14.11.2000
(51) Int. Cl.: C12N 15/82, C07K 14/415, A01H 5/00

(54) **Yield-related genes**

(30) Priority: 17.11.1999 US 166228 P; 17.04.2000 US 197899 P; 22.08.2000 US 227439 P
(62) Divisional of application: 00980406.3
(71) Applicant: Mendel Biotechnology, Inc., Hayward, CA 94541 (US)
(72) Inventor: Jiang, Cai-Zhong, Fremont, CA 94555 (US); Heard, Jacqueline, San Mateo, CA 94402 (US); Pineda, Omaira, Vero Beach, FL 32960 (US); Pilgrim, Marsha, Fremont, CA 94356 (US); Adam, Luc, Hayward, CA 94545 (US); Riechmann, Jose Luis, Pasadena, CA 91101 (US); Yu, Guo-Liang, Berkeley, CA 94705 (US); Samaha, Raymond, Capitola, CA 95010 (US)
(74) Representative: von Kreisler Selting Werner

(57) **Abstract**

Recombinant polynucleotides and methods for modifying the phenotype of a plant are provided. In particular, the phenotype that is being modified is a plant's sugar-sensing characteristics.

SEQUENCE LISTING

<110> Cai-Zhong, Jiang
      Jacqueline, Heard
      Omaira, Pineda
      Pilgrim, Marsha
      Adam, Luc
      Riechmann, Jose Luis
      Yu, Guo-Liang
      Samaha, Raymond

<120> Yield-related genes

<130> MBI-0019

<150> 60/166,228
<151> 1999-11-17

<150> 60/197,899
<151> 2000-04-17

<150> Plant Trait Modification III
<151> 2000-08-22

<160> 70

<170> PatentIn version 3.0

<210> 1
<211> 1084
<212> DNA
<213> Arabidopsis thaliana

<220>
<221> CDS
<222> (73)..(729)
<223> G26

<400> 1
ttggcttgta cccaaaccca tctttgactt caaaaataaa ataaaaataa tcataattga      60

catcatcgga ta atg cat agc ggg aag aga cct cta tca cca gaa tca atg      111
              Met His Ser Gly Lys Arg Pro Leu Ser Pro Glu Ser Met
              1               5                   10

gcc gga aat aga gaa gag aaa aaa gag ttg tgt tgt tgc tca act ttg      159
Ala Gly Asn Arg Glu Glu Lys Lys Glu Leu Cys Cys Cys Ser Thr Leu
    15                  20                  25

tcg gaa tct gat gtg tct gat ttt gtc tct gaa ctc act ggt caa ccc      207
Ser Glu Ser Asp Val Ser Asp Phe Val Ser Glu Leu Thr Gly Gln Pro
30                  35                  40                  45

atc cca tca tcc att gat gat caa tct tcg tcg ctt act ctt caa gaa      255
Ile Pro Ser Ser Ile Asp Asp Gln Ser Ser Ser Leu Thr Leu Gln Glu
                50                  55                  60

aaa agt aac tcg agg caa cga aac tac aga ggc gtg agg caa aga ccg      303
Lys Ser Asn Ser Arg Gln Arg Asn Tyr Arg Gly Val Arg Gln Arg Pro
            65                  70                  75

tgg gga aaa tgg gcg gct gag att cgt gac ccg aac aag gca gct cgt      351
Trp Gly Lys Trp Ala Ala Glu Ile Arg Asp Pro Asn Lys Ala Ala Arg
        80                  85                  90

gtg tgg ctt ggg acg ttc gac act gca gaa gaa gcc gcc tta gcg tat      399
Val Trp Leu Gly Thr Phe Asp Thr Ala Glu Glu Ala Ala Leu Ala Tyr
    95                  100                 105

gat aaa gct gca ttt gag ttt aga ggt cac aag gcc aag ctt aac ttc      447
Asp Lys Ala Ala Phe Glu Phe Arg Gly His Lys Ala Lys Leu Asn Phe
110                 115                 120                 125

ccc gag cat att cgt gtc aac cct act caa ctc tat cca tcg ccc gct      495

Pro Glu His Ile Arg Val Asn Pro Thr Gln Leu Tyr Pro Ser Pro Ala
                130                 135                 140

act tcc cat gat cgc att atc gtg aca cca cct agt cca cct cca cca 543
Thr Ser His Asp Arg Ile Ile Val Thr Pro Pro Ser Pro Pro Pro Pro
            145                 150                 155

att gct cct gac ata ctt ctt gat caa tat ggc cac ttt caa tct cga 591
Ile Ala Pro Asp Ile Leu Leu Asp Gln Tyr Gly His Phe Gln Ser Arg
        160                 165                 170

agt agt gat tcc agt gcc aac ttg tcc atg aat atg ctg tct tct tcg 639
Ser Ser Asp Ser Ser Ala Asn Leu Ser Met Asn Met Leu Ser Ser Ser
    175                 180                 185

tct tca tct ttg aat cat caa ggg cta aga cca aat ttg gag gat ggt 687
Ser Ser Ser Leu Asn His Gln Gly Leu Arg Pro Asn Leu Glu Asp Gly
190                 195                 200                 205

gaa aac gtg aag aac att agt atc cac aaa cga cga aaa taa 729
Glu Asn Val Lys Asn Ile Ser Ile His Lys Arg Arg Lys
                210                 215

catgttaatg gcataaatat ctcttcgtcc aagttatcaa acgcattgac ctccggcttt 789

gatcatttta ggcgcttaat ctctttacga cttcattttg gtagtcttta aagagtctat 849

ggagtggatt tagctaggaa tcaggcctta tggatgaaaa atatataaat tttgaacatg 909

actatgcaag aatgggatga agactactta gcttggaaaa cgtcctgata ggtcatgacg 969

actatatcca cagaagatga ccgacggaga caacaacatg cctcacctga tcgaccgatc 1029

aaatgagata atgtgttgac cggaccggtc ggatcaggtt gggtcgagta tatca 1084

<210> 2
<211> 218
<212> PRT
<213> Arabidopsis thaliana

<400> 2
   Met His Ser Gly Lys Arg Pro Leu Ser Pro Glu Ser Met Ala Gly Asn
   1 5 10 15
   Arg Glu Glu Lys Lys Glu Leu Cys Cys Cys Ser Thr Leu Ser Glu Ser
   20 25 30
   Asp Val Ser Asp Phe Val Ser Glu Leu Thr Gly Gln Pro Ile Pro Ser
   35 40 45
   Ser Ile Asp Asp Gln Ser Ser Ser Leu Thr Leu Gln Glu Lys Ser Asn
   50 55 60
   Ser Arg Gln Arg Asn Tyr Arg Gly Val Arg Gln Arg Pro Trp Gly Lys
   65 70 75 80
   Trp Ala Ala Glu Ile Arg Asp Pro Asn Lys Ala Ala Arg Val Trp Leu
   85 90 95
   Gly Thr Phe Asp Thr Ala Glu Glu Ala Ala Leu Ala Tyr Asp Lys Ala
   100 105 110
   Ala Phe Glu Phe Arg Gly His Lys Ala Lys Leu Asn Phe Pro Glu His
   115 120 125
   Ile Arg Val Asn Pro Thr Gln Leu Tyr Pro Ser Pro Ala Thr Ser His
       130                 135                 140
   Asp Arg Ile Ile Val Thr Pro Pro Ser Pro Pro Pro Pro Ile Ala Pro
   145                 150                 155                 160
   Asp Ile Leu Leu Asp Gln Tyr Gly His Phe Gln Ser Arg Ser Ser Asp
                   165                 170                 175
   Ser Ser Ala Asn Leu Ser Met Asn Met Leu Ser Ser Ser Ser Ser Ser
               180                 185                 190
   Leu Asn His Gln Gly Leu Arg Pro Asn Leu Glu Asp Gly Glu Asn Val
           195                 200                 205
   Lys Asn Ile Ser Ile His Lys Arg Arg Lys
       210                 215

<210>  3
<211>  1440
<212>  DNA
<213>  Arabidopsis thaliana

<220>
<221>  CDS
<222>  (149)..(1156)
<223>  G38

<400>  3
gaggaaaact cgaaaaagct acacacaaga agaagaagaa aagatacgag caagaagact      60

aaacacgaaa gcgatttatc aactcgaagg aagagacttt gattttcaaa tttcgtcccc     120

tatagattgt gttgtttctg ggaaggag atg gca gtt tat gat cag agt gga       172
                               Met Ala Val Tyr Asp Gln Ser Gly
                               1               5

gat aga aac aga aca caa att gat aca tcg agg aaa agg aaa tct aga       220
Asp Arg Asn Arg Thr Gln Ile Asp Thr Ser Arg Lys Arg Lys Ser Arg
    10                  15                  20

agt aga ggt gac ggt act act gtg gct gag aga tta aag aga tgg aaa       268
Ser Arg Gly Asp Gly Thr Thr Val Ala Glu Arg Leu Lys Arg Trp Lys
25                  30                  35                  40

gag tat aac gag acc gta gaa gaa gtt tct acc aag aag agg aaa gta       316
Glu Tyr Asn Glu Thr Val Glu Glu Val Ser Thr Lys Lys Arg Lys Val
                45                  50                  55

cct gcg aaa ggg tcg aag aag ggt tgt atg aaa ggt aaa gga gga cca       364
Pro Ala Lys Gly Ser Lys Lys Gly Cys Met Lys Gly Lys Gly Gly Pro
            60                  65                  70

gag aat agc cga tgt agt ttc aga gga gtt agg caa agg att tgg ggt       412
Glu Asn Ser Arg Cys Ser Phe Arg Gly Val Arg Gln Arg Ile Trp Gly
        75                  80                  85

aaa tgg gtt gct gag atc aga gag cct aat cga ggt agc agg ctt tgg       460
Lys Trp Val Ala Glu Ile Arg Glu Pro Asn Arg Gly Ser Arg Leu Trp
    90                  95                  100

ctt ggt act ttc cct act gct caa gaa gct gct tct gct tat gat gag       508
Leu Gly Thr Phe Pro Thr Ala Gln Glu Ala Ala Ser Ala Tyr Asp Glu
105                 110                 115                 120

gct gct aaa gct atg tat ggt cct ttg gct cgt ctt aat ttc cct cgg       556
Ala Ala Lys Ala Met Tyr Gly Pro Leu Ala Arg Leu Asn Phe Pro Arg
                125                 130                 135

tct gat gcg tct gag gtt acg agt acc tca agt cag tct gag gtg tgt 604
Ser Asp Ala Ser Glu Val Thr Ser Thr Ser Ser Gln Ser Glu Val Cys
            140                 145                 150

act gtt gag act cct ggt tgt gtt cat gtg aaa aca gag gat cca gat 652
Thr Val Glu Thr Pro Gly Cys Val His Val Lys Thr Glu Asp Pro Asp
        155                 160                 165

tgt gaa tct aaa ccc ttc tcc ggt gga gtg gag ccg atg tat tgt ctg 700
Cys Glu Ser Lys Pro Phe Ser Gly Gly Val Glu Pro Met Tyr Cys Leu
    170                 175                 180

gag aat ggt gcg gaa gag atg aag aga ggt gtt aaa gcg gat aag cat 748
Glu Asn Gly Ala Glu Glu Met Lys Arg Gly Val Lys Ala Asp Lys His
185                 190                 195                 200

tgg ctg agc gag ttt gaa cat aac tat tgg agt gat att ctg aaa gag 796
Trp Leu Ser Glu Phe Glu His Asn Tyr Trp Ser Asp Ile Leu Lys Glu
                205                 210                 215

aaa gag aaa cag aag gag caa ggg att gta gaa acc tgt cag caa caa 844
Lys Glu Lys Gln Lys Glu Gln Gly Ile Val Glu Thr Cys Gln Gln Gln
            220                 225                 230

cag cag gat tcg cta tct gtt gca gac tat ggt tgg ccc aat gat gtg 892
Gln Gln Asp Ser Leu Ser Val Ala Asp Tyr Gly Trp Pro Asn Asp Val
        235                 240                 245

gat cag agt cac ttg gat tct tca gac atg ttt gat gtc gat gag ctt 940
Asp Gln Ser His Leu Asp Ser Ser Asp Met Phe Asp Val Asp Glu Leu
    250                 255                 260

cta cgt gac cta aat ggc gac gat gtg ttt gca ggc tta aat cag gac 988
Leu Arg Asp Leu Asn Gly Asp Asp Val Phe Ala Gly Leu Asn Gln Asp
265                 270                 275                 280

cgg tac ccg ggg aac agt gtt gcc aac ggt tca tac agg ccc gag agt 1036
Arg Tyr Pro Gly Asn Ser Val Ala Asn Gly Ser Tyr Arg Pro Glu Ser
                285                 290                 295

caa caa agt ggt ttt gat ccg cta caa agc ctc aac tac gga ata cct 1084
Gln Gln Ser Gly Phe Asp Pro Leu Gln Ser Leu Asn Tyr Gly Ile Pro
            300                 305                 310

ccg ttt cag ctc gag gga aag gat ggt aat gga ttc ttc gac gac ttg 1132
Pro Phe Gln Leu Glu Gly Lys Asp Gly Asn Gly Phe Phe Asp Asp Leu
        315                 320                 325

agt tac ttg gat ctg gag aac taa acaaaacaat atgaagcttt ttggatttga 1186
Ser Tyr Leu Asp Leu Glu Asn
    330                 335

tatttgcctt aatcccacaa cgactgttga ttctctatcc gagttttagt gatatagaga 1246

actacagaac acgtttttc ttgttataaa ggtgaactgt atatatcgaa acagtgatat 1306

gacaatagag aagacaacta tagtttgtta gtctgcttct cttaagttgt tctttagata 1366

tgttttatgt tttgtaacaa caggaatgaa taatacacac ttgtgaagct tttaaaaaaa 1426

aaaaaaaaaa aaaa 1440

<210> 4
<211> 335
<212> PRT
<213> Arabidopsis thaliana

<400> 4
   Met Ala Val Tyr Asp Gln Ser Gly Asp Arg Asn Arg Thr Gln Ile Asp
   1 5 10 15
   Thr Ser Arg Lys Arg Lys Ser Arg Ser Arg Gly Asp Gly Thr Thr Val
   20 25 30
   Ala Glu Arg Leu Lys Arg Trp Lys Glu Tyr Asn Glu Thr Val Glu Glu
   35 40 45
   Val Ser Thr Lys Lys Arg Lys Val Pro Ala Lys Gly Ser Lys Lys Gly
   50 55 60
   Cys Met Lys Gly Lys Gly Gly Pro Glu Asn Ser Arg Cys Ser Phe Arg
   65 70 75 80
   Gly Val Arg Gln Arg Ile Trp Gly Lys Trp Val Ala Glu Ile Arg Glu
   85 90 95
   Pro Asn Arg Gly Ser Arg Leu Trp Leu Gly Thr Phe Pro Thr Ala Gln
   100 105 110
   Glu Ala Ala Ser Ala Tyr Asp Glu Ala Ala Lys Ala Met Tyr Gly Pro
   115 120 125
   Leu Ala Arg Leu Asn Phe Pro Arg Ser Asp Ala Ser Glu Val Thr Ser
   130 135 140
   Thr Ser Ser Gln Ser Glu Val Cys Thr Val Glu Thr Pro Gly Cys Val
   145 150 155 160
   His Val Lys Thr Glu Asp Pro Asp Cys Glu Ser Lys Pro Phe Ser Gly
   165 170 175
   Gly Val Glu Pro Met Tyr Cys Leu Glu Asn Gly Ala Glu Glu Met Lys
   180 185 190
   Arg Gly Val Lys Ala Asp Lys His Trp Leu Ser Glu Phe Glu His Asn
   195 200 205
   Tyr Trp Ser Asp Ile Leu Lys Glu Lys Glu Lys Gln Lys Glu Gln Gly
   210 215 220
   Ile Val Glu Thr Cys Gln Gln Gln Gln Gln Asp Ser Leu Ser Val Ala
   225 230 235 240
   Asp Tyr Gly Trp Pro Asn Asp Val Asp Gln Ser His Leu Asp Ser Ser
   245 250 255
   Asp Met Phe Asp Val Asp Glu Leu Leu Arg Asp Leu Asn Gly Asp Asp
   260 265 270
   Val Phe Ala Gly Leu Asn Gln Asp Arg Tyr Pro Gly Asn Ser Val Ala
   275 280 285
   Asn Gly Ser Tyr Arg Pro Glu Ser Gln Gln Ser Gly Phe Asp Pro Leu
   290 295 300
   Gln Ser Leu Asn Tyr Gly Ile Pro Pro Phe Gln Leu Glu Gly Lys Asp
   305 310 315 320
   Gly Asn Gly Phe Phe Asp Asp Leu Ser Tyr Leu Asp Leu Glu Asn
   325 330 335

<210>  5
<211>  909
<212>  DNA
<213>  Arabidopsis thaliana

<220>
<221>  CDS
<222>  (38)..(643)
<223>  G43

<400>  5
ctcctgtctt gtctaaagaa aaaagagaga ggaagaa atg gag act ttt gag gaa       55
                                         Met Glu Thr Phe Glu Glu
                                         1               5

agc tct gat ttg gat gtt ata cag aaa cat cta ttt gaa gac ttg atg       103
Ser Ser Asp Leu Asp Val Ile Gln Lys His Leu Phe Glu Asp Leu Met
            10                  15                  20

atc cct gat ggt ttc att gaa gat ttt gtc ttt gat gat act gct ttt       151
Ile Pro Asp Gly Phe Ile Glu Asp Phe Val Phe Asp Asp Thr Ala Phe
        25                  30                  35

gtc tcc gga ctc tgg tct cta gaa ccc ttt aac cca gtt ccg aaa ctg       199
Val Ser Gly Leu Trp Ser Leu Glu Pro Phe Asn Pro Val Pro Lys Leu
    40                  45                  50

gaa cct agt tca cct gtt ctt gat cca gat tcc tat gtc caa gag att       247
Glu Pro Ser Ser Pro Val Leu Asp Pro Asp Ser Tyr Val Gln Glu Ile
55                  60                  65                  70

ctg caa atg gaa gca gaa tca tca tca tca tca tca aca aca acg tca       295
Leu Gln Met Glu Ala Glu Ser Ser Ser Ser Ser Ser Thr Thr Thr Ser
                75                  80                  85

cct gag gtt gag act gtc tca aac cgg aaa aaa aca aag agg ttt gaa       343
Pro Glu Val Glu Thr Val Ser Asn Arg Lys Lys Thr Lys Arg Phe Glu
            90                  95                  100

gaa acg aga cat tac aga ggc gtg aga agg agg cca tgg ggg aaa ttt       391
Glu Thr Arg His Tyr Arg Gly Val Arg Arg Arg Pro Trp Gly Lys Phe
        105                 110                 115

gca gca gag att cga gat ccg gca aag aaa gga tcc agg att tgg tta       439
Ala Ala Glu Ile Arg Asp Pro Ala Lys Lys Gly Ser Arg Ile Trp Leu
    120                 125                 130

ggc act ttt gag agt gat att gat gct gca agg gct tac gac tat gca       487
Gly Thr Phe Glu Ser Asp Ile Asp Ala Ala Arg Ala Tyr Asp Tyr Ala
135                 140                 145                 150

gct ttt aag ctc agg gga aga aaa gct gtt ctc aac ttt cct ttg gat       535
Ala Phe Lys Leu Arg Gly Arg Lys Ala Val Leu Asn Phe Pro Leu Asp
                155                 160                 165

gcc gga aag tat gat gct ccg gtc aat tca tgc cga aaa agg agg aga       583
Ala Gly Lys Tyr Asp Ala Pro Val Asn Ser Cys Arg Lys Arg Arg Arg
            170                 175                 180

acc gat gta cca cag cct caa gga aca aca aca agt act tca tca tcg       631
Thr Asp Val Pro Gln Pro Gln Gly Thr Thr Thr Ser Thr Ser Ser Ser
        185                 190                 195

tca tca aac taa tgggggaata gtgatgttta attagtatat ataggttaat           683
Ser Ser Asn
    200

atcttaagta tgtgaagcat catgtataga gccaagaacc tgttagacta gtgtactgaa     743

aagaactctt gcaaaatatg tactaaagag ttcctgtaac aatggaactt ctgcgttttc 803

tcttgtctta aagagcttaa ggttctagaa acaaagttct tgtcctttcg gtttaaaaaa 863

aaaaaaaaaa aaaaaaaaaa aaaaaaaaaa aaaaaaaaaa aaaaaa 909

<210> 6
<211> 201
<212> PRT
<213> Arabidopsis thaliana

<400> 6

Met Glu Thr Phe Glu Glu Ser Ser Asp Leu Asp Val Ile Gln Lys His
1               5                   10                  15

Leu Phe Glu Asp Leu Met Ile Pro Asp Gly Phe Ile Glu Asp Phe Val
            20                  25                  30

Phe Asp Asp Thr Ala Phe Val Ser Gly Leu Trp Ser Leu Glu Pro Phe
        35                  40                  45

Asn Pro Val Pro Lys Leu Glu Pro Ser Ser Pro Val Leu Asp Pro Asp
    50                  55                  60

Ser Tyr Val Gln Glu Ile Leu Gln Met Glu Ala Glu Ser Ser Ser Ser
65                  70                  75                  80

Ser Ser Thr Thr Thr Ser Pro Glu Val Glu Thr Val Ser Asn Arg Lys
                85                  90                  95

Lys Thr Lys Arg Phe Glu Glu Thr Arg His Tyr Arg Gly Val Arg Arg
            100                 105                 110

Arg Pro Trp Gly Lys Phe Ala Ala Glu Ile Arg Asp Pro Ala Lys Lys
        115                 120                 125

Gly Ser Arg Ile Trp Leu Gly Thr Phe Glu Ser Asp Ile Asp Ala Ala
    130                 135                 140

Arg Ala Tyr Asp Tyr Ala Ala Phe Lys Leu Arg Gly Arg Lys Ala Val
145                 150                 155                 160

Leu Asn Phe Pro Leu Asp Ala Gly Lys Tyr Asp Ala Pro Val Asn Ser
                165                 170                 175

Cys Arg Lys Arg Arg Arg Thr Asp Val Pro Gln Pro Gln Gly Thr Thr
            180                 185                 190

Thr Ser Thr Ser Ser Ser Ser Ser Asn
        195                 200

<210> 7
<211> 1107
<212> DNA
<213> Arabidopsis thaliana

<220>
<221> CDS
<222> (16)..(930)
<223> G207

<400> 7
   aaaagatctg tttca atg gcg gat cgt gtt aaa ggt cca tgg agt caa gaa 51
   Met Ala Asp Arg Val Lys Gly Pro Trp Ser Gln Glu
   1 5 10
   gaa gat gag cag cta cga agg atg gtt gag aaa tac gga ccg agg aat 99
   Glu Asp Glu Gln Leu Arg Arg Met Val Glu Lys Tyr Gly Pro Arg Asn
   15 20 25
   tgg tct gcg att agc aaa tcg att cca ggt cga tct ggt aaa tcg tgt 147
   Trp Ser Ala Ile Ser Lys Ser Ile Pro Gly Arg Ser Gly Lys Ser Cys
   30 35 40
   aga tta cgt tgg tgt aat cag tta tct ccg gag gtt gag cat cgt cct 195
   Arg Leu Arg Trp Cys Asn Gln Leu Ser Pro Glu Val Glu His Arg Pro
   45 50 55 60
   ttc tcg ccg gag gaa gat gag act att gta acc gcc cgt gct cag ttt 243
   Phe Ser Pro Glu Glu Asp Glu Thr Ile Val Thr Ala Arg Ala Gln Phe
   65 70 75
   ggt aac aag tgg gcg acg att gct cgt ctt ctt aac ggt cgt acg gat 291
   Gly Asn Lys Trp Ala Thr Ile Ala Arg Leu Leu Asn Gly Arg Thr Asp
   80 85 90
   aac gcc gtt aaa aat cac tgg aac tct acg ctt aag agg aaa tgc agc 339
   Asn Ala Val Lys Asn His Trp Asn Ser Thr Leu Lys Arg Lys Cys Ser
   95 100 105
   gga ggt gtg gcg gtt acg acg gtg acg gag acg gag gaa gat cag gat 387
   Gly Gly Val Ala Val Thr Thr Val Thr Glu Thr Glu Glu Asp Gln Asp
   110 115 120
   cgg ccg aag aag agg aga tct gtt agc ttt gat cct gct ttt gct ccg 435
   Arg Pro Lys Lys Arg Arg Ser Val Ser Phe Asp Pro Ala Phe Ala Pro
   125 130 135 140
   gtg gat act gga ttg tac atg agt cct gag agt cct aac gga atc gat 483
   Val Asp Thr Gly Leu Tyr Met Ser Pro Glu Ser Pro Asn Gly Ile Asp
   145 150 155
   gtt agt gat tct agc acg att ccg tca ccg tcg tct cct gtt gct cag 531
   Val Ser Asp Ser Ser Thr Ile Pro Ser Pro Ser Ser Pro Val Ala Gln
   160 165 170
   ctg ttt aaa cca atg ccg att tcc ggc ggt ttt acg gtg gtt ccg cag 579
   Leu Phe Lys Pro Met Pro Ile Ser Gly Gly Phe Thr Val Val Pro Gln
   175 180 185
   ccg tta ccg gtt gaa atg tct tcg tct tcg gag gat cca cct act tcg 627
   Pro Leu Pro Val Glu Met Ser Ser Ser Ser Glu Asp Pro Pro Thr Ser
   190 195 200
   ttg agt ttg tca cta cct gga gct gag aac acg agt tcg agc cat aac 675
   Leu Ser Leu Ser Leu Pro Gly Ala Glu Asn Thr Ser Ser Ser His Asn
   205 210 215 220
   aat aac aac aac gcg ttg atg ttt ccg aga ttt gag agt cag atg aag 723
   Asn Asn Asn Asn Ala Leu Met Phe Pro Arg Phe Glu Ser Gln Met Lys
   225 230 235
   att aat gta gag gag aga gga gga gga gga gaa gga cgt aga ggt gag 771
   Ile Asn Val Glu Glu Arg Gly Gly Gly Gly Glu Gly Arg Arg Gly Glu
   240 245 250
   ttt atg acg gtg gtg cag gag atg ata aaa gct gaa gtg agg agt tac 819
   Phe Met Thr Val Val Gln Glu Met Ile Lys Ala Glu Val Arg Ser Tyr
   255 260 265
   atg gcg gaa atg cag aaa aca agt ggt gga ttc gtc gtc gga ggt tta 867
   Met Ala Glu Met Gln Lys Thr Ser Gly Gly Phe Val Val Gly Gly Leu
   270 275 280
   tac gaa tcc ggc ggc aat ggt ggt ttt agg gat tgt gga gta ata aca 915
   Tyr Glu Ser Gly Gly Asn Gly Gly Phe Arg Asp Cys Gly Val Ile Thr
   285 290 295 300
   cct aag gtt gag tag ttttggttta gggttaaaac ttgaatcgat tggggatttt 970
   Pro Lys Val Glu
   caagagcatt catttttggg gtttatggta aaattaaaaa caaaaacaaa atgtacagag 1030
   gaattaaaat ttctatggaa taatcttaaa tctcaaatat ttgttacttg ttttggtgat 1090
   tcataaccaa aatcaaa 1107

<210> 8
<211> 304
<212> PRT
<213> Arabidopsis thaliana

<400> 8

Met Ala Asp Arg Val Lys Gly Pro Trp Ser Gln Glu Glu Asp Glu Gln
1               5                   10                  15

Leu Arg Arg Met Val Glu Lys Tyr Gly Pro Arg Asn Trp Ser Ala Ile
            20                  25                  30

Ser Lys Ser Ile Pro Gly Arg Ser Gly Lys Ser Cys Arg Leu Arg Trp
        35                  40                  45

Cys Asn Gln Leu Ser Pro Glu Val Glu His Arg Pro Phe Ser Pro Glu
    50                  55                  60

Glu Asp Glu Thr Ile Val Thr Ala Arg Ala Gln Phe Gly Asn Lys Trp
65                  70                  75                  80

Ala Thr Ile Ala Arg Leu Leu Asn Gly Arg Thr Asp Asn Ala Val Lys
                85                  90                  95

Asn His Trp Asn Ser Thr Leu Lys Arg Lys Cys Ser Gly Gly Val Ala
            100                 105                 110

Val Thr Thr Val Thr Glu Thr Glu Glu Asp Gln Asp Arg Pro Lys Lys
        115                 120                 125

Arg Arg Ser Val Ser Phe Asp Pro Ala Phe Ala Pro Val Asp Thr Gly
    130                 135                 140

Leu Tyr Met Ser Pro Glu Ser Pro Asn Gly Ile Asp Val Ser Asp Ser
145                 150                 155                 160

Ser Thr Ile Pro Ser Pro Ser Ser Pro Val Ala Gln Leu Phe Lys Pro
                165                 170                 175

Met Pro Ile Ser Gly Gly Phe Thr Val Val Pro Gln Pro Leu Pro Val
            180                 185                 190

Glu Met Ser Ser Ser Ser Glu Asp Pro Pro Thr Ser Leu Ser Leu Ser
        195                 200                 205

Leu Pro Gly Ala Glu Asn Thr Ser Ser Ser His Asn Asn Asn Asn Asn

210                 215                 220

Ala Leu Met Phe Pro Arg Phe Glu Ser Gln Met Lys Ile Asn Val Glu
225                 230                 235                 240

Glu Arg Gly Gly Gly Gly Glu Gly Arg Arg Gly Glu Phe Met Thr Val
                245                 250                 255

Val Gln Glu Met Ile Lys Ala Glu Val Arg Ser Tyr Met Ala Glu Met
            260                 265                 270

Gln Lys Thr Ser Gly Gly Phe Val Val Gly Gly Leu Tyr Glu Ser Gly
        275                 280                 285

Gly Asn Gly Gly Phe Arg Asp Cys Gly Val Ile Thr Pro Lys Val Glu
    290                 295                 300

<210>  9
<211>  1046
<212>  DNA
<213>  Arabidopsis thaliana

<220>
<221>  CDS
<222>  (46)..(867)
<223>  G241

<400>  9
gaaaaacatt tcaacttctt ttatcagcaa tcacaaatca aagag atg gga aga gct      57
                                                  Met Gly Arg Ala
                                                  1

cca tgc tgt gag aag atg ggg ttg aag aga gga cca tgg aca cct gaa      105
Pro Cys Cys Glu Lys Met Gly Leu Lys Arg Gly Pro Trp Thr Pro Glu
5                   10                  15                  20

gaa gat caa atc ttg gtc tct ttt atc ctc aac cat gga cat agt aac      153
Glu Asp Gln Ile Leu Val Ser Phe Ile Leu Asn His Gly His Ser Asn
                25                  30                  35

tgg cga gcc ctc cct aag caa gct ggt ctt ttg aga tgt gga aaa agc      201
Trp Arg Ala Leu Pro Lys Gln Ala Gly Leu Leu Arg Cys Gly Lys Ser
            40                  45                  50

tgt aga ctt agg tgg atg aac tat tta aag cct gat att aaa cgt ggc      249
Cys Arg Leu Arg Trp Met Asn Tyr Leu Lys Pro Asp Ile Lys Arg Gly
        55                  60                  65

aat ttc acc aaa gaa gag gaa gat gct atc atc agc tta cac caa ata      297
Asn Phe Thr Lys Glu Glu Glu Asp Ala Ile Ile Ser Leu His Gln Ile
    70                  75                  80

ctt ggc aat aga tgg tca gcg att gca gca aaa ctg cct gga aga acc      345
Leu Gly Asn Arg Trp Ser Ala Ile Ala Ala Lys Leu Pro Gly Arg Thr
85                  90                  95                  100

gat aac gag atc aag aac gta tgg cac act cac ttg aag aag aga ctc      393
Asp Asn Glu Ile Lys Asn Val Trp His Thr His Leu Lys Lys Arg Leu
                105                 110                 115

gaa gat tat caa cca gct aaa cct aag acc agc aac aaa aag aag ggt      441
Glu Asp Tyr Gln Pro Ala Lys Pro Lys Thr Ser Asn Lys Lys Lys Gly
            120                 125                 130

act aaa cca aaa tct gaa tcc gta ata acg agc tcg aac agt act aga      489
Thr Lys Pro Lys Ser Glu Ser Val Ile Thr Ser Ser Asn Ser Thr Arg
        135                 140                 145

agc gaa tcg gag cta gca gat tca tca aac cct tct gga gaa agc tta 537
Ser Glu Ser Glu Leu Ala Asp Ser Ser Asn Pro Ser Gly Glu Ser Leu
    150                 155                 160

ttt tcg aca tcg cct tcg aca agt gag gtt tct tcg atg aca ctc ata 585
Phe Ser Thr Ser Pro Ser Thr Ser Glu Val Ser Ser Met Thr Leu Ile
165                 170                 175                 180

agc cac gac ggc tat agc aac gag att aat atg gat aac aaa ccg gga 633
Ser His Asp Gly Tyr Ser Asn Glu Ile Asn Met Asp Asn Lys Pro Gly
                185                 190                 195

gat atc agt act atc gat caa gaa tgt gtt tct ttc gaa act ttt ggt 681
Asp Ile Ser Thr Ile Asp Gln Glu Cys Val Ser Phe Glu Thr Phe Gly
            200                 205                 210

gcg gat atc gat gaa agc ttc tgg aaa gag aca ctg tat agc caa gat 729
Ala Asp Ile Asp Glu Ser Phe Trp Lys Glu Thr Leu Tyr Ser Gln Asp
        215                 220                 225

gaa cac aac tac gta tcg aat gac cta gaa gtc gct ggt tta gtt gag 777
Glu His Asn Tyr Val Ser Asn Asp Leu Glu Val Ala Gly Leu Val Glu
    230                 235                 240

ata caa caa gag ttt caa aac ttg ggc tcc gct aat aat gag atg att 825
Ile Gln Gln Glu Phe Gln Asn Leu Gly Ser Ala Asn Asn Glu Met Ile
245                 250                 255                 260

ttt gac agt gag atg gaa ctt ctg gtt cga tgt att ggc tag 867
Phe Asp Ser Glu Met Glu Leu Leu Val Arg Cys Ile Gly
                265                 270

aaccggcggg gaacaagatc tcttagccgg gctctagtta acatgtttga ggagtaaagt 927

gaaatggtgc aaattagtta aggctaagaa attcaaaagc ttttgtttac cgagaaaaaa 987

acacactcta actcttgatg tgatgtagtt agtgtattaa ttagaggctg cgttttcaa 1046

<210> 10
<211> 273
<212> PRT
<213> Arabidopsis thaliana

<400> 10

Met Gly Arg Ala Pro Cys Cys Glu Lys Met Gly Leu Lys Arg Gly Pro
1               5                   10                  15

Trp Thr Pro Glu Glu Asp Gln Ile Leu Val Ser Phe Ile Leu Asn His
            20                  25                  30

Gly His Ser Asn Trp Arg Ala Leu Pro Lys Gln Ala Gly Leu Leu Arg
        35                  40                  45

Cys Gly Lys Ser Cys Arg Leu Arg Trp Met Asn Tyr Leu Lys Pro Asp
    50                  55                  60

Ile Lys Arg Gly Asn Phe Thr Lys Glu Glu Glu Asp Ala Ile Ile Ser
65                  70                  75                  80

Leu His Gln Ile Leu Gly Asn Arg Trp Ser Ala Ile Ala Ala Lys Leu
                85                  90                  95

Pro Gly Arg Thr Asp Asn Glu Ile Lys Asn Val Trp His Thr His Leu
            100                 105                 110

Lys Lys Arg Leu Glu Asp Tyr Gln Pro Ala Lys Pro Lys Thr Ser Asn

115                 120                 125

Lys Lys Lys Gly Thr Lys Pro Lys Ser Glu Ser Val Ile Thr Ser Ser
    130                 135                 140

Asn Ser Thr Arg Ser Glu Ser Glu Leu Ala Asp Ser Ser Asn Pro Ser
145                 150                 155                 160

Gly Glu Ser Leu Phe Ser Thr Ser Pro Ser Thr Ser Glu Val Ser Ser
                165                 170                 175

Met Thr Leu Ile Ser His Asp Gly Tyr Ser Asn Glu Ile Asn Met Asp
            180                 185                 190

Asn Lys Pro Gly Asp Ile Ser Thr Ile Asp Gln Glu Cys Val Ser Phe
        195                 200                 205

Glu Thr Phe Gly Ala Asp Ile Asp Glu Ser Phe Trp Lys Glu Thr Leu
    210                 215                 220

Tyr Ser Gln Asp Glu His Asn Tyr Val Ser Asn Asp Leu Glu Val Ala
225                 230                 235                 240

Gly Leu Val Glu Ile Gln Gln Glu Phe Gln Asn Leu Gly Ser Ala Asn
                245                 250                 255

Asn Glu Met Ile Phe Asp Ser Glu Met Glu Leu Leu Val Arg Cys Ile
            260                 265                 270

Gly


<210>  11
<211>  1391
<212>  DNA
<213>  Arabidopsis thaliana

<220>
<221>  CDS
<222>  (15)..(923)
<223>  G254


<400>  11
cgatttcgag ctct atg gtg tcc gta aac cct aga cct aag ggt ttt cca      50
                Met Val Ser Val Asn Pro Arg Pro Lys Gly Phe Pro
                1               5                   10

gtt ttc gat tcc tcg aat atg agt tta cca agc tcc gat gga ttt ggt      98
Val Phe Asp Ser Ser Asn Met Ser Leu Pro Ser Ser Asp Gly Phe Gly
        15                  20                  25

tcg att ccg gcc acg gga cgg acc agt acg gtg tcg ttt tct gag gat      146
Ser Ile Pro Ala Thr Gly Arg Thr Ser Thr Val Ser Phe Ser Glu Asp
    30                  35                  40

ccg acg acg aag att cgg aag ccg tac aca atc aag aag tcg aga gag      194
Pro Thr Thr Lys Ile Arg Lys Pro Tyr Thr Ile Lys Lys Ser Arg Glu
45                  50                  55                  60

aat tgg aca gat caa gag cac gat aaa ttt cta gaa gct ctt cac tta      242
Asn Trp Thr Asp Gln Glu His Asp Lys Phe Leu Glu Ala Leu His Leu
                65                  70                  75

ttc gat agg gat tgg aag aaa ata gaa gcc ttt gtt gga tca aaa aca 290
Phe Asp Arg Asp Trp Lys Lys Ile Glu Ala Phe Val Gly Ser Lys Thr
            80                  85                  90

gta gtt cag ata cga agc cac gct cag aaa tac ttt ctc aaa gtt cag 338
Val Val Gln Ile Arg Ser His Ala Gln Lys Tyr Phe Leu Lys Val Gln
        95                  100                 105

aag agt ggt gct aac gaa cat ctt cca ctt cct cga cct aag agg aaa 386
Lys Ser Gly Ala Asn Glu His Leu Pro Leu Pro Arg Pro Lys Arg Lys
    110                 115                 120

gcg agt cat cct tat cct ata aag gct cct aaa aat gtt gct tat acc 434
Ala Ser His Pro Tyr Pro Ile Lys Ala Pro Lys Asn Val Ala Tyr Thr
125                 130                 135                 140

tct ctc ccg tct tcg agt aca tta ccg ttg ctt gag cct ggt tat ttg 482
Ser Leu Pro Ser Ser Ser Thr Leu Pro Leu Leu Glu Pro Gly Tyr Leu
                145                 150                 155

tat agc tct gat tcg aag tca ttg atg gga aac cag gct gtt tgt gca 530
Tyr Ser Ser Asp Ser Lys Ser Leu Met Gly Asn Gln Ala Val Cys Ala
            160                 165                 170

tct acc tct tct tcg tgg aat cat gaa tcg aca aat ctg cca aaa ccg 578
Ser Thr Ser Ser Ser Trp Asn His Glu Ser Thr Asn Leu Pro Lys Pro
        175                 180                 185

gtg att gaa gag gaa ccg gga gtc tcg gcc acg gct cct ctc cca aat 626
Val Ile Glu Glu Glu Pro Gly Val Ser Ala Thr Ala Pro Leu Pro Asn
    190                 195                 200

aat cgc tgc aga cag gaa gat aca gag agg gta cga gca gtg aca aag 674
Asn Arg Cys Arg Gln Glu Asp Thr Glu Arg Val Arg Ala Val Thr Lys
205                 210                 215                 220

cca aat aac gaa gaa agt tgt gaa aag cca cat aga gtg atg ccg aat 722
Pro Asn Asn Glu Glu Ser Cys Glu Lys Pro His Arg Val Met Pro Asn
                225                 230                 235

ttt gct gaa gtt tac agc ttc att gga agt gtc ttc gat ccc aac aca 770
Phe Ala Glu Val Tyr Ser Phe Ile Gly Ser Val Phe Asp Pro Asn Thr
            240                 245                 250

tca ggc cac ctc cag aga tta aag cag atg gat cca ata aat atg gaa 818
Ser Gly His Leu Gln Arg Leu Lys Gln Met Asp Pro Ile Asn Met Glu
        255                 260                 265

acg gtt ctt tta ctg atg caa aac ctg tct gta aat ctg aca agt ccc 866
Thr Val Leu Leu Leu Met Gln Asn Leu Ser Val Asn Leu Thr Ser Pro
    270                 275                 280

gag ttt gca gag caa agg agg ttg ata tca tca tac agc gct aaa gct 914
Glu Phe Ala Glu Gln Arg Arg Leu Ile Ser Ser Tyr Ser Ala Lys Ala
285                 290                 295                 300

ttg aaa tag agatagaata aaacaataat gtaccttatg tgagatcaag 963
Leu Lys

agacaatcat ccaaggtctg tatgcattgc ttggatttag gcctcgtgtt ctcactacag 1023

gagcagaacc aatcgcaaag actcttagat ggctactgag ttgtggtttt tatgtctctg 1083

taagtcgcgg tggagcacac gtgtttgtcc tgtcttgtgt atgtgtgtat agataataca 1143

aggttttgca gagtaaggtc acagttagct gcaagtgagt ttggatcaat cttaagatta 1203

aaaccctgag agtgagtgtc caaagagact gtgtaatatt ggtttggcgg tcagcagaag 1263

agttttgaag tgcacatcca gttagtgata acacggttga agaaaaggta aggttacaag 1323

tttagttttg aataattgta tactcaaaaa atatgaatgt ataaagaata atcacttgag 1383

tcgcctta 1391

<210> 12
<211> 302
<212> PRT
<213> Arabidopsis thaliana

<400> 12

Met Val Ser Val Asn Pro Arg Pro Lys Gly Phe Pro Val Phe Asp Ser
1               5                   10                  15

Ser Asn Met Ser Leu Pro Ser Ser Asp Gly Phe Gly Ser Ile Pro Ala
            20                  25                  30

Thr Gly Arg Thr Ser Thr Val Ser Phe Ser Glu Asp Pro Thr Thr Lys
        35                  40                  45

Ile Arg Lys Pro Tyr Thr Ile Lys Lys Ser Arg Glu Asn Trp Thr Asp
    50                  55                  60

Gln Glu His Asp Lys Phe Leu Glu Ala Leu His Leu Phe Asp Arg Asp
65                  70                  75                  80

Trp Lys Lys Ile Glu Ala Phe Val Gly Ser Lys Thr Val Val Gln Ile
                85                  90                  95

Arg Ser His Ala Gln Lys Tyr Phe Leu Lys Val Gln Lys Ser Gly Ala
            100                 105                 110

Asn Glu His Leu Pro Leu Pro Arg Pro Lys Arg Lys Ala Ser His Pro
        115                 120                 125

Tyr Pro Ile Lys Ala Pro Lys Asn Val Ala Tyr Thr Ser Leu Pro Ser
    130                 135                 140

Ser Ser Thr Leu Pro Leu Leu Glu Pro Gly Tyr Leu Tyr Ser Ser Asp
145                 150                 155                 160

Ser Lys Ser Leu Met Gly Asn Gln Ala Val Cys Ala Ser Thr Ser Ser
                165                 170                 175

Ser Trp Asn His Glu Ser Thr Asn Leu Pro Lys Pro Val Ile Glu Glu
            180                 185                 190

Glu Pro Gly Val Ser Ala Thr Ala Pro Leu Pro Asn Asn Arg Cys Arg
        195                 200                 205

Gln Glu Asp Thr Glu Arg Val Arg Ala Val Thr Lys Pro Asn Asn Glu
    210                 215                 220

Glu Ser Cys Glu Lys Pro His Arg Val Met Pro Asn Phe Ala Glu Val
225                 230                 235                 240

Tyr Ser Phe Ile Gly Ser Val Phe Asp Pro Asn Thr Ser Gly His Leu
                245                 250                 255

Gln Arg Leu Lys Gln Met Asp Pro Ile Asn Met Glu Thr Val Leu Leu
            260                 265                 270

Leu Met Gln Asn Leu Ser Val Asn Leu Thr Ser Pro Glu Phe Ala Glu
        275                 280                 285

Gln Arg Arg Leu Ile Ser Ser Tyr Ser Ala Lys Ala Leu Lys
    290                 295                 300

<210> 13
<211> 1121
<212> DNA
<213> Arabidopsis thaliana

<220>
<221> CDS
<222> (48)..(902)
<223> G263

<400> 13
tttttagttt tatttttctg tggtaaaata aaaaaagttc gccggag atg acg gct 56
Met Thr Ala
1

gtg acg gcg gcg caa aga tca gtt ccg gcg ccg ttt tta agc aaa acg 104
Val Thr Ala Ala Gln Arg Ser Val Pro Ala Pro Phe Leu Ser Lys Thr
    5                   10                  15

tat cag cta gtt gat gat cat agc aca gac gac gtc gtt tca tgg aac 152
Tyr Gln Leu Val Asp Asp His Ser Thr Asp Asp Val Val Ser Trp Asn
20                  25                  30                  35

gaa gaa gga aca gct ttt gtc gtg tgg aaa aca gca gag ttt gct aaa 200
Glu Glu Gly Thr Ala Phe Val Val Trp Lys Thr Ala Glu Phe Ala Lys
                40                  45                  50

gat ctt ctt cct caa tac ttc aag cat aat aat ttc tca agc ttc att 248
Asp Leu Leu Pro Gln Tyr Phe Lys His Asn Asn Phe Ser Ser Phe Ile
            55                  60                  65

cgt cag ctc aac act tac gga ttt cgt aaa act gta ccg gat aaa tgg 296
Arg Gln Leu Asn Thr Tyr Gly Phe Arg Lys Thr Val Pro Asp Lys Trp
        70                  75                  80

gaa ttt gca aac gat tat ttc cgg aga ggc ggg gag gat ctg ttg acg 344
Glu Phe Ala Asn Asp Tyr Phe Arg Arg Gly Gly Glu Asp Leu Leu Thr
    85                  90                  95

gac ata cga cgg cgt aaa tcg gtg att gct tca acg gcg ggg aaa tgt 392
Asp Ile Arg Arg Arg Lys Ser Val Ile Ala Ser Thr Ala Gly Lys Cys
100                 105                 110                 115

gtt gtt gtt ggt tcg cct tct gag tct aat tct ggt ggt ggt gat gat 440
Val Val Val Gly Ser Pro Ser Glu Ser Asn Ser Gly Gly Gly Asp Asp
                120                 125                 130

cac ggt tca agc tcc acg tca tca ccc ggt tcg tcg aag aat cct ggt 488
His Gly Ser Ser Ser Thr Ser Ser Pro Gly Ser Ser Lys Asn Pro Gly
            135                 140                 145

tcg gtg gag aac atg gtt gct gat tta tca gga gag aac gag aag ctt 536
Ser Val Glu Asn Met Val Ala Asp Leu Ser Gly Glu Asn Glu Lys Leu
        150                 155                 160

aaa cgt gaa aac aat aac ttg agc tcg gag ctc gcg gcg gcg aag aag 584
Lys Arg Glu Asn Asn Asn Leu Ser Ser Glu Leu Ala Ala Ala Lys Lys
    165                 170                 175

cag cgc gat gag cta gtg acg ttc ttg acg ggt cat ctg aaa gta aga 632
Gln Arg Asp Glu Leu Val Thr Phe Leu Thr Gly His Leu Lys Val Arg
180                 185                 190                 195

ccg gaa caa atc gat aaa atg atc aaa gga ggg aaa ttt aaa ccg gtg 680

Pro Glu Gln Ile Asp Lys Met Ile Lys Gly Gly Lys Phe Lys Pro Val
                200                 205                 210

gag tct gac gaa gag agt gag tgc gaa ggt tgc gac ggc ggc gga gga 728
Glu Ser Asp Glu Glu Ser Glu Cys Glu Gly Cys Asp Gly Gly Gly Gly
            215                 220                 225

gca gag gag ggg gta ggt gaa gga ttg aaa ttg ttt ggg gtg tgg ttg 776
Ala Glu Glu Gly Val Gly Glu Gly Leu Lys Leu Phe Gly Val Trp Leu
        230                 235                 240

aaa gga gag aga aaa aag agg gac cgg gat gaa aag aat tat gtg gtg 824
Lys Gly Glu Arg Lys Lys Arg Asp Arg Asp Glu Lys Asn Tyr Val Val
    245                 250                 255

agt ggg tcc cgt atg acg gaa ata aag aac gtg gac ttt cac gcg ccg 872
Ser Gly Ser Arg Met Thr Glu Ile Lys Asn Val Asp Phe His Ala Pro
260                 265                 270                 275

ttg tgg aaa agc agc aaa gtc tgc aac taa aaaaagagta gaagactgtt 922
Leu Trp Lys Ser Ser Lys Val Cys Asn
                280

caaaccagcg tgtgacacgt catcgacgac gacgaaaaaa atgatttaaa aaactatttt 982

tttccgtaag gaagaaaagt tatttttatg ttttaaaaag gtgaagaagg tccagaagga 1042

tcaacgcaaa tatataaatg gattttcatg tattatataa tttaattagt gtattaagaa 1102

aataaaacaa aaaaaaaaa 1121

<210> 14
<211> 284
<212> PRT
<213> Arabidopsis thaliana

<400> 14

Met Thr Ala Val Thr Ala Ala Gln Arg Ser Val Pro Ala Pro Phe Leu
1               5                   10                  15

Ser Lys Thr Tyr Gln Leu Val Asp Asp His Ser Thr Asp Asp Val Val
            20                  25                  30

Ser Trp Asn Glu Glu Gly Thr Ala Phe Val Val Trp Lys Thr Ala Glu
        35                  40                  45

Phe Ala Lys Asp Leu Leu Pro Gln Tyr Phe Lys His Asn Asn Phe Ser
    50                  55                  60

Ser Phe Ile Arg Gln Leu Asn Thr Tyr Gly Phe Arg Lys Thr Val Pro
65                  70                  75                  80

Asp Lys Trp Glu Phe Ala Asn Asp Tyr Phe Arg Arg Gly Gly Glu Asp
                85                  90                  95

Leu Leu Thr Asp Ile Arg Arg Arg Lys Ser Val Ile Ala Ser Thr Ala
            100                 105                 110

Gly Lys Cys Val Val Val Gly Ser Pro Ser Glu Ser Asn Ser Gly Gly
        115                 120                 125

Gly Asp Asp His Gly Ser Ser Ser Thr Ser Ser Pro Gly Ser Ser Lys
    130                 135                 140

Asn Pro Gly Ser Val Glu Asn Met Val Ala Asp Leu Ser Gly Glu Asn
145                 150                 155                 160

Glu Lys Leu Lys Arg Glu Asn Asn Asn Leu Ser Ser Glu Leu Ala Ala
                165                 170                 175

Ala Lys Lys Gln Arg Asp Glu Leu Val Thr Phe Leu Thr Gly His Leu
            180                 185                 190

Lys Val Arg Pro Glu Gln Ile Asp Lys Met Ile Lys Gly Gly Lys Phe
        195                 200                 205

Lys Pro Val Glu Ser Asp Glu Glu Ser Glu Cys Glu Gly Cys Asp Gly
    210                 215                 220

Gly Gly Gly Ala Glu Glu Gly Val Gly Glu Gly Leu Lys Leu Phe Gly
225                 230                 235                 240

Val Trp Leu Lys Gly Glu Arg Lys Lys Arg Asp Arg Asp Glu Lys Asn
                245                 250                 255

Tyr Val Val Ser Gly Ser Arg Met Thr Glu Ile Lys Asn Val Asp Phe
            260                 265                 270

His Ala Pro Leu Trp Lys Ser Ser Lys Val Cys Asn
        275                 280

<210>  15
<211>  1951
<212>  DNA
<213>  Arabidopsis thaliana

<220>
<221>  CDS
<222>  (196)..(1794)
<223>  G308

<400>  15
   agtaatttag tttttttttt ttttttttac aatttatttt gttattagaa gtggtagtgg      60
   agtgaaaaaa caaatcctaa gcagtcctaa ccgatccccg aagctaaaga ttcttcacct     120
   tcccaaataa agcaaaacct agatccgaca ttgaaggaaa aaccttttag atccatctct     180
   gaaaaaaacc caacc atg aag aga gat cat cat cat cat cat caa gat aag     231
                    Met Lys Arg Asp His His His His His Gln Asp Lys
                    1               5                   10
   aag act atg atg atg aat gaa gaa gac gac ggt aac ggc atg gat gag     279
   Lys Thr Met Met Met Asn Glu Glu Asp Asp Gly Asn Gly Met Asp Glu
           15                  20                  25
   ctt cta gct gtt ctt ggt tac aag gtt agg tca tcg gaa atg gct gat     327
   Leu Leu Ala Val Leu Gly Tyr Lys Val Arg Ser Ser Glu Met Ala Asp
       30                  35                  40
   gtt gct cag aaa ctc gag cag ctt gaa gtt atg atg tct aat gtt caa     375
   Val Ala Gln Lys Leu Glu Gln Leu Glu Val Met Met Ser Asn Val Gln
   45                  50                  55                  60
   gaa gac gat ctt tct caa ctc gct act gag act gtt cac tat aat ccg     423
   Glu Asp Asp Leu Ser Gln Leu Ala Thr Glu Thr Val His Tyr Asn Pro
                   65                  70                  75
   gcg gag ctt tac acg tgg ctt gat tct atg ctc acc gac ctt aat cct     471
   Ala Glu Leu Tyr Thr Trp Leu Asp Ser Met Leu Thr Asp Leu Asn Pro
   80 85 90
   ccg tcg tct aac gcc gag tac gat ctt aaa gct att ccc ggt gac gcg 519
   Pro Ser Ser Asn Ala Glu Tyr Asp Leu Lys Ala Ile Pro Gly Asp Ala
   95 100 105
   att ctc aat cag ttc gct atc gat tcg gct tct tcg tct aac caa ggc 567
   Ile Leu Asn Gln Phe Ala Ile Asp Ser Ala Ser Ser Ser Asn Gln Gly
   110 115 120
   ggc gga gga gat acg tat act aca aac aag cgg ttg aaa tgc tca aac 615
   Gly Gly Gly Asp Thr Tyr Thr Thr Asn Lys Arg Leu Lys Cys Ser Asn
   125 130 135 140
   ggc gtc gtg gaa acc acc aca gcg acg gct gag tca act cgg cat gtt 663
   Gly Val Val Glu Thr Thr Thr Ala Thr Ala Glu Ser Thr Arg His Val
   145 150 155
   gtc ctg gtt gac tcg cag gag aac ggt gtg cgt ctc gtt cac gcg ctt 711
   Val Leu Val Asp Ser Gln Glu Asn Gly Val Arg Leu Val His Ala Leu
   160 165 170
   ttg gct tgc gct gaa gct gtt cag aag gag aat ctg act gtg gcg gaa 759
   Leu Ala Cys Ala Glu Ala Val Gln Lys Glu Asn Leu Thr Val Ala Glu
   175 180 185
   gct ctg gtg aag caa atc gga ttc tta gct gtt tct caa atc gga gct 807
   Ala Leu Val Lys Gln Ile Gly Phe Leu Ala Val Ser Gln Ile Gly Ala
   190 195 200
   atg aga caa gtc gct act tac ttc gcc gaa gct ctc gcg cgg cgg att 855
   Met Arg Gln Val Ala Thr Tyr Phe Ala Glu Ala Leu Ala Arg Arg Ile
   205 210 215 220
   tac cgt ctc tct ccg tcg cag agt cca atc gac cac tct ctc tcc gat 903
   Tyr Arg Leu Ser Pro Ser Gln Ser Pro Ile Asp His Ser Leu Ser Asp
   225 230 235
   act ctt cag atg cac ttc tac gag act tgt cct tat ctc aag ttc gct 951
   Thr Leu Gln Met His Phe Tyr Glu Thr Cys Pro Tyr Leu Lys Phe Ala
   240 245 250
   cac ttc acg gcg aat caa gcg att ctc gaa gct ttt caa ggg aag aaa 999
   His Phe Thr Ala Asn Gln Ala Ile Leu Glu Ala Phe Gln Gly Lys Lys
   255 260 265
   aga gtt cat gtc att gat ttc tct atg agt caa ggt ctt caa tgg ccg 1047
   Arg Val His Val Ile Asp Phe Ser Met Ser Gln Gly Leu Gln Trp Pro
   270 275 280
   gcg ctt atg cag gct ctt gcg ctt cga cct ggt ggt cct cct gtt ttc 1095
   Ala Leu Met Gln Ala Leu Ala Leu Arg Pro Gly Gly Pro Pro Val Phe
   285 290 295 300
   cgg tta acc gga att ggt cca ccg gca ccg gat aat ttc gat tat ctt 1143
   Arg Leu Thr Gly Ile Gly Pro Pro Ala Pro Asp Asn Phe Asp Tyr Leu
   305 310 315
   cat gaa gtt ggg tgt aag ctg gct cat tta gct gag gcg att cac gtt 1191
   His Glu Val Gly Cys Lys Leu Ala His Leu Ala Glu Ala Ile His Val
   320 325 330
   gag ttt gag tac aga gga ttt gtg gct aac act tta gct gat ctt gat 1239
   Glu Phe Glu Tyr Arg Gly Phe Val Ala Asn Thr Leu Ala Asp Leu Asp
   335 340 345
   gct tcg atg ctt gag ctt aga cca agt gag att gaa tct gtt gcg gtt 1287
   Ala Ser Met Leu Glu Leu Arg Pro Ser Glu Ile Glu Ser Val Ala Val
   350 355 360
   aac tct gtt ttc gag ctt cac aag ctc ttg gga cga cct ggt gcg atc 1335
   Asn Ser Val Phe Glu Leu His Lys Leu Leu Gly Arg Pro Gly Ala Ile
   365 370 375 380
   gat aag gtt ctt ggt gtg gtg aat cag att aaa ccg gag att ttc act 1383
   Asp Lys Val Leu Gly Val Val Asn Gln Ile Lys Pro Glu Ile Phe Thr
   385 390 395
   gtg gtt gag cag gaa tcg aac cat aat agt ccg att ttc tta gat cgg 1431
   Val Val Glu Gln Glu Ser Asn His Asn Ser Pro Ile Phe Leu Asp Arg
   400 405 410
   ttt act gag tcg ttg cat tat tac tcg acg ttg ttt gac tcg ttg gaa 1479
   Phe Thr Glu Ser Leu His Tyr Tyr Ser Thr Leu Phe Asp Ser Leu Glu
   415 420 425
   ggt gta ccg agt ggt caa gac aag gtc atg tcg gag gtt tac ttg ggt 1527
   Gly Val Pro Ser Gly Gln Asp Lys Val Met Ser Glu Val Tyr Leu Gly
   430 435 440
   aaa cag atc tgc aac gtt gtg gct tgt gat gga cct gac cga gtt gag 1575
   Lys Gln Ile Cys Asn Val Val Ala Cys Asp Gly Pro Asp Arg Val Glu
   445 450 455 460
   cgt cat gaa acg ttg agt cag tgg agg aac cgg ttc ggg tct gct ggg 1623
   Arg His Glu Thr Leu Ser Gln Trp Arg Asn Arg Phe Gly Ser Ala Gly
   465 470 475
   ttt gcg gct gca cat att ggt tcg aat gcg ttt aag caa gcg agt atg 1671
   Phe Ala Ala Ala His Ile Gly Ser Asn Ala Phe Lys Gln Ala Ser Met
   480 485 490
   ctt ttg gct ctg ttc aac ggc ggt gag ggt tat cgg gtg gag gag agt 1719
   Leu Leu Ala Leu Phe Asn Gly Gly Glu Gly Tyr Arg Val Glu Glu Ser
   495 500 505
   gac ggc tgt ctc atg ttg ggt tgg cac aca cga ccg ctc ata gcc acc 1767
   Asp Gly Cys Leu Met Leu Gly Trp His Thr Arg Pro Leu Ile Ala Thr
   510 515 520
   tcg gct tgg aaa ctc tcc acc aat tag atggtggctc aatgaattga 1814
   Ser Ala Trp Lys Leu Ser Thr Asn
   525 530
   tctgttgaac cggttatgat gatagatttc cgaccgaagc caaactaaat cctactgttt 1874
   ttccctttgt cacttgttaa gatcttatct ttcattatat taggtaattg aaaaatttta 1934
   atctcgccta aattact 1951

<210> 16
<211> 532
<212> PRT
<213> Arabidopsis thaliana

<400> 16

Met Lys Arg Asp His His His His His Gln Asp Lys Lys Thr Met Met
1               5                   10                  15

Met Asn Glu Glu Asp Asp Gly Asn Gly Met Asp Glu Leu Leu Ala Val
            20                  25                  30

Leu Gly Tyr Lys Val Arg Ser Ser Glu Met Ala Asp Val Ala Gln Lys
        35                  40                  45

Leu Glu Gln Leu Glu Val Met Met Ser Asn Val Gln Glu Asp Asp Leu
    50                  55                  60

Ser Gln Leu Ala Thr Glu Thr Val His Tyr Asn Pro Ala Glu Leu Tyr
65                  70                  75                  80

Thr Trp Leu Asp Ser Met Leu Thr Asp Leu Asn Pro Pro Ser Ser Asn

                85                  90                  95

Ala Glu Tyr Asp Leu Lys Ala Ile Pro Gly Asp Ala Ile Leu Asn Gln
            100                 105                 110

Phe Ala Ile Asp Ser Ala Ser Ser Ser Asn Gln Gly Gly Gly Gly Asp
        115                 120                 125

Thr Tyr Thr Thr Asn Lys Arg Leu Lys Cys Ser Asn Gly Val Val Glu
    130                 135                 140

Thr Thr Thr Ala Thr Ala Glu Ser Thr Arg His Val Val Leu Val Asp
145                 150                 155                 160

Ser Gln Glu Asn Gly Val Arg Leu Val His Ala Leu Leu Ala Cys Ala
                165                 170                 175

Glu Ala Val Gln Lys Glu Asn Leu Thr Val Ala Glu Ala Leu Val Lys
            180                 185                 190

Gln Ile Gly Phe Leu Ala Val Ser Gln Ile Gly Ala Met Arg Gln Val
        195                 200                 205

Ala Thr Tyr Phe Ala Glu Ala Leu Ala Arg Arg Ile Tyr Arg Leu Ser
    210                 215                 220

Pro Ser Gln Ser Pro Ile Asp His Ser Leu Ser Asp Thr Leu Gln Met
225                 230                 235                 240

His Phe Tyr Glu Thr Cys Pro Tyr Leu Lys Phe Ala His Phe Thr Ala
                245                 250                 255

Asn Gln Ala Ile Leu Glu Ala Phe Gln Gly Lys Lys Arg Val His Val
            260                 265                 270

Ile Asp Phe Ser Met Ser Gln Gly Leu Gln Trp Pro Ala Leu Met Gln
        275                 280                 285

Ala Leu Ala Leu Arg Pro Gly Gly Pro Pro Val Phe Arg Leu Thr Gly
    290                 295                 300

Ile Gly Pro Pro Ala Pro Asp Asn Phe Asp Tyr Leu His Glu Val Gly
305                 310                 315                 320

Cys Lys Leu Ala His Leu Ala Glu Ala Ile His Val Glu Phe Glu Tyr
                325                 330                 335

Arg Gly Phe Val Ala Asn Thr Leu Ala Asp Leu Asp Ala Ser Met Leu
            340                 345                 350

Glu Leu Arg Pro Ser Glu Ile Glu Ser Val Ala Val Asn Ser Val Phe
        355                 360                 365

Glu Leu His Lys Leu Leu Gly Arg Pro Gly Ala Ile Asp Lys Val Leu
    370                 375                 380

Gly Val Val Asn Gln Ile Lys Pro Glu Ile Phe Thr Val Val Glu Gln
385                 390                 395                 400

Glu Ser Asn His Asn Ser Pro Ile Phe Leu Asp Arg Phe Thr Glu Ser
                405                 410                 415

Leu His Tyr Tyr Ser Thr Leu Phe Asp Ser Leu Glu Gly Val Pro Ser
            420                 425                 430

Gly Gln Asp Lys Val Met Ser Glu Val Tyr Leu Gly Lys Gln Ile Cys
        435                 440                 445

Asn Val Val Ala Cys Asp Gly Pro Asp Arg Val Glu Arg His Glu Thr
    450                 455                 460

Leu Ser Gln Trp Arg Asn Arg Phe Gly Ser Ala Gly Phe Ala Ala Ala
465                 470                 475                 480

His Ile Gly Ser Asn Ala Phe Lys Gln Ala Ser Met Leu Leu Ala Leu
                485                 490                 495

Phe Asn Gly Gly Glu Gly Tyr Arg Val Glu Glu Ser Asp Gly Cys Leu
            500                 505                 510

Met Leu Gly Trp His Thr Arg Pro Leu Ile Ala Thr Ser Ala Trp Lys
        515                 520                 525

Leu Ser Thr Asn
    530

<210>  17
<211>  768
<212>  DNA
<213>  Arabidopsis thaliana

<220>
<221>  CDS
<222>  (1)..(768)
<223>  G536

<400>  17
atg tcg aca agg gaa gag aat gtt tac atg gcg aaa tta gcc gaa caa      48
Met Ser Thr Arg Glu Glu Asn Val Tyr Met Ala Lys Leu Ala Glu Gln
1               5                   10                  15

gct gaa cgt tac gaa gaa atg gtt gaa ttc atg gag aaa gtt gcg aaa      96
Ala Glu Arg Tyr Glu Glu Met Val Glu Phe Met Glu Lys Val Ala Lys
            20                  25                  30

act gtt gat gtt gag gaa ctt tca gtt gaa gag agg aat ctt ctc tct      144
Thr Val Asp Val Glu Glu Leu Ser Val Glu Glu Arg Asn Leu Leu Ser
        35                  40                  45

gtt gct tac aag aac gtg att gga gcg aga aga gct tcg tgg aga atc      192
Val Ala Tyr Lys Asn Val Ile Gly Ala Arg Arg Ala Ser Trp Arg Ile
    50                  55                  60

att tct tcg att gag cag aaa gaa gag agc aaa ggg aac gaa gat cat      240
Ile Ser Ser Ile Glu Gln Lys Glu Glu Ser Lys Gly Asn Glu Asp His
65                  70                  75                  80

gtt gct att atc aag gat tac aga gga gag att gaa tcc gag ctt agc      288
Val Ala Ile Ile Lys Asp Tyr Arg Gly Glu Ile Glu Ser Glu Leu Ser
                85                  90                  95

aaa atc tgt gat ggg att ttg aat gtt ctt gaa gct cat ctt att cct 336
Lys Ile Cys Asp Gly Ile Leu Asn Val Leu Glu Ala His Leu Ile Pro
            100                 105                 110

tct gct tca cca gct gaa tct aaa gtg ttt tat ctt aag atg aag ggt 384
Ser Ala Ser Pro Ala Glu Ser Lys Val Phe Tyr Leu Lys Met Lys Gly
        115                 120                 125

gat tat cat agg tat ctt gct gag ttt aag gct ggt gct gaa agg aaa 432
Asp Tyr His Arg Tyr Leu Ala Glu Phe Lys Ala Gly Ala Glu Arg Lys
    130                 135                 140

gaa gct gct gaa agc act ttg gtt gct tac aag tct gct tcc gac att 480
Glu Ala Ala Glu Ser Thr Leu Val Ala Tyr Lys Ser Ala Ser Asp Ile
145                 150                 155                 160

gcc act gct gag tta gct cct act cac ccg ata agg ctt ggt ctt gca 528
Ala Thr Ala Glu Leu Ala Pro Thr His Pro Ile Arg Leu Gly Leu Ala
                165                 170                 175

ctc aac ttc tct gtg ttt tac tat gaa atc ctc aac tcg cct gat cgt 576
Leu Asn Phe Ser Val Phe Tyr Tyr Glu Ile Leu Asn Ser Pro Asp Arg
            180                 185                 190

gct tgc agc ctc gca aag cag gcg ttt gat gat gca atc gct gag tta 624
Ala Cys Ser Leu Ala Lys Gln Ala Phe Asp Asp Ala Ile Ala Glu Leu
        195                 200                 205

gat aca ttg ggt gag gaa tca tac aag gac agt aca ctg att atg cag 672
Asp Thr Leu Gly Glu Glu Ser Tyr Lys Asp Ser Thr Leu Ile Met Gln
    210                 215                 220

ctt ctt aga gac aat ctc act ctc tgg act tca gat atg act gac gaa 720
Leu Leu Arg Asp Asn Leu Thr Leu Trp Thr Ser Asp Met Thr Asp Glu
225                 230                 235                 240

gca gga gat gag att aag gag gca tca aag ccc gat ggt gcc gag taa 768
Ala Gly Asp Glu Ile Lys Glu Ala Ser Lys Pro Asp Gly Ala Glu
                245                 250                 255

<210> 18
<211> 255
<212> PRT
<213> Arabidopsis thaliana

<400> 18

Met Ser Thr Arg Glu Glu Asn Val Tyr Met Ala Lys Leu Ala Glu Gln
1               5                   10                  15

Ala Glu Arg Tyr Glu Glu Met Val Glu Phe Met Glu Lys Val Ala Lys
            20                  25                  30

Thr Val Asp Val Glu Glu Leu Ser Val Glu Glu Arg Asn Leu Leu Ser
        35                  40                  45

Val Ala Tyr Lys Asn Val Ile Gly Ala Arg Arg Ala Ser Trp Arg Ile
    50                  55                  60

Ile Ser Ser Ile Glu Gln Lys Glu Glu Ser Lys Gly Asn Glu Asp His
65                  70                  75                  80

Val Ala Ile Ile Lys Asp Tyr Arg Gly Glu Ile Glu Ser Glu Leu Ser
                85                  90                  95

Lys Ile Cys Asp Gly Ile Leu Asn Val Leu Glu Ala His Leu Ile Pro
            100                 105                 110

Ser Ala Ser Pro Ala Glu Ser Lys Val Phe Tyr Leu Lys Met Lys Gly
        115                 120                 125

Asp Tyr His Arg Tyr Leu Ala Glu Phe Lys Ala Gly Ala Glu Arg Lys
    130                 135                 140

Glu Ala Ala Glu Ser Thr Leu Val Ala Tyr Lys Ser Ala Ser Asp Ile
145                 150                 155                 160

Ala Thr Ala Glu Leu Ala Pro Thr His Pro Ile Arg Leu Gly Leu Ala
                165                 170                 175

Leu Asn Phe Ser Val Phe Tyr Tyr Glu Ile Leu Asn Ser Pro Asp Arg
            180                 185                 190

Ala Cys Ser Leu Ala Lys Gln Ala Phe Asp Asp Ala Ile Ala Glu Leu
        195                 200                 205

Asp Thr Leu Gly Glu Glu Ser Tyr Lys Asp Ser Thr Leu Ile Met Gln
    210                 215                 220

Leu Leu Arg Asp Asn Leu Thr Leu Trp Thr Ser Asp Met Thr Asp Glu
225                 230                 235                 240

Ala Gly Asp Glu Ile Lys Glu Ala Ser Lys Pro Asp Gly Ala Glu
                245                 250                 255

<210>  19
<211>  2526
<212>  DNA
<213>  Arabidopsis thaliana

<220>
<221>  CDS
<222>  (338)..(2275)
<223>  G680

<400>  19
cagttatctt cttccttctt ctctctgttt tttaaattta tttttagaga attttttttg      60

ttttgcttcc gatttgatta tttccgggaa cgatgacttc tccggggagt tcccggtgag     120

atgataagtc agattgcata cttgtctcct ccatggctac tctcaagggt tttggctgcg     180

gtggattcgt ttggtttctc tagaatctaa agaggttatc acaacggctt tgcaatttga     240

aaactttcat gtttggggag atcaaagatg gtttcttttt tatactttac ttgttagaga     300

ggatttgaag cagcgaatag ctgcaaccgg tcctgtt atg gat act aat aca tct      355
                                         Met Asp Thr Asn Thr Ser
                                         1               5

gga gaa gaa tta tta gct aag gca aga aag cca tat aca ata aca aag      403
Gly Glu Glu Leu Leu Ala Lys Ala Arg Lys Pro Tyr Thr Ile Thr Lys
            10                  15                  20

cag cga gag cga tgg act gag gat gag cat gag agg ttt cta gaa gcc      451
Gln Arg Glu Arg Trp Thr Glu Asp Glu His Glu Arg Phe Leu Glu Ala
        25                  30                  35

ttg agg ctt tat gga aga gct tgg caa cga att gaa gaa cat att ggg      499
Leu Arg Leu Tyr Gly Arg Ala Trp Gln Arg Ile Glu Glu His Ile Gly
    40                  45                  50

aca aag act gct gtt cag atc aga agt cat gca caa aag ttc ttc aca 547
Thr Lys Thr Ala Val Gln Ile Arg Ser His Ala Gln Lys Phe Phe Thr
55                  60                  65                  70

aag ttg gag aaa gag gct gaa gtt aaa ggc atc cct gtt tgc caa gct 595
Lys Leu Glu Lys Glu Ala Glu Val Lys Gly Ile Pro Val Cys Gln Ala
                75                  80                  85

ttg gac ata gaa att ccg cct cct cgt cct aaa cga aaa ccc aat act 643
Leu Asp Ile Glu Ile Pro Pro Pro Arg Pro Lys Arg Lys Pro Asn Thr
            90                  95                  100

cct tat cct cga aaa cct ggg aac aac ggt aca tct tcc tct caa gta 691
Pro Tyr Pro Arg Lys Pro Gly Asn Asn Gly Thr Ser Ser Ser Gln Val
        105                 110                 115

tca tca gca aaa gat gca aaa ctt gtt tca tcg gcc tct tct tca cag 739
Ser Ser Ala Lys Asp Ala Lys Leu Val Ser Ser Ala Ser Ser Ser Gln
    120                 125                 130

ttg aat cag gcg ttc ttg gat ttg gaa aaa atg ccg ttc tct gag aaa 787
Leu Asn Gln Ala Phe Leu Asp Leu Glu Lys Met Pro Phe Ser Glu Lys
135                 140                 145                 150

aca tca act gga aaa gaa aat caa gat gag aat tgc tcg ggt gtt tct 835
Thr Ser Thr Gly Lys Glu Asn Gln Asp Glu Asn Cys Ser Gly Val Ser
                155                 160                 165

act gtg aac aag tat ccc tta cca acg aaa cag gta agt ggc gac att 883
Thr Val Asn Lys Tyr Pro Leu Pro Thr Lys Gln Val Ser Gly Asp Ile
            170                 175                 180

gaa aca agt aag acc tca act gtg gac aac gcg gtt caa gat gtt ccc 931
Glu Thr Ser Lys Thr Ser Thr Val Asp Asn Ala Val Gln Asp Val Pro
        185                 190                 195

aag aag aac aaa gac aaa gat ggt aac gat ggt act act gtg cac agc 979
Lys Lys Asn Lys Asp Lys Asp Gly Asn Asp Gly Thr Thr Val His Ser
    200                 205                 210

atg caa aac tac cct tgg cat ttc cac gca gat att gtg aac ggg aat 1027
Met Gln Asn Tyr Pro Trp His Phe His Ala Asp Ile Val Asn Gly Asn
215                 220                 225                 230

ata gca aaa tgc cct caa aat cat ccc tca ggt atg gta tct caa gac 1075
Ile Ala Lys Cys Pro Gln Asn His Pro Ser Gly Met Val Ser Gln Asp
                235                 240                 245

ttc atg ttt cat cct atg aga gaa gaa act cac ggg cac gca aat ctt 1123
Phe Met Phe His Pro Met Arg Glu Glu Thr His Gly His Ala Asn Leu
            250                 255                 260

caa gct aca aca gca tct gct act act aca gct tct cat caa gcg ttt 1171
Gln Ala Thr Thr Ala Ser Ala Thr Thr Thr Ala Ser His Gln Ala Phe
        265                 270                 275

cca gct tgt cat tca cag gat gat tac cgt tcg ttt ctc cag ata tca 1219
Pro Ala Cys His Ser Gln Asp Asp Tyr Arg Ser Phe Leu Gln Ile Ser
    280                 285                 290

tct act ttc tcc aat ctt att atg tca act ctc cta cag aat cct gca 1267
Ser Thr Phe Ser Asn Leu Ile Met Ser Thr Leu Leu Gln Asn Pro Ala
295                 300                 305                 310

gct cat gct gca gct aca ttc gct gct tcg gtc tgg cct tat gcg agt 1315
Ala His Ala Ala Ala Thr Phe Ala Ala Ser Val Trp Pro Tyr Ala Ser
                315                 320                 325

gtc ggg aat tct ggt gat tca tca acc cca atg agc tct tct cct cca 1363
Val Gly Asn Ser Gly Asp Ser Ser Thr Pro Met Ser Ser Ser Pro Pro
            330                 335                 340

agt ata act gcc att gcc gct gct aca gta gct gct gca act gct tgg 1411
Ser Ile Thr Ala Ile Ala Ala Ala Thr Val Ala Ala Ala Thr Ala Trp

345                 350                 355

tgg gct tct cat gga ctt ctt cct gta tgc gct cca gct cca ata aca     1459
Trp Ala Ser His Gly Leu Leu Pro Val Cys Ala Pro Ala Pro Ile Thr
    360                 365                 370

tgt gtt cca ttc tca act gtt gca gtt cca act cca gca atg act gaa     1507
Cys Val Pro Phe Ser Thr Val Ala Val Pro Thr Pro Ala Met Thr Glu
375                 380                 385                 390

atg gat acc gtt gaa aat act caa ccg ttt gag aaa caa aac aca gct     1555
Met Asp Thr Val Glu Asn Thr Gln Pro Phe Glu Lys Gln Asn Thr Ala
                395                 400                 405

ctg caa gat caa acc ttg gct tcg aaa tct cca gct tca tca tct gat     1603
Leu Gln Asp Gln Thr Leu Ala Ser Lys Ser Pro Ala Ser Ser Ser Asp
            410                 415                 420

gat tca gat gag act gga gta acc aag cta aat gcc gac tca aaa acc     1651
Asp Ser Asp Glu Thr Gly Val Thr Lys Leu Asn Ala Asp Ser Lys Thr
        425                 430                 435

aat gat gat aaa att gag gag gtt gtt gtt act gcc gct gtg cat gac     1699
Asn Asp Asp Lys Ile Glu Glu Val Val Val Thr Ala Ala Val His Asp
    440                 445                 450

tca aac act gcc cag aag aaa aat ctt gtg gac cgc tca tcg tgt ggc     1747
Ser Asn Thr Ala Gln Lys Lys Asn Leu Val Asp Arg Ser Ser Cys Gly
455                 460                 465                 470

tca aat aca cct tca ggg agt gac gca gaa act gat gca tta gat aaa     1795
Ser Asn Thr Pro Ser Gly Ser Asp Ala Glu Thr Asp Ala Leu Asp Lys
                475                 480                 485

atg gag aaa gat aaa gag gat gtg aag gag aca gat gag aat cag cca     1843
Met Glu Lys Asp Lys Glu Asp Val Lys Glu Thr Asp Glu Asn Gln Pro
            490                 495                 500

gat gtt att gag tta aat aac cgt aag att aaa atg aga gac aac aac     1891
Asp Val Ile Glu Leu Asn Asn Arg Lys Ile Lys Met Arg Asp Asn Asn
        505                 510                 515

agc aac aac aat gca act act gat tcg tgg aag gaa gtc tcc gaa gag     1939
Ser Asn Asn Asn Ala Thr Thr Asp Ser Trp Lys Glu Val Ser Glu Glu
    520                 525                 530

ggt cgt ata gcg ttt cag gct ctc ttt gca aga gaa aga ttg cct caa     1987
Gly Arg Ile Ala Phe Gln Ala Leu Phe Ala Arg Glu Arg Leu Pro Gln
535                 540                 545                 550

agc ttt tcg cct cct caa gtg gca gag aat gtg aat aga aaa caa agt     2035
Ser Phe Ser Pro Pro Gln Val Ala Glu Asn Val Asn Arg Lys Gln Ser
                555                 560                 565

gac acg tca atg cca ttg gct cct aat ttc aaa agc cag gat tct tgt     2083
Asp Thr Ser Met Pro Leu Ala Pro Asn Phe Lys Ser Gln Asp Ser Cys
            570                 575                 580

gct gca gac caa gaa gga gta gta atg atc ggt gtt gga aca tgc aag     2131
Ala Ala Asp Gln Glu Gly Val Val Met Ile Gly Val Gly Thr Cys Lys
        585                 590                 595

agt ctt aaa acg aga cag aca gga ttt aag cca tac aag aga tgt tca     2179
Ser Leu Lys Thr Arg Gln Thr Gly Phe Lys Pro Tyr Lys Arg Cys Ser
    600                 605                 610

atg gaa gtg aaa gag agc caa gtt ggg aac ata aac aat caa agt gat     2227
Met Glu Val Lys Glu Ser Gln Val Gly Asn Ile Asn Asn Gln Ser Asp
615                 620                 625                 630

gaa aaa gtc tgc aaa agg ctt cga ttg gaa gga gaa gct tct aca tga     2275
Glu Lys Val Cys Lys Arg Leu Arg Leu Glu Gly Glu Ala Ser Thr
                635                 640                 645

cagacttgga ggtaaaaaaa aaacatccac atttttatca atatctttaa atctagtgtt     2335

agtagtttgc ttctccaatc tttatgaaag agacttttaa ttttccttcc gaacatttct 2395

ttggtcatgt caggttctgt accatattac cccatgtctt gtctcttgtc tctgtttgtg 2455

tatgctactt gtggtctata tgtcatctgc tactactgtt aattaaccat taagcaatgg 2515

atttgtcttt a 2526

<210> 20
<211> 645
<212> PRT
<213> Arabidopsis thaliana

<400> 20

Met Asp Thr Asn Thr Ser Gly Glu Glu Leu Leu Ala Lys Ala Arg Lys
1               5                   10                  15

Pro Tyr Thr Ile Thr Lys Gln Arg Glu Arg Trp Thr Glu Asp Glu His
            20                  25                  30

Glu Arg Phe Leu Glu Ala Leu Arg Leu Tyr Gly Arg Ala Trp Gln Arg
        35                  40                  45

Ile Glu Glu His Ile Gly Thr Lys Thr Ala Val Gln Ile Arg Ser His
    50                  55                  60

Ala Gln Lys Phe Phe Thr Lys Leu Glu Lys Glu Ala Glu Val Lys Gly
65                  70                  75                  80

Ile Pro Val Cys Gln Ala Leu Asp Ile Glu Ile Pro Pro Pro Arg Pro
                85                  90                  95

Lys Arg Lys Pro Asn Thr Pro Tyr Pro Arg Lys Pro Gly Asn Asn Gly
            100                 105                 110

Thr Ser Ser Ser Gln Val Ser Ser Ala Lys Asp Ala Lys Leu Val Ser
        115                 120                 125

Ser Ala Ser Ser Ser Gln Leu Asn Gln Ala Phe Leu Asp Leu Glu Lys
    130                 135                 140

Met Pro Phe Ser Glu Lys Thr Ser Thr Gly Lys Glu Asn Gln Asp Glu
145                 150                 155                 160

Asn Cys Ser Gly Val Ser Thr Val Asn Lys Tyr Pro Leu Pro Thr Lys
                165                 170                 175

Gln Val Ser Gly Asp Ile Glu Thr Ser Lys Thr Ser Thr Val Asp Asn
            180                 185                 190

Ala Val Gln Asp Val Pro Lys Lys Asn Lys Asp Lys Asp Gly Asn Asp
        195                 200                 205

Gly Thr Thr Val His Ser Met Gln Asn Tyr Pro Trp His Phe His Ala
    210                 215                 220

Asp Ile Val Asn Gly Asn Ile Ala Lys Cys Pro Gln Asn His Pro Ser
225                 230                 235                 240

Gly Met Val Ser Gln Asp Phe Met Phe His Pro Met Arg Glu Glu Thr
                245                 250                 255

His Gly His Ala Asn Leu Gln Ala Thr Thr Ala Ser Ala Thr Thr Thr
            260                 265                 270

Ala Ser His Gln Ala Phe Pro Ala Cys His Ser Gln Asp Asp Tyr Arg
        275                 280                 285

Ser Phe Leu Gln Ile Ser Ser Thr Phe Ser Asn Leu Ile Met Ser Thr
    290                 295                 300

Leu Leu Gln Asn Pro Ala Ala His Ala Ala Ala Thr Phe Ala Ala Ser
305                 310                 315                 320

Val Trp Pro Tyr Ala Ser Val Gly Asn Ser Gly Asp Ser Ser Thr Pro
                325                 330                 335

Met Ser Ser Ser Pro Pro Ser Ile Thr Ala Ile Ala Ala Ala Thr Val
            340                 345                 350

Ala Ala Ala Thr Ala Trp Trp Ala Ser His Gly Leu Leu Pro Val Cys
        355                 360                 365

Ala Pro Ala Pro Ile Thr Cys Val Pro Phe Ser Thr Val Ala Val Pro
    370                 375                 380

Thr Pro Ala Met Thr Glu Met Asp Thr Val Glu Asn Thr Gln Pro Phe
385                 390                 395                 400

Glu Lys Gln Asn Thr Ala Leu Gln Asp Gln Thr Leu Ala Ser Lys Ser
                405                 410                 415

Pro Ala Ser Ser Ser Asp Asp Ser Asp Glu Thr Gly Val Thr Lys Leu
            420                 425                 430

Asn Ala Asp Ser Lys Thr Asn Asp Asp Lys Ile Glu Glu Val Val Val
        435                 440                 445

Thr Ala Ala Val His Asp Ser Asn Thr Ala Gln Lys Lys Asn Leu Val
    450                 455                 460

Asp Arg Ser Ser Cys Gly Ser Asn Thr Pro Ser Gly Ser Asp Ala Glu
465                 470                 475                 480

Thr Asp Ala Leu Asp Lys Met Glu Lys Asp Lys Glu Asp Val Lys Glu
                485                 490                 495

Thr Asp Glu Asn Gln Pro Asp Val Ile Glu Leu Asn Asn Arg Lys Ile
            500                 505                 510

Lys Met Arg Asp Asn Asn Ser Asn Asn Asn Ala Thr Thr Asp Ser Trp
        515                 520                 525

Lys Glu Val Ser Glu Glu Gly Arg Ile Ala Phe Gln Ala Leu Phe Ala

530                 535                 540

Arg Glu Arg Leu Pro Gln Ser Phe Ser Pro Pro Gln Val Ala Glu Asn
545                 550                 555                 560

Val Asn Arg Lys Gln Ser Asp Thr Ser Met Pro Leu Ala Pro Asn Phe
                565                 570                 575

Lys Ser Gln Asp Ser Cys Ala Ala Asp Gln Glu Gly Val Val Met Ile
            580                 585                 590

Gly Val Gly Thr Cys Lys Ser Leu Lys Thr Arg Gln Thr Gly Phe Lys
        595                 600                 605

Pro Tyr Lys Arg Cys Ser Met Glu Val Lys Glu Ser Gln Val Gly Asn
    610                 615                 620

Ile Asn Asn Gln Ser Asp Glu Lys Val Cys Lys Arg Leu Arg Leu Glu
625                 630                 635                 640

Gly Glu Ala Ser Thr
                645

<210>  21
<211>  1281
<212>  DNA
<213>  Arabidopsis thaliana

<220>
<221>  CDS
<222>  (64)..(1098)
<223>  G867

<400>  21
cacaacacaa acacatttct gttttctcca ttgtttcaaa ccataaaaaa aaacacagat      60

taa atg gaa tcg agt agc gtt gat gag agt act aca agt aca ggt tcc      108
    Met Glu Ser Ser Ser Val Asp Glu Ser Thr Thr Ser Thr Gly Ser
    1               5                   10                  15

atc tgt gaa acc ccg gcg ata act ccg gcg aaa aag tcg tcg gta ggt      156
Ile Cys Glu Thr Pro Ala Ile Thr Pro Ala Lys Lys Ser Ser Val Gly
                20                  25                  30

aac tta tac agg atg gga agc gga tca agc gtt gtg tta gat tca gag      204
Asn Leu Tyr Arg Met Gly Ser Gly Ser Ser Val Val Leu Asp Ser Glu
            35                  40                  45

aac ggc gta gaa gct gaa tct agg aag ctt ccg tcg tca aaa tac aaa      252
Asn Gly Val Glu Ala Glu Ser Arg Lys Leu Pro Ser Ser Lys Tyr Lys
        50                  55                  60

ggt gtg gtg cca caa cca aac gga aga tgg gga gct cag att tac gag      300
Gly Val Val Pro Gln Pro Asn Gly Arg Trp Gly Ala Gln Ile Tyr Glu
    65                  70                  75

aaa cac cag cgc gtg tgg ctc ggg aca ttc aac gaa gaa gac gaa gcc      348
Lys His Gln Arg Val Trp Leu Gly Thr Phe Asn Glu Glu Asp Glu Ala
80                  85                  90                  95

gct cgt gcc tac gac gtc gcg gtt cac agg ttc cgt cgc cgt gac gcc      396
Ala Arg Ala Tyr Asp Val Ala Val His Arg Phe Arg Arg Arg Asp Ala
                100                 105                 110

gtc aca aat ttc aaa gac gtg aag atg gac gaa gac gag gtc gat ttc      444
Val Thr Asn Phe Lys Asp Val Lys Met Asp Glu Asp Glu Val Asp Phe

115                 120                 125

ttg aat tct cat tcg aaa tct gag atc gtt gat atg ttg agg aaa cat      492
Leu Asn Ser His Ser Lys Ser Glu Ile Val Asp Met Leu Arg Lys His
        130                 135                 140

act tat aac gaa gag tta gag cag agt aaa cgg cgt cgt aat ggt aac      540
Thr Tyr Asn Glu Glu Leu Glu Gln Ser Lys Arg Arg Arg Asn Gly Asn
    145                 150                 155

gga aac atg act agg acg ttg tta acg tcg ggg ttg agt aat gat ggt      588
Gly Asn Met Thr Arg Thr Leu Leu Thr Ser Gly Leu Ser Asn Asp Gly
160                 165                 170                 175

gtt tct acg acg ggg ttt aga tcg gcg gag gca ctg ttt gag aaa gcg      636
Val Ser Thr Thr Gly Phe Arg Ser Ala Glu Ala Leu Phe Glu Lys Ala
                180                 185                 190

gta acg cca agc gac gtt ggg aag cta aac cgt ttg gtt ata ccg aaa      684
Val Thr Pro Ser Asp Val Gly Lys Leu Asn Arg Leu Val Ile Pro Lys
            195                 200                 205

cat cac gca gag aaa cat ttt ccg tta ccg tca agt aac gtt tcc gtg      732
His His Ala Glu Lys His Phe Pro Leu Pro Ser Ser Asn Val Ser Val
        210                 215                 220

aaa gga gtg ttg ttg aac ttt gag gac gtt aac ggg aaa gtg tgg agg      780
Lys Gly Val Leu Leu Asn Phe Glu Asp Val Asn Gly Lys Val Trp Arg
    225                 230                 235

ttc cgt tac tcg tat tgg aac agt agt cag agt tat gtt ttg act aaa      828
Phe Arg Tyr Ser Tyr Trp Asn Ser Ser Gln Ser Tyr Val Leu Thr Lys
240                 245                 250                 255

ggt tgg agc agg ttc gtt aag gag aag aat cta cgt gct ggt gac gtg      876
Gly Trp Ser Arg Phe Val Lys Glu Lys Asn Leu Arg Ala Gly Asp Val
                260                 265                 270

gtt agt ttc agt aga tct aac ggt cag gat caa cag ttg tac att ggg      924
Val Ser Phe Ser Arg Ser Asn Gly Gln Asp Gln Gln Leu Tyr Ile Gly
            275                 280                 285

tgg aag tcg aga tcc ggg tca gat tta gat gcg ggt cgg gtt ttg aga      972
Trp Lys Ser Arg Ser Gly Ser Asp Leu Asp Ala Gly Arg Val Leu Arg
        290                 295                 300

ttg ttc gga gtt aac att tca ccg gag agt tca aga aac gac gtc gta      1020
Leu Phe Gly Val Asn Ile Ser Pro Glu Ser Ser Arg Asn Asp Val Val
    305                 310                 315

gga aac aaa aga gtg aac gat act gag atg tta tcg ttg gtg tgt agc      1068
Gly Asn Lys Arg Val Asn Asp Thr Glu Met Leu Ser Leu Val Cys Ser
320                 325                 330                 335

aag aag caa cgc atc ttt cac gcc tcg taa caactcttct tctttttttt      1118
Lys Lys Gln Arg Ile Phe His Ala Ser
                340

tcttttgttg ttttaataat ttttaaaaac tccattttcg ttttctttat ttgcatcggt      1178

ttctttcttc ttgtttacca aaggttcatg agttgttttt gttgtattga tgaactgtaa      1238

attttattta taggataaat tttaaaaaaa aaaaaaaaaa aaa                        1281

<210>  22
<211>  344
<212>  PRT
<213>  Arabidopsis thaliana

<400>  22

Met Glu Ser Ser Ser Val Asp Glu Ser Thr Thr Ser Thr Gly Ser Ile
1               5                   10                  15

Cys Glu Thr Pro Ala Ile Thr Pro Ala Lys Lys Ser Ser Val Gly Asn
            20                  25                  30

Leu Tyr Arg Met Gly Ser Gly Ser Ser Val Val Leu Asp Ser Glu Asn
        35                  40                  45

Gly Val Glu Ala Glu Ser Arg Lys Leu Pro Ser Ser Lys Tyr Lys Gly
    50                  55                  60

Val Val Pro Gln Pro Asn Gly Arg Trp Gly Ala Gln Ile Tyr Glu Lys
65                  70                  75                  80

His Gln Arg Val Trp Leu Gly Thr Phe Asn Glu Glu Asp Glu Ala Ala
                85                  90                  95

Arg Ala Tyr Asp Val Ala Val His Arg Phe Arg Arg Arg Asp Ala Val
            100                 105                 110

Thr Asn Phe Lys Asp Val Lys Met Asp Glu Asp Glu Val Asp Phe Leu
        115                 120                 125

Asn Ser His Ser Lys Ser Glu Ile Val Asp Met Leu Arg Lys His Thr
    130                 135                 140

Tyr Asn Glu Glu Leu Glu Gln Ser Lys Arg Arg Arg Asn Gly Asn Gly
145                 150                 155                 160

Asn Met Thr Arg Thr Leu Leu Thr Ser Gly Leu Ser Asn Asp Gly Val
                165                 170                 175

Ser Thr Thr Gly Phe Arg Ser Ala Glu Ala Leu Phe Glu Lys Ala Val
            180                 185                 190

Thr Pro Ser Asp Val Gly Lys Leu Asn Arg Leu Val Ile Pro Lys His
        195                 200                 205

His Ala Glu Lys His Phe Pro Leu Pro Ser Ser Asn Val Ser Val Lys
    210                 215                 220

Gly Val Leu Leu Asn Phe Glu Asp Val Asn Gly Lys Val Trp Arg Phe
225                 230                 235                 240

Arg Tyr Ser Tyr Trp Asn Ser Ser Gln Ser Tyr Val Leu Thr Lys Gly
                245                 250                 255

Trp Ser Arg Phe Val Lys Glu Lys Asn Leu Arg Ala Gly Asp Val Val
            260                 265                 270

Ser Phe Ser Arg Ser Asn Gly Gln Asp Gln Gln Leu Tyr Ile Gly Trp
        275                 280                 285

Lys Ser Arg Ser Gly Ser Asp Leu Asp Ala Gly Arg Val Leu Arg Leu
    290                 295                 300

Phe Gly Val Asn Ile Ser Pro Glu Ser Ser Arg Asn Asp Val Val Gly
305                 310                 315                 320

Asn Lys Arg Val Asn Asp Thr Glu Met Leu Ser Leu Val Cys Ser Lys
                325                 330                 335

Lys Gln Arg Ile Phe His Ala Ser
            340

<210> 23
<211> 724
<212> DNA
<213> Arabidopsis thaliana

<220>
<221> CDS
<222> (20)..(694)
<223> G912

<400> 23
catcttatcc aaagaaaaa atg aat cca ttt tac tct aca ttc cca gac tcg 52
Met Asn Pro Phe Tyr Ser Thr Phe Pro Asp Ser
1               5                   10

ttt ctc tca atc tcc gat cat aga tct ccg gtt tca gac agt agt gag 100
Phe Leu Ser Ile Ser Asp His Arg Ser Pro Val Ser Asp Ser Ser Glu
            15                  20                  25

tgt tca cca aag tta gct tca agt tgt cca aag aaa cga gct ggg agg 148
Cys Ser Pro Lys Leu Ala Ser Ser Cys Pro Lys Lys Arg Ala Gly Arg
        30                  35                  40

aag aag ttt cgt gag aca cgt cat ccg att tac aga gga gtt cgt cag 196
Lys Lys Phe Arg Glu Thr Arg His Pro Ile Tyr Arg Gly Val Arg Gln
    45                  50                  55

agg aat tct ggt aaa tgg gtt tgt gaa gtt aga gag cct aat aag aaa 244
Arg Asn Ser Gly Lys Trp Val Cys Glu Val Arg Glu Pro Asn Lys Lys
60                  65                  70                  75

tct agg att tgg tta ggt act ttt ccg acg gtt gaa atg gct gct cgt 292
Ser Arg Ile Trp Leu Gly Thr Phe Pro Thr Val Glu Met Ala Ala Arg
                80                  85                  90

gct cat gat gtt gct gct tta gct ctt cgt ggt cgc tct gct tgt ctc 340
Ala His Asp Val Ala Ala Leu Ala Leu Arg Gly Arg Ser Ala Cys Leu
            95                  100                 105

aat ttc gct gat tct gct tgg cgg ctt cgt att cct gag act act tgt 388
Asn Phe Ala Asp Ser Ala Trp Arg Leu Arg Ile Pro Glu Thr Thr Cys
        110                 115                 120

cct aag gag att cag aaa gct gcg tct gaa gct gca atg gcg ttt cag 436
Pro Lys Glu Ile Gln Lys Ala Ala Ser Glu Ala Ala Met Ala Phe Gln
    125                 130                 135

aat gag act acg acg gag gga tct aaa act gcg gcg gag gca gag gag 484
Asn Glu Thr Thr Thr Glu Gly Ser Lys Thr Ala Ala Glu Ala Glu Glu
140                 145                 150                 155

gcg gca ggg gag ggg gtg agg gag ggg gag agg agg gcg gag gag cag 532
Ala Ala Gly Glu Gly Val Arg Glu Gly Glu Arg Arg Ala Glu Glu Gln
                160                 165                 170

aat ggt ggt gtg ttt tat atg gat gat gag gcg ctt ttg ggg atg ccc 580
Asn Gly Gly Val Phe Tyr Met Asp Asp Glu Ala Leu Leu Gly Met Pro
            175                 180                 185

aac ttt ttt gag aat atg gcg gag ggg atg ctt ttg ccg ccg ccg gaa 628
Asn Phe Phe Glu Asn Met Ala Glu Gly Met Leu Leu Pro Pro Pro Glu
        190                 195                 200

gtt ggc tgg aat cat aac gac ttt gac gga gtg ggt gac gtg tca ctc 676

Val Gly Trp Asn His Asn Asp Phe Asp Gly Val Gly Asp Val Ser Leu
    205                 210                 215

tgg agt ttt gac gag taa tttttggct ctttttctgg ataataagtt          724
Trp Ser Phe Asp Glu
220

<210>  24
<211>  224
<212>  PRT
<213>  Arabidopsis thaliana

<400>  24

Met Asn Pro Phe Tyr Ser Thr Phe Pro Asp Ser Phe Leu Ser Ile Ser
1               5                   10                  15

Asp His Arg Ser Pro Val Ser Asp Ser Ser Glu Cys Ser Pro Lys Leu
            20                  25                  30

Ala Ser Ser Cys Pro Lys Lys Arg Ala Gly Arg Lys Lys Phe Arg Glu
        35                  40                  45

Thr Arg His Pro Ile Tyr Arg Gly Val Arg Gln Arg Asn Ser Gly Lys
    50                  55                  60

Trp Val Cys Glu Val Arg Glu Pro Asn Lys Lys Ser Arg Ile Trp Leu
65                  70                  75                  80

Gly Thr Phe Pro Thr Val Glu Met Ala Ala Arg Ala His Asp Val Ala
                85                  90                  95

Ala Leu Ala Leu Arg Gly Arg Ser Ala Cys Leu Asn Phe Ala Asp Ser
            100                 105                 110

Ala Trp Arg Leu Arg Ile Pro Glu Thr Thr Cys Pro Lys Glu Ile Gln
        115                 120                 125

Lys Ala Ala Ser Glu Ala Ala Met Ala Phe Gln Asn Glu Thr Thr Thr
    130                 135                 140

Glu Gly Ser Lys Thr Ala Ala Glu Ala Glu Glu Ala Ala Gly Glu Gly
145                 150                 155                 160

Val Arg Glu Gly Glu Arg Arg Ala Glu Glu Gln Asn Gly Gly Val Phe
                165                 170                 175

Tyr Met Asp Asp Glu Ala Leu Leu Gly Met Pro Asn Phe Phe Glu Asn
            180                 185                 190

Met Ala Glu Gly Met Leu Leu Pro Pro Pro Glu Val Gly Trp Asn His
        195                 200                 205

Asn Asp Phe Asp Gly Val Gly Asp Val Ser Leu Trp Ser Phe Asp Glu
    210                 215                 220

<210>  25
<211>  1082
<212>  DNA
<213>  Arabidopsis thaliana

<220>
<221> CDS
<222> (53)..(1063)
<223> G996

<400> 25
cgatcgatct tgaattgatt ctttgtagta ttttatttac atatatatat ag atg gga 58
Met Gly
1

aga cat tca tgt tgt tac aaa cag aaa ctg agg aaa gga ctt tgg tct 106
Arg His Ser Cys Cys Tyr Lys Gln Lys Leu Arg Lys Gly Leu Trp Ser
        5                   10                  15

cct gaa gaa gat gag aag ctt ctt cgt tac atc act aag tat ggt cat 154
Pro Glu Glu Asp Glu Lys Leu Leu Arg Tyr Ile Thr Lys Tyr Gly His
    20                  25                  30

ggt tgc tgg agc tct gtc cct aaa caa gct ggt tta cag aga tgt gga 202
Gly Cys Trp Ser Ser Val Pro Lys Gln Ala Gly Leu Gln Arg Cys Gly
35                  40                  45                  50

aaa agt tgt aga tta aga tgg ata aat tat tta aga cca gat ttg aag 250
Lys Ser Cys Arg Leu Arg Trp Ile Asn Tyr Leu Arg Pro Asp Leu Lys
                55                  60                  65

aga gga gca ttt tct caa gat gaa gaa aat ctc att att gaa ctt cat 298
Arg Gly Ala Phe Ser Gln Asp Glu Glu Asn Leu Ile Ile Glu Leu His
            70                  75                  80

gcc gtt ctt ggc aat aga tgg tct cag ata gct gca cag ctt cct gga 346
Ala Val Leu Gly Asn Arg Trp Ser Gln Ile Ala Ala Gln Leu Pro Gly
        85                  90                  95

aga acc gac aat gaa atc aag aat ctt tgg aat tct tgt ttg aag aag 394
Arg Thr Asp Asn Glu Ile Lys Asn Leu Trp Asn Ser Cys Leu Lys Lys
    100                 105                 110

aaa ttg agg ctg aga gga att gac ccg gtt aca cac aag ctc tta acc 442
Lys Leu Arg Leu Arg Gly Ile Asp Pro Val Thr His Lys Leu Leu Thr
115                 120                 125                 130

gaa atc gaa acc ggt aca gat gac aaa aca aaa ccg gtt gag aag agt 490
Glu Ile Glu Thr Gly Thr Asp Asp Lys Thr Lys Pro Val Glu Lys Ser
                135                 140                 145

caa cag acc tac ctc gtt gag act gat ggc tcc tct agt acc act act 538
Gln Gln Thr Tyr Leu Val Glu Thr Asp Gly Ser Ser Ser Thr Thr Thr
            150                 155                 160

tgt agt act aac caa aac aac aac act gat cat ctt tat acc gga aat 586
Cys Ser Thr Asn Gln Asn Asn Asn Thr Asp His Leu Tyr Thr Gly Asn
        165                 170                 175

ttc ggt ttt caa cgg tta agt cta gaa aac ggt tca aga atc gca gcc 634
Phe Gly Phe Gln Arg Leu Ser Leu Glu Asn Gly Ser Arg Ile Ala Ala
    180                 185                 190

ggt tct gac ctc ggt atc tgg att ccc caa acc gga aga aac cat cat 682
Gly Ser Asp Leu Gly Ile Trp Ile Pro Gln Thr Gly Arg Asn His His
195                 200                 205                 210

cat cat gtc gat gaa acc atc cct agt gca gtg gta cta ccc ggt tca 730
His His Val Asp Glu Thr Ile Pro Ser Ala Val Val Leu Pro Gly Ser
                215                 220                 225

atg ttc tca tcc ggt tta acc ggt tat aga tcc tcc aat ctc ggt tta 778
Met Phe Ser Ser Gly Leu Thr Gly Tyr Arg Ser Ser Asn Leu Gly Leu
            230                 235                 240

att gaa ttg gaa aac tca ttc tca acc ggg cca atg atg aca gag cat 826
Ile Glu Leu Glu Asn Ser Phe Ser Thr Gly Pro Met Met Thr Glu His
        245                 250                 255

cag caa att caa gag agt aac tac aac aat tca aca ttc ttt gga aat 874
Gln Gln Ile Gln Glu Ser Asn Tyr Asn Asn Ser Thr Phe Phe Gly Asn
    260                 265                 270

ggg aat ctg aat tgg gga tta aca atg gag gaa aat caa aat cca ttc 922
Gly Asn Leu Asn Trp Gly Leu Thr Met Glu Glu Asn Gln Asn Pro Phe
275                 280                 285                 290

aca ata tcg aat cat tca aat tcg tcc tta tac agt gat ata aaa tca 970
Thr Ile Ser Asn His Ser Asn Ser Ser Leu Tyr Ser Asp Ile Lys Ser
                295                 300                 305

gag acc aat ttt ttt ggc aca gag gct aca aat gtt ggt atg tgg cca 1018
Glu Thr Asn Phe Phe Gly Thr Glu Ala Thr Asn Val Gly Met Trp Pro
            310                 315                 320

tgt aac cag ctt cag cct cag caa cat gca tat ggc cat ata taa 1063
Cys Asn Gln Leu Gln Pro Gln Gln His Ala Tyr Gly His Ile
        325                 330                 335

atcttcttgt atattataa 1082

<210> 26
<211> 336
<212> PRT
<213> Arabidopsis thaliana

<400> 26

Met Gly Arg His Ser Cys Cys Tyr Lys Gln Lys Leu Arg Lys Gly Leu
1               5                   10                  15

Trp Ser Pro Glu Glu Asp Glu Lys Leu Leu Arg Tyr Ile Thr Lys Tyr
            20                  25                  30

Gly His Gly Cys Trp Ser Ser Val Pro Lys Gln Ala Gly Leu Gln Arg
        35                  40                  45

Cys Gly Lys Ser Cys Arg Leu Arg Trp Ile Asn Tyr Leu Arg Pro Asp
    50                  55                  60

Leu Lys Arg Gly Ala Phe Ser Gln Asp Glu Glu Asn Leu Ile Ile Glu
65                  70                  75                  80

Leu His Ala Val Leu Gly Asn Arg Trp Ser Gln Ile Ala Ala Gln Leu
                85                  90                  95

Pro Gly Arg Thr Asp Asn Glu Ile Lys Asn Leu Trp Asn Ser Cys Leu
            100                 105                 110

Lys Lys Lys Leu Arg Leu Arg Gly Ile Asp Pro Val Thr His Lys Leu
        115                 120                 125

Leu Thr Glu Ile Glu Thr Gly Thr Asp Asp Lys Thr Lys Pro Val Glu
    130                 135                 140

Lys Ser Gln Gln Thr Tyr Leu Val Glu Thr Asp Gly Ser Ser Ser Thr
145                 150                 155                 160

Thr Thr Cys Ser Thr Asn Gln Asn Asn Asn Thr Asp His Leu Tyr Thr
                165                 170                 175

Gly Asn Phe Gly Phe Gln Arg Leu Ser Leu Glu Asn Gly Ser Arg Ile
        180                 185                 190

Ala Ala Gly Ser Asp Leu Gly Ile Trp Ile Pro Gln Thr Gly Arg Asn
    195                 200                 205

His His His His Val Asp Glu Thr Ile Pro Ser Ala Val Val Leu Pro
    210                 215                 220

Gly Ser Met Phe Ser Ser Gly Leu Thr Gly Tyr Arg Ser Ser Asn Leu
225                 230                 235                 240

Gly Leu Ile Glu Leu Glu Asn Ser Phe Ser Thr Gly Pro Met Met Thr
                245                 250                 255

Glu His Gln Gln Ile Gln Glu Ser Asn Tyr Asn Asn Ser Thr Phe Phe
            260                 265                 270

Gly Asn Gly Asn Leu Asn Trp Gly Leu Thr Met Glu Glu Asn Gln Asn
        275                 280                 285

Pro Phe Thr Ile Ser Asn His Ser Asn Ser Ser Leu Tyr Ser Asp Ile
    290                 295                 300

Lys Ser Glu Thr Asn Phe Phe Gly Thr Glu Ala Thr Asn Val Gly Met
305                 310                 315                 320

Trp Pro Cys Asn Gln Leu Gln Pro Gln Gln His Ala Tyr Gly His Ile
                325                 330                 335

<210>  27
<211>  1606
<212>  DNA
<213>  Arabidopsis thaliana

<220>
<221>  CDS
<222>  (150)..(1310)
<223>  G1068

<400>  27
gagagttgtt agctagctca cacgctttcg cttaaaactc aaaaacctgc actttctcgt      60

ctattttctc ggcattcgta aaacagaaaa gtgggtctcc aagaaaatta ccctaaattc     120

acaaagattc atacttttct ccacctcca atg gat tcc aga gag atc cac cac       173
                                Met Asp Ser Arg Glu Ile His His
                                1               5

caa caa cag caa caa caa caa caa caa cag cag cag cag caa caa cag       221
Gln Gln Gln Gln Gln Gln Gln Gln Gln Gln Gln Gln Gln Gln Gln Gln
    10                  15                  20

caa cat cta caa caa cag caa caa cca ccg cca ggg atg tta atg agt       269
Gln His Leu Gln Gln Gln Gln Gln Pro Pro Pro Gly Met Leu Met Ser
25                  30                  35                  40

cac cac aat tcc tac aat cga aac cct aac gcc gcc gcc gct gtt tta       317
His His Asn Ser Tyr Asn Arg Asn Pro Asn Ala Ala Ala Ala Val Leu
                45                  50                  55

atg ggt cac aac acc tcc aca tct caa gct atg cat caa aga tta cct       365
Met Gly His Asn Thr Ser Thr Ser Gln Ala Met His Gln Arg Leu Pro
            60                  65                  70

ttt ggt ggt tct atg tca ccg cat cag cct caa caa cat cag tat cat 413
Phe Gly Gly Ser Met Ser Pro His Gln Pro Gln Gln His Gln Tyr His
        75                  80                  85

cat cct cag cct cag caa cag ata gat cag aag act ctt gaa tct ctt 461
His Pro Gln Pro Gln Gln Gln Ile Asp Gln Lys Thr Leu Glu Ser Leu
    90                  95                  100

gga ttt cct act tcg cct ctt cct tct gct tct aat tct tac ggt ggt 509
Gly Phe Pro Thr Ser Pro Leu Pro Ser Ala Ser Asn Ser Tyr Gly Gly
105                 110                 115                 120

gga aat gaa gga ggt ggt ggt ggt gat agc gcc gga gct aat gct aac 557
Gly Asn Glu Gly Gly Gly Gly Gly Asp Ser Ala Gly Ala Asn Ala Asn
                125                 130                 135

tct tcc gat cca cct gct aaa cgg aac aga gga cgt cct cct ggc tcc 605
Ser Ser Asp Pro Pro Ala Lys Arg Asn Arg Gly Arg Pro Pro Gly Ser
            140                 145                 150

ggt aag aag cag ctc gat gct tta gga gga aca gga gga gtt ggg ttc 653
Gly Lys Lys Gln Leu Asp Ala Leu Gly Gly Thr Gly Gly Val Gly Phe
        155                 160                 165

acg cct cat gtc att gag gtt aaa aca gga gag gac ata gct acg aag 701
Thr Pro His Val Ile Glu Val Lys Thr Gly Glu Asp Ile Ala Thr Lys
    170                 175                 180

ata ttg gcg ttt acg aac caa ggg cca cgc gca atc tgt att ctc tca 749
Ile Leu Ala Phe Thr Asn Gln Gly Pro Arg Ala Ile Cys Ile Leu Ser
185                 190                 195                 200

gct aca gga gct gta act aat gtg atg ctt cgt caa gct aac aat agc 797
Ala Thr Gly Ala Val Thr Asn Val Met Leu Arg Gln Ala Asn Asn Ser
                205                 210                 215

aat cct act gga act gtt aag tat gag ggc cga ttt gaa atc att tct 845
Asn Pro Thr Gly Thr Val Lys Tyr Glu Gly Arg Phe Glu Ile Ile Ser
            220                 225                 230

ctg tca ggt tct ttc ttg aat tct gag agt aat ggt act gtg acc aaa 893
Leu Ser Gly Ser Phe Leu Asn Ser Glu Ser Asn Gly Thr Val Thr Lys
        235                 240                 245

act ggt aac ttg agt gtg tcg ctg gct gga cac gaa ggc cgg att gtg 941
Thr Gly Asn Leu Ser Val Ser Leu Ala Gly His Glu Gly Arg Ile Val
    250                 255                 260

ggt gga tgt gtt gat gga atg cta gta gct gga tca caa gtc cag gtc 989
Gly Gly Cys Val Asp Gly Met Leu Val Ala Gly Ser Gln Val Gln Val
265                 270                 275                 280

att gtg gga agc ttt gta cca gat gga agg aag cag aaa caa agt gcg 1037
Ile Val Gly Ser Phe Val Pro Asp Gly Arg Lys Gln Lys Gln Ser Ala
                285                 290                 295

ggg cgt gct cag aat act ccg gag cca gct tca gca cca gcc aat atg 1085
Gly Arg Ala Gln Asn Thr Pro Glu Pro Ala Ser Ala Pro Ala Asn Met
            300                 305                 310

ttg agc ttt ggt ggt gtt ggt gga ccg gga agc cct cga tct caa gga 1133
Leu Ser Phe Gly Gly Val Gly Gly Pro Gly Ser Pro Arg Ser Gln Gly
        315                 320                 325

caa caa cac tcg agc gag tca tca gag gaa aac gaa agt aat tct ccg 1181
Gln Gln His Ser Ser Glu Ser Ser Glu Glu Asn Glu Ser Asn Ser Pro
    330                 335                 340

ttg cac cgt aga agc aac aac aac aac agc aac aat cat ggg ata ttt 1229
Leu His Arg Arg Ser Asn Asn Asn Asn Ser Asn Asn His Gly Ile Phe
345                 350                 355                 360

gga aac tct aca cct caa ccg ctt cac caa att cct atg cag atg tac 1277
Gly Asn Ser Thr Pro Gln Pro Leu His Gln Ile Pro Met Gln Met Tyr

                365                 370                 375

cag aat ctc tgg cct ggc aac agt cct caa taa acagatggtt catgggtcaa 1330
Gln Asn Leu Trp Pro Gly Asn Ser Pro Gln
            380                 385

gatttgaccg ggtttgcttc tctgttcctt ttgacacatc tctccatcag atttatctct 1390

ataaagtaga ttgagctctc ttactctctc atcttcttct cctttactat ttctcttaaa 1450

tttagctttg gttttagata aatagagaga gagagacatg ttaagtaggt ttcaaattca 1510

atcttgttta gtttgtttct tagtagtttc ttttgattgt gatgatcata aagacttgtt 1570

ctttttctcc tatattcaac gaattatcca ctttaa 1606

<210> 28
<211> 386
<212> PRT
<213> Arabidopsis thaliana

<400> 28

Met Asp Ser Arg Glu Ile His His Gln Gln Gln Gln Gln Gln Gln Gln
1               5                   10                  15

Gln Gln Gln Gln Gln Gln Gln Gln Gln His Leu Gln Gln Gln Gln Gln
            20                  25                  30

Pro Pro Pro Gly Met Leu Met Ser His His Asn Ser Tyr Asn Arg Asn
        35                  40                  45

Pro Asn Ala Ala Ala Ala Val Leu Met Gly His Asn Thr Ser Thr Ser
    50                  55                  60

Gln Ala Met His Gln Arg Leu Pro Phe Gly Gly Ser Met Ser Pro His
65                  70                  75                  80

Gln Pro Gln Gln His Gln Tyr His His Pro Gln Pro Gln Gln Gln Ile
                85                  90                  95

Asp Gln Lys Thr Leu Glu Ser Leu Gly Phe Pro Thr Ser Pro Leu Pro
            100                 105                 110

Ser Ala Ser Asn Ser Tyr Gly Gly Gly Asn Glu Gly Gly Gly Gly Gly
        115                 120                 125

Asp Ser Ala Gly Ala Asn Ala Asn Ser Ser Asp Pro Pro Ala Lys Arg
    130                 135                 140

Asn Arg Gly Arg Pro Pro Gly Ser Gly Lys Lys Gln Leu Asp Ala Leu
145                 150                 155                 160

Gly Gly Thr Gly Gly Val Gly Phe Thr Pro His Val Ile Glu Val Lys
                165                 170                 175

Thr Gly Glu Asp Ile Ala Thr Lys Ile Leu Ala Phe Thr Asn Gln Gly
            180                 185                 190

Pro Arg Ala Ile Cys Ile Leu Ser Ala Thr Gly Ala Val Thr Asn Val
        195                 200                 205

Met Leu Arg Gln Ala Asn Asn Ser Asn Pro Thr Gly Thr Val Lys Tyr
    210                 215                 220

Glu Gly Arg Phe Glu Ile Ile Ser Leu Ser Gly Ser Phe Leu Asn Ser
225                 230                 235                 240

Glu Ser Asn Gly Thr Val Thr Lys Thr Gly Asn Leu Ser Val Ser Leu
                245                 250                 255

Ala Gly His Glu Gly Arg Ile Val Gly Gly Cys Val Asp Gly Met Leu
            260                 265                 270

Val Ala Gly Ser Gln Val Gln Val Ile Val Gly Ser Phe Val Pro Asp
        275                 280                 285

Gly Arg Lys Gln Lys Gln Ser Ala Gly Arg Ala Gln Asn Thr Pro Glu
    290                 295                 300

Pro Ala Ser Ala Pro Ala Asn Met Leu Ser Phe Gly Gly Val Gly Gly
305                 310                 315                 320

Pro Gly Ser Pro Arg Ser Gln Gly Gln Gln His Ser Ser Glu Ser Ser
                325                 330                 335

Glu Glu Asn Glu Ser Asn Ser Pro Leu His Arg Arg Ser Asn Asn Asn
            340                 345                 350

Asn Ser Asn Asn His Gly Ile Phe Gly Asn Ser Thr Pro Gln Pro Leu
        355                 360                 365

His Gln Ile Pro Met Gln Met Tyr Gln Asn Leu Trp Pro Gly Asn Ser
    370                 375                 380

Pro Gln
385

<210>  29
<211>  1630
<212>  DNA
<213>  Arabidopsis thaliana

<220>
<221>  CDS
<222>  (97)..(1398)
<223>  G1337

<400>  29
aatggatttg tcatcattct tctcaccgtc cttagtctct gaaaataaat tctgattttg      60

atttcgaatt ttagggattt tgagagagag tcagtt atg agt agt tcg gag aga      114
                                        Met Ser Ser Ser Glu Arg
                                        1               5

gta ccg tgc gat ttc tgc ggc gag cgt acg gcg gtt ttg ttt tgt aga      162
Val Pro Cys Asp Phe Cys Gly Glu Arg Thr Ala Val Leu Phe Cys Arg
            10                  15                  20

gcc gat acg gcg aag ctg tgt ttg cct tgt gat cag caa gtt cac acg      210
Ala Asp Thr Ala Lys Leu Cys Leu Pro Cys Asp Gln Gln Val His Thr
        25                  30                  35

gcg aat ctg ttg tcg agg aag cac gtg cga tct cag atc tgc gat aat 258
Ala Asn Leu Leu Ser Arg Lys His Val Arg Ser Gln Ile Cys Asp Asn
    40                  45                  50

tgc ggt aac gag cca gtc tct gtt cgg tgt ttc acc gat aat ctg att 306
Cys Gly Asn Glu Pro Val Ser Val Arg Cys Phe Thr Asp Asn Leu Ile
55                  60                  65                  70

ttg tgt cag gag tgt gat tgg gat gtt cac gga agt tgt tca gtt tcc 354
Leu Cys Gln Glu Cys Asp Trp Asp Val His Gly Ser Cys Ser Val Ser
                75                  80                  85

gat gct cat gtt cga tcc gcc gtg gaa ggt ttt tcc ggt tgt cca tcg 402
Asp Ala His Val Arg Ser Ala Val Glu Gly Phe Ser Gly Cys Pro Ser
            90                  95                  100

gcg ttg gag ctt gct gct tta tgg gga ctt gat ttg gag caa ggg agg 450
Ala Leu Glu Leu Ala Ala Leu Trp Gly Leu Asp Leu Glu Gln Gly Arg
        105                 110                 115

aaa gat gaa gag aat caa gtt ccg atg atg gcg atg atg atg gat aat 498
Lys Asp Glu Glu Asn Gln Val Pro Met Met Ala Met Met Met Asp Asn
    120                 125                 130

ttc ggg atg cag ttg gat tct tgg gtt ttg gga tct aat gaa ttg att 546
Phe Gly Met Gln Leu Asp Ser Trp Val Leu Gly Ser Asn Glu Leu Ile
135                 140                 145                 150

gtt ccc agc gat acg acg ttt aag aag cgt gga tct tgt gga tct agt 594
Val Pro Ser Asp Thr Thr Phe Lys Lys Arg Gly Ser Cys Gly Ser Ser
                155                 160                 165

tgt ggg agg tat aag cag gta ttg tgt aag cag ctt gag gag ttg ctt 642
Cys Gly Arg Tyr Lys Gln Val Leu Cys Lys Gln Leu Glu Glu Leu Leu
            170                 175                 180

aag agt ggt gtt gtc ggt ggt gat ggc gat gat ggt gat cgt gac cgt 690
Lys Ser Gly Val Val Gly Gly Asp Gly Asp Asp Gly Asp Arg Asp Arg
        185                 190                 195

gat tgt gac cgt gag ggt gct tgt gat gga gat gga gat gga gaa gca 738
Asp Cys Asp Arg Glu Gly Ala Cys Asp Gly Asp Gly Asp Gly Glu Ala
    200                 205                 210

gga gag ggg ctt atg gtt ccg gag atg tca gag aga ttg aaa tgg tca 786
Gly Glu Gly Leu Met Val Pro Glu Met Ser Glu Arg Leu Lys Trp Ser
215                 220                 225                 230

aga gat gtt gag gag atc aat ggt ggc gga gga gga gga gtt aac cag 834
Arg Asp Val Glu Glu Ile Asn Gly Gly Gly Gly Gly Gly Val Asn Gln
                235                 240                 245

cag tgg aat gct act act act aat cct agt ggt ggc cag agt tct cag 882
Gln Trp Asn Ala Thr Thr Thr Asn Pro Ser Gly Gly Gln Ser Ser Gln
            250                 255                 260

ata tgg gat ttt aac ttg gga cag tca cgg gga cct gag gat acg agt 930
Ile Trp Asp Phe Asn Leu Gly Gln Ser Arg Gly Pro Glu Asp Thr Ser
        265                 270                 275

cga gtg gaa gct gca tat gta ggg aaa ggt gct gct tct tca ttc aca 978
Arg Val Glu Ala Ala Tyr Val Gly Lys Gly Ala Ala Ser Ser Phe Thr
    280                 285                 290

atc aac aat ttt gtt gac cat atg aat gaa act tgt tcc act aat gtg 1026
Ile Asn Asn Phe Val Asp His Met Asn Glu Thr Cys Ser Thr Asn Val
295                 300                 305                 310

aaa ggt gtc aaa gag att aaa aag gat gac tac aag cga tca act tca 1074
Lys Gly Val Lys Glu Ile Lys Lys Asp Asp Tyr Lys Arg Ser Thr Ser
                315                 320                 325

ggc cag gta caa cca aca aaa tct gag agc aac aat cgt cca att acc 1122
Gly Gln Val Gln Pro Thr Lys Ser Glu Ser Asn Asn Arg Pro Ile Thr
            330                 335                 340

ttt ggc tct gag aaa ggt tcg aac tcc tcc agt gac ttg cat ttc aca 1170
Phe Gly Ser Glu Lys Gly Ser Asn Ser Ser Ser Asp Leu His Phe Thr
        345                 350                 355

gag cat att gct gga act agt tgt aag acc aca aga cta gtt gca act 1218
Glu His Ile Ala Gly Thr Ser Cys Lys Thr Thr Arg Leu Val Ala Thr
    360                 365                 370

aag gct gat ctg gag cgg ctg gct cag aac aga gga gat gca atg cag 1266
Lys Ala Asp Leu Glu Arg Leu Ala Gln Asn Arg Gly Asp Ala Met Gln
375                 380                 385                 390

cgt tac aag gaa aag agg aag aca cgg aga tat gat aag acc ata agg 1314
Arg Tyr Lys Glu Lys Arg Lys Thr Arg Arg Tyr Asp Lys Thr Ile Arg
                395                 400                 405

tat gaa tcg agg aag gca aga gct gac act agg ttg cgt gtc aga ggc 1362
Tyr Glu Ser Arg Lys Ala Arg Ala Asp Thr Arg Leu Arg Val Arg Gly
            410                 415                 420

aga ttt gtg aaa gct agt gaa gct cct tac cct taa ccttaagttt 1408
Arg Phe Val Lys Ala Ser Glu Ala Pro Tyr Pro
        425                 430

tttcacatag gcttcctttt agctacaaac ttagttactt tttttactcc actgcctcat 1468

aaatgtacag accggtctcg tttcatctgg ccgcccttct tgttttattg ccttatctgg 1528

cccttttatg taccttggaa tcttatctag tttaaaaaag attgtaacct tctagaaaac 1588

catattctgt tgacagtata tacatgtcta tccaagcaaa aa 1630

<210> 30
<211> 433
<212> PRT
<213> Arabidopsis thaliana

<400> 30

Met Ser Ser Ser Glu Arg Val Pro Cys Asp Phe Cys Gly Glu Arg Thr
1               5                   10                  15

Ala Val Leu Phe Cys Arg Ala Asp Thr Ala Lys Leu Cys Leu Pro Cys
            20                  25                  30

Asp Gln Gln Val His Thr Ala Asn Leu Leu Ser Arg Lys His Val Arg
        35                  40                  45

Ser Gln Ile Cys Asp Asn Cys Gly Asn Glu Pro Val Ser Val Arg Cys
    50                  55                  60

Phe Thr Asp Asn Leu Ile Leu Cys Gln Glu Cys Asp Trp Asp Val His
65                  70                  75                  80

Gly Ser Cys Ser Val Ser Asp Ala His Val Arg Ser Ala Val Glu Gly
                85                  90                  95

Phe Ser Gly Cys Pro Ser Ala Leu Glu Leu Ala Ala Leu Trp Gly Leu
            100                 105                 110

Asp Leu Glu Gln Gly Arg Lys Asp Glu Glu Asn Gln Val Pro Met Met
        115                 120                 125

Ala Met Met Met Asp Asn Phe Gly Met Gln Leu Asp Ser Trp Val Leu
    130                 135                 140

Gly Ser Asn Glu Leu Ile Val Pro Ser Asp Thr Thr Phe Lys Lys Arg
145                 150                 155                 160

Gly Ser Cys Gly Ser Ser Cys Gly Arg Tyr Lys Gln Val Leu Cys Lys
                165                 170                 175

Gln Leu Glu Glu Leu Leu Lys Ser Gly Val Val Gly Gly Asp Gly Asp
            180                 185                 190

Asp Gly Asp Arg Asp Arg Asp Cys Asp Arg Glu Gly Ala Cys Asp Gly
        195                 200                 205

Asp Gly Asp Gly Glu Ala Gly Glu Gly Leu Met Val Pro Glu Met Ser
    210                 215                 220

Glu Arg Leu Lys Trp Ser Arg Asp Val Glu Glu Ile Asn Gly Gly Gly
225                 230                 235                 240

Gly Gly Gly Val Asn Gln Gln Trp Asn Ala Thr Thr Thr Asn Pro Ser
                245                 250                 255

Gly Gly Gln Ser Ser Gln Ile Trp Asp Phe Asn Leu Gly Gln Ser Arg
            260                 265                 270

Gly Pro Glu Asp Thr Ser Arg Val Glu Ala Ala Tyr Val Gly Lys Gly
        275                 280                 285

Ala Ala Ser Ser Phe Thr Ile Asn Asn Phe Val Asp His Met Asn Glu
    290                 295                 300

Thr Cys Ser Thr Asn Val Lys Gly Val Lys Glu Ile Lys Lys Asp Asp
305                 310                 315                 320

Tyr Lys Arg Ser Thr Ser Gly Gln Val Gln Pro Thr Lys Ser Glu Ser
                325                 330                 335

Asn Asn Arg Pro Ile Thr Phe Gly Ser Glu Lys Gly Ser Asn Ser Ser
            340                 345                 350

Ser Asp Leu His Phe Thr Glu His Ile Ala Gly Thr Ser Cys Lys Thr
        355                 360                 365

Thr Arg Leu Val Ala Thr Lys Ala Asp Leu Glu Arg Leu Ala Gln Asn
    370                 375                 380

Arg Gly Asp Ala Met Gln Arg Tyr Lys Glu Lys Arg Lys Thr Arg Arg
385                 390                 395                 400

Tyr Asp Lys Thr Ile Arg Tyr Glu Ser Arg Lys Ala Arg Ala Asp Thr
                405                 410                 415

Arg Leu Arg Val Arg Gly Arg Phe Val Lys Ala Ser Glu Ala Pro Tyr
            420                 425                 430

Pro

<210> 31
<211> 1413
<212> DNA
<213> Arabidopsis thaliana

<220>
<221> CDS
<222> (208)..(1200)
<223> G1141

<400> 31
gtctttacaa aaagaaaaga aaaaagagac gctagaaaga acgcgaaagc ttgcgaagaa 60

gatttgcttt tgatcgactt aacacgaaca acaaacaaca tctgcgtgat aaagaagaga 120

tttttgccta aataaagaag agattcgact ctaatcctgg agttatcatt cacgatagat 180

tcttagattg cgactataaa gaagaag atg gct gta tat gaa caa acc gga acc 234
Met Ala Val Tyr Glu Gln Thr Gly Thr
1               5

gag cag ccg aag aaa agg aaa tct agg gct cga gca ggt ggt tta acg 282
Glu Gln Pro Lys Lys Arg Lys Ser Arg Ala Arg Ala Gly Gly Leu Thr
10                  15                  20                  25

gtg gct gat agg cta aag aag tgg aaa gag tac aac gag att gtt gaa 330
Val Ala Asp Arg Leu Lys Lys Trp Lys Glu Tyr Asn Glu Ile Val Glu
                30                  35                  40

gct tcg gct gtt aaa gaa gga gag aaa ccg aaa cgc aaa gtt cct gcg 378
Ala Ser Ala Val Lys Glu Gly Glu Lys Pro Lys Arg Lys Val Pro Ala
            45                  50                  55

aaa ggg tcg aag aaa ggt tgt atg aag ggt aaa gga gga cca gat aat 426
Lys Gly Ser Lys Lys Gly Cys Met Lys Gly Lys Gly Gly Pro Asp Asn
        60                  65                  70

tct cac tgt agt ttt aga gga gtt aga caa agg att tgg ggt aaa tgg 474
Ser His Cys Ser Phe Arg Gly Val Arg Gln Arg Ile Trp Gly Lys Trp
    75                  80                  85

gtt gca gag att cga gaa ccg aaa ata gga act aga ctt tgg ctt ggt 522
Val Ala Glu Ile Arg Glu Pro Lys Ile Gly Thr Arg Leu Trp Leu Gly
90                  95                  100                 105

act ttt cct acc gcg gaa aaa gct gct tcc gct tat gat gaa gcg gct 570
Thr Phe Pro Thr Ala Glu Lys Ala Ala Ser Ala Tyr Asp Glu Ala Ala
                110                 115                 120

acc gct atg tac ggt tca ttg gct cgt ctt aac ttc cct cag tct gtt 618
Thr Ala Met Tyr Gly Ser Leu Ala Arg Leu Asn Phe Pro Gln Ser Val
            125                 130                 135

ggg tct gag ttt act agt acg tct agt caa tct gag gtg tgt acg gtt 666
Gly Ser Glu Phe Thr Ser Thr Ser Ser Gln Ser Glu Val Cys Thr Val
        140                 145                 150

gaa aat aag gcg gtt gtt tgt ggt gat gtt tgt gtg aag cat gaa gat 714
Glu Asn Lys Ala Val Val Cys Gly Asp Val Cys Val Lys His Glu Asp
    155                 160                 165

act gat tgt gaa tct aat cca ttt agt cag att tta gat gtt aga gaa 762
Thr Asp Cys Glu Ser Asn Pro Phe Ser Gln Ile Leu Asp Val Arg Glu
170                 175                 180                 185

gag tct tgt gga acc agg ccg gac agt tgc acg gtt gga cat caa gat 810
Glu Ser Cys Gly Thr Arg Pro Asp Ser Cys Thr Val Gly His Gln Asp
                190                 195                 200

atg aat tct tcg ctg aat tac gat ttg ctg tta gag ttt gag cag cag 858
Met Asn Ser Ser Leu Asn Tyr Asp Leu Leu Leu Glu Phe Glu Gln Gln

            205                 210                 215

tat tgg ggc caa gtt ttg cag gag aaa gag aaa ccg aag cag gaa gaa 906
Tyr Trp Gly Gln Val Leu Gln Glu Lys Glu Lys Pro Lys Gln Glu Glu
        220                 225                 230

gag gag ata cag caa cag caa cag gaa cag caa cag caa cag ctg caa 954
Glu Glu Ile Gln Gln Gln Gln Gln Glu Gln Gln Gln Gln Gln Leu Gln
    235                 240                 245

ccg gat ttg ctt act gtt gca gat tac ggt tgg cct tgg tct aat gat 1002
Pro Asp Leu Leu Thr Val Ala Asp Tyr Gly Trp Pro Trp Ser Asn Asp
250                 255                 260                 265

att gta aat gat cag act tct tgg gat cct aat gag tgc ttt gat att 1050
Ile Val Asn Asp Gln Thr Ser Trp Asp Pro Asn Glu Cys Phe Asp Ile
                270                 275                 280

aat gaa ctc ctt gga gat ttg aat gaa cct ggt ccc cat cag agc caa 1098
Asn Glu Leu Leu Gly Asp Leu Asn Glu Pro Gly Pro His Gln Ser Gln
            285                 290                 295

gac caa aac cac gta aat tct ggt agt tat gat ttg cat ccg ctt cat 1146
Asp Gln Asn His Val Asn Ser Gly Ser Tyr Asp Leu His Pro Leu His
        300                 305                 310

ctc gag cca cac gat ggt cac gag ttc aat ggt ttg agt tct ctg gat 1194
Leu Glu Pro His Asp Gly His Glu Phe Asn Gly Leu Ser Ser Leu Asp
    315                 320                 325

att tga gagttctgag gcaatggtcc tacaagacta caacataatc tttggattga 1250
Ile
330

tcataggaga aacaagaaat aggtgttaat gatctgattc acaatgaaaa aatatttaat 1310

aactctatag tttttgttct ttccttggat catgaactgt tgcttctcat ctattgagtt 1370

aatatagcga atagcagagt ttctctcata aaaaaaaaaa aaa 1413

<210> 32
<211> 330
<212> PRT
<213> Arabidopsis thaliana

<400> 32

Met Ala Val Tyr Glu Gln Thr Gly Thr Glu Gln Pro Lys Lys Arg Lys
1               5                   10                  15

Ser Arg Ala Arg Ala Gly Gly Leu Thr Val Ala Asp Arg Leu Lys Lys
            20                  25                  30

Trp Lys Glu Tyr Asn Glu Ile Val Glu Ala Ser Ala Val Lys Glu Gly
        35                  40                  45

Glu Lys Pro Lys Arg Lys Val Pro Ala Lys Gly Ser Lys Lys Gly Cys
    50                  55                  60

Met Lys Gly Lys Gly Gly Pro Asp Asn Ser His Cys Ser Phe Arg Gly
65                  70                  75                  80

Val Arg Gln Arg Ile Trp Gly Lys Trp Val Ala Glu Ile Arg Glu Pro
                85                  90                  95

Lys Ile Gly Thr Arg Leu Trp Leu Gly Thr Phe Pro Thr Ala Glu Lys
            100                 105                 110

Ala Ala Ser Ala Tyr Asp Glu Ala Ala Thr Ala Met Tyr Gly Ser Leu
        115                 120                 125

Ala Arg Leu Asn Phe Pro Gln Ser Val Gly Ser Glu Phe Thr Ser Thr
    130                 135                 140

Ser Ser Gln Ser Glu Val Cys Thr Val Glu Asn Lys Ala Val Val Cys
145                 150                 155                 160

Gly Asp Val Cys Val Lys His Glu Asp Thr Asp Cys Glu Ser Asn Pro
                165                 170                 175

Phe Ser Gln Ile Leu Asp Val Arg Glu Glu Ser Cys Gly Thr Arg Pro
            180                 185                 190

Asp Ser Cys Thr Val Gly His Gln Asp Met Asn Ser Ser Leu Asn Tyr
        195                 200                 205

Asp Leu Leu Leu Glu Phe Glu Gln Gln Tyr Trp Gly Gln Val Leu Gln
    210                 215                 220

Glu Lys Glu Lys Pro Lys Gln Glu Glu Glu Glu Ile Gln Gln Gln Gln
225                 230                 235                 240

Gln Glu Gln Gln Gln Gln Gln Leu Gln Pro Asp Leu Leu Thr Val Ala
                245                 250                 255

Asp Tyr Gly Trp Pro Trp Ser Asn Asp Ile Val Asn Asp Gln Thr Ser
            260                 265                 270

Trp Asp Pro Asn Glu Cys Phe Asp Ile Asn Glu Leu Leu Gly Asp Leu
        275                 280                 285

Asn Glu Pro Gly Pro His Gln Ser Gln Asp Gln Asn His Val Asn Ser
    290                 295                 300

Gly Ser Tyr Asp Leu His Pro Leu His Leu Glu Pro His Asp Gly His
305                 310                 315                 320

Glu Phe Asn Gly Leu Ser Ser Leu Asp Ile
                325                 330

<210>  33
<211>  818
<212>  DNA
<213>  Arabidopsis thaliana

<220>
<221>  CDS
<222>  (18)..(641)
<223>  G46

<400>  33
ttctctcttt cgtaaaa atg gcg agt ttt gag gaa agc tct gat ttg gaa        50
                   Met Ala Ser Phe Glu Glu Ser Ser Asp Leu Glu
                   1               5                   10

gct ata cag agc cat ctc tta gaa gac ttg ttg gtt tgt gat ggt ttc        98
Ala Ile Gln Ser His Leu Leu Glu Asp Leu Leu Val Cys Asp Gly Phe

15                  20                  25

atg gga gat ttt gac ttc gat gct tct ttt gtc tca gga ctt tgg tgt      146
Met Gly Asp Phe Asp Phe Asp Ala Ser Phe Val Ser Gly Leu Trp Cys
        30                  35                  40

ata gaa cca cac gtt cct aaa caa gaa cct gat tct cca gtt ctt gat      194
Ile Glu Pro His Val Pro Lys Gln Glu Pro Asp Ser Pro Val Leu Asp
    45                  50                  55

ccg gat tct ttc gtc aac gag ttc ttg caa gtg gaa ggg gaa tca tca      242
Pro Asp Ser Phe Val Asn Glu Phe Leu Gln Val Glu Gly Glu Ser Ser
60                  65                  70                  75

tca tca tca tca cca gag ctg aat tca tcg tca tca aca tat gag act      290
Ser Ser Ser Ser Pro Glu Leu Asn Ser Ser Ser Ser Thr Tyr Glu Thr
                80                  85                  90

gat cag agt gtg aaa aag gca gag agg ttc gaa gaa gaa gta gat gct      338
Asp Gln Ser Val Lys Lys Ala Glu Arg Phe Glu Glu Glu Val Asp Ala
            95                  100                 105

aga cat tac cga gga gtg agg cga agg ccg tgg ggg aaa ttt gca gca      386
Arg His Tyr Arg Gly Val Arg Arg Arg Pro Trp Gly Lys Phe Ala Ala
        110                 115                 120

gag att cga gat cca gca aag aaa gga tca aga atc tgg cta gga aca      434
Glu Ile Arg Asp Pro Ala Lys Lys Gly Ser Arg Ile Trp Leu Gly Thr
    125                 130                 135

ttt gag agt gat gtt gat gct gca aga gcc tat gac tgt gca gct ttc      482
Phe Glu Ser Asp Val Asp Ala Ala Arg Ala Tyr Asp Cys Ala Ala Phe
140                 145                 150                 155

aag ctc cgg gga aga aaa gcc gtg ctc aac ttc cct ctt gac gcc ggg      530
Lys Leu Arg Gly Arg Lys Ala Val Leu Asn Phe Pro Leu Asp Ala Gly
                160                 165                 170

aaa tat gaa gct cca gcg aat tca gga agg aaa agg aag aga agt gat      578
Lys Tyr Glu Ala Pro Ala Asn Ser Gly Arg Lys Arg Lys Arg Ser Asp
            175                 180                 185

gtg cat gaa gag ctt caa aga act cag agc aat tca tct tca tct tcc      626
Val His Glu Glu Leu Gln Arg Thr Gln Ser Asn Ser Ser Ser Ser Ser
        190                 195                 200

tgt gat gca ttt tag catattaaga gtgtgagcag tttccttaag ttgtataaag      681
Cys Asp Ala Phe
    205

taattgtaca gaggaaacga attgtgtagg tttagtgtgc ttgcaagttg caacaaatgt      741

gtatggatgt tctgtttctt catgtcccta agatttagaa acatcttctt atttccaaga      801

aaaaaaaaaa aaaaaaa                                                     818

<210>  34
<211>  207
<212>  PRT
<213>  Arabidopsis thaliana

<400>  34

Met Ala Ser Phe Glu Glu Ser Ser Asp Leu Glu Ala Ile Gln Ser His
1               5                   10                  15

Leu Leu Glu Asp Leu Leu Val Cys Asp Gly Phe Met Gly Asp Phe Asp
            20                  25                  30

Phe Asp Ala Ser Phe Val Ser Gly Leu Trp Cys Ile Glu Pro His Val
        35                  40                  45

Pro Lys Gln Glu Pro Asp Ser Pro Val Leu Asp Pro Asp Ser Phe Val
50                  55                  60

Asn Glu Phe Leu Gln Val Glu Gly Glu Ser Ser Ser Ser Ser Ser Pro
65                  70                  75                  80

Glu Leu Asn Ser Ser Ser Ser Thr Tyr Glu Thr Asp Gln Ser Val Lys
                85                  90                  95

Lys Ala Glu Arg Phe Glu Glu Glu Val Asp Ala Arg His Tyr Arg Gly
            100                 105                 110

Val Arg Arg Arg Pro Trp Gly Lys Phe Ala Ala Glu Ile Arg Asp Pro
        115                 120                 125

Ala Lys Lys Gly Ser Arg Ile Trp Leu Gly Thr Phe Glu Ser Asp Val
    130                 135                 140

Asp Ala Ala Arg Ala Tyr Asp Cys Ala Ala Phe Lys Leu Arg Gly Arg
145                 150                 155                 160

Lys Ala Val Leu Asn Phe Pro Leu Asp Ala Gly Lys Tyr Glu Ala Pro
                165                 170                 175

Ala Asn Ser Gly Arg Lys Arg Lys Arg Ser Asp Val His Glu Glu Leu
            180                 185                 190

Gln Arg Thr Gln Ser Asn Ser Ser Ser Ser Ser Cys Asp Ala Phe
        195                 200                 205

<210>  35
<211>  1195
<212>  DNA
<213>  Arabidopsis thaliana

<220>
<221>  CDS
<222>  (66)..(983)
<223>  G242

<400>  35
ctctcaaaac caaaatcact aaagaggaga agattgctaa agtttgataa aacattccaa      60

aatca atg gct gat agg atc aaa ggt cca tgg agt cct gaa gaa gac gag      110
      Met Ala Asp Arg Ile Lys Gly Pro Trp Ser Pro Glu Glu Asp Glu
      1               5                   10                  15

cag ctt cgt agg ctt gtt gtt aaa tac ggt cca aga aac tgg aca gtg      158
Gln Leu Arg Arg Leu Val Val Lys Tyr Gly Pro Arg Asn Trp Thr Val
                20                  25                  30

att agc aaa tct att ccc ggt aga tcg ggg aaa tcg tgt cgt tta cgg      206
Ile Ser Lys Ser Ile Pro Gly Arg Ser Gly Lys Ser Cys Arg Leu Arg
            35                  40                  45

tgg tgc aac cag ctt tcg ccg caa gtt gag cat cgg ccg ttt tcg gct      254
Trp Cys Asn Gln Leu Ser Pro Gln Val Glu His Arg Pro Phe Ser Ala
        50                  55                  60

gag gaa gac gag acg atc gca cgt gct cac gct cag ttc ggg aat aaa      302
Glu Glu Asp Glu Thr Ile Ala Arg Ala His Ala Gln Phe Gly Asn Lys
    65                  70                  75

tgg gcg acg att gct cgt ctt ctc aac ggt cgt acg gac aac gcc gtg 350
Trp Ala Thr Ile Ala Arg Leu Leu Asn Gly Arg Thr Asp Asn Ala Val
80                  85                  90                  95

aag aat cac tgg aac tcg acg ctc aag agg aaa tgc ggc ggt tac gac 398
Lys Asn His Trp Asn Ser Thr Leu Lys Arg Lys Cys Gly Gly Tyr Asp
                100                 105                 110

cat cgg ggt tac gat ggt tcg gag gat cat cgg ccg gtt aag aga tcg 446
His Arg Gly Tyr Asp Gly Ser Glu Asp His Arg Pro Val Lys Arg Ser
            115                 120                 125

gtg agt gcg gga tct cca cct gtt gtt act ggg ctt tac atg agc cca 494
Val Ser Ala Gly Ser Pro Pro Val Val Thr Gly Leu Tyr Met Ser Pro
        130                 135                 140

gga agc cca act gga tct gat gtc agt gat tca agt act atc ccg ata 542
Gly Ser Pro Thr Gly Ser Asp Val Ser Asp Ser Ser Thr Ile Pro Ile
    145                 150                 155

tta cct tcc gtt gag ctt ttc aag cct gtg cct aga cct ggt gct gtt 590
Leu Pro Ser Val Glu Leu Phe Lys Pro Val Pro Arg Pro Gly Ala Val
160                 165                 170                 175

gtg cta ccg ctt cct atc gaa acg tcg tct ttt tcc gat gat cca ccg 638
Val Leu Pro Leu Pro Ile Glu Thr Ser Ser Phe Ser Asp Asp Pro Pro
                180                 185                 190

act tcg tta agc ttg tca ctt cct ggt gcc gac gta agc gag gag tca 686
Thr Ser Leu Ser Leu Ser Leu Pro Gly Ala Asp Val Ser Glu Glu Ser
            195                 200                 205

aac cgt agc cac gag tca acg aat atc aac aac acc act tcg agc cgc 734
Asn Arg Ser His Glu Ser Thr Asn Ile Asn Asn Thr Thr Ser Ser Arg
        210                 215                 220

cac aac cac aac aat acg gtg tcg ttt atg ccg ttt agt ggt ggg ttt 782
His Asn His Asn Asn Thr Val Ser Phe Met Pro Phe Ser Gly Gly Phe
    225                 230                 235

aga ggt gcg att gag gaa atg ggg aag tct ttt ccc ggt aac gga ggc 830
Arg Gly Ala Ile Glu Glu Met Gly Lys Ser Phe Pro Gly Asn Gly Gly
240                 245                 250                 255

gag ttt atg gcg gtg gtg caa gag atg att aag gcg gaa gtg agg agt 878
Glu Phe Met Ala Val Val Gln Glu Met Ile Lys Ala Glu Val Arg Ser
                260                 265                 270

tac atg acg gag atg caa cgg aac aat ggt ggc gga ttc gtc gga gga 926
Tyr Met Thr Glu Met Gln Arg Asn Asn Gly Gly Gly Phe Val Gly Gly
            275                 280                 285

ttc att gat aat ggc atg att ccg atg agt caa att gga gtt ggg aga 974
Phe Ile Asp Asn Gly Met Ile Pro Met Ser Gln Ile Gly Val Gly Arg
        290                 295                 300

atc gag tag acaaagtgag attattagga aactgtttaa attggagaag 1023
Ile Glu
    305

aagaaaaatg ctctgttttt ttctcctttg gattaggctt aagaattttg ggttttaagg 1083

aaatgtatag aggaaatcga gtgaacaaag ctcgagagct ggggacgtag tgacgaagac 1143

gaagatcaaa tttctcttaa gctattcagg aaaataaaat aaatttttat tt 1195

<210> 36
<211> 305
<212> PRT
<213> Arabidopsis thaliana

<400> 36

Met Ala Asp Arg Ile Lys Gly Pro Trp Ser Pro Glu Glu Asp Glu Gln

1               5                   10                  15

Leu Arg Arg Leu Val Val Lys Tyr Gly Pro Arg Asn Trp Thr Val Ile
            20                  25                  30

Ser Lys Ser Ile Pro Gly Arg Ser Gly Lys Ser Cys Arg Leu Arg Trp
        35                  40                  45

Cys Asn Gln Leu Ser Pro Gln Val Glu His Arg Pro Phe Ser Ala Glu
    50                  55                  60

Glu Asp Glu Thr Ile Ala Arg Ala His Ala Gln Phe Gly Asn Lys Trp
65                  70                  75                  80

Ala Thr Ile Ala Arg Leu Leu Asn Gly Arg Thr Asp Asn Ala Val Lys
                85                  90                  95

Asn His Trp Asn Ser Thr Leu Lys Arg Lys Cys Gly Gly Tyr Asp His
            100                 105                 110

Arg Gly Tyr Asp Gly Ser Glu Asp His Arg Pro Val Lys Arg Ser Val
        115                 120                 125

Ser Ala Gly Ser Pro Pro Val Val Thr Gly Leu Tyr Met Ser Pro Gly
    130                 135                 140

Ser Pro Thr Gly Ser Asp Val Ser Asp Ser Ser Thr Ile Pro Ile Leu
145                 150                 155                 160

Pro Ser Val Glu Leu Phe Lys Pro Val Pro Arg Pro Gly Ala Val Val
                165                 170                 175

Leu Pro Leu Pro Ile Glu Thr Ser Ser Phe Ser Asp Asp Pro Pro Thr
            180                 185                 190

Ser Leu Ser Leu Ser Leu Pro Gly Ala Asp Val Ser Glu Glu Ser Asn
        195                 200                 205

Arg Ser His Glu Ser Thr Asn Ile Asn Asn Thr Thr Ser Ser Arg His
    210                 215                 220

Asn His Asn Asn Thr Val Ser Phe Met Pro Phe Ser Gly Gly Phe Arg
225                 230                 235                 240

Gly Ala Ile Glu Glu Met Gly Lys Ser Phe Pro Gly Asn Gly Gly Glu
                245                 250                 255

Phe Met Ala Val Val Gln Glu Met Ile Lys Ala Glu Val Arg Ser Tyr
            260                 265                 270

Met Thr Glu Met Gln Arg Asn Asn Gly Gly Gly Phe Val Gly Gly Phe
        275                 280                 285

Ile Asp Asn Gly Met Ile Pro Met Ser Gln Ile Gly Val Gly Arg Ile
    290                 295                 300

Glu
305

<210> 37
<211> 989
<212> DNA
<213> Arabidopsis thaliana

<220>
<221> CDS
<222> (21)..(983)
<223> G227

<400> 37
gtaccgtcga cgatccggcg atg tca aac ccg acc cgt aag aat atg gag agg 53
Met Ser Asn Pro Thr Arg Lys Asn Met Glu Arg
1               5                   10

att aaa ggt cca tgg agt cca gaa gaa gat gat ctg ttg cag agg ctt 101
Ile Lys Gly Pro Trp Ser Pro Glu Glu Asp Asp Leu Leu Gln Arg Leu
            15                  20                  25

gtt cag aaa cat ggt ccg agg aac tgg tct ttg att agc aaa tca atc 149
Val Gln Lys His Gly Pro Arg Asn Trp Ser Leu Ile Ser Lys Ser Ile
        30                  35                  40

cct gga cgt tcc ggc aaa tct tgt cgt ctc cgg tgg tgt aac cag cta 197
Pro Gly Arg Ser Gly Lys Ser Cys Arg Leu Arg Trp Cys Asn Gln Leu
    45                  50                  55

tct ccg gag gta gag cac cgt gct ttt tcg cag gaa gaa gac gag acg 245
Ser Pro Glu Val Glu His Arg Ala Phe Ser Gln Glu Glu Asp Glu Thr
60                  65                  70                  75

att att cga gct cac gct cgg ttt ggt aac aag tgg gct acg atc tct 293
Ile Ile Arg Ala His Ala Arg Phe Gly Asn Lys Trp Ala Thr Ile Ser
                80                  85                  90

cgt ctt ctc aat gga cga acc gat aac gct atc aag aat cat tgg aac 341
Arg Leu Leu Asn Gly Arg Thr Asp Asn Ala Ile Lys Asn His Trp Asn
            95                  100                 105

tcg acg ctg aag cga aaa tgc agc gtc gaa ggg caa agt tgt gat ttt 389
Ser Thr Leu Lys Arg Lys Cys Ser Val Glu Gly Gln Ser Cys Asp Phe
        110                 115                 120

ggt ggt aat gga ggg tat gat ggt aat tta gga gaa gag caa ccg ttg 437
Gly Gly Asn Gly Gly Tyr Asp Gly Asn Leu Gly Glu Glu Gln Pro Leu
    125                 130                 135

aaa cgt acg gcg agt ggt ggt ggt ggt gtc tcg act ggc ttg tat atg 485
Lys Arg Thr Ala Ser Gly Gly Gly Gly Val Ser Thr Gly Leu Tyr Met
140                 145                 150                 155

agt ccc gga agt cca tcg gga tct gac gtc agc gag caa tct agt ggt 533
Ser Pro Gly Ser Pro Ser Gly Ser Asp Val Ser Glu Gln Ser Ser Gly
                160                 165                 170

ggt gca cac gtg ttt aaa cca acg gtt aga tct gag gtt aca gcg tca 581
Gly Ala His Val Phe Lys Pro Thr Val Arg Ser Glu Val Thr Ala Ser
            175                 180                 185

tcg tct ggt gaa gat cct cca act tat ctt agt ttg tct ctt cct tgg 629
Ser Ser Gly Glu Asp Pro Pro Thr Tyr Leu Ser Leu Ser Leu Pro Trp
        190                 195                 200

act gac gag acg gtt cga gtc aac gag ccg gtt caa ctt aac cag aat 677
Thr Asp Glu Thr Val Arg Val Asn Glu Pro Val Gln Leu Asn Gln Asn
    205                 210                 215

acg gtt atg gac ggt ggt tat acg gcg gag ctg ttt ccg gtt aga aag 725
Thr Val Met Asp Gly Gly Tyr Thr Ala Glu Leu Phe Pro Val Arg Lys
220                 225                 230                 235

gaa gag caa gtg gaa gta gaa gaa gaa gaa gcg aag ggg ata tct ggt 773
Glu Glu Gln Val Glu Val Glu Glu Glu Glu Ala Lys Gly Ile Ser Gly
                240                 245                 250

gga ttc ggt ggt gag ttc atg acg gtg gtt cag gag atg ata agg acg 821
Gly Phe Gly Gly Glu Phe Met Thr Val Val Gln Glu Met Ile Arg Thr
            255                 260                 265

gag gtg agg agt tac atg gcg gat tta cag cga gga aac gtc ggt ggt 869
Glu Val Arg Ser Tyr Met Ala Asp Leu Gln Arg Gly Asn Val Gly Gly
        270                 275                 280

agt agt tct ggc ggc gga ggt ggc ggt tcg tgt atg cca caa agt gta 917
Ser Ser Ser Gly Gly Gly Gly Gly Gly Ser Cys Met Pro Gln Ser Val
    285                 290                 295

aac agc cgt cgt gtt ggg ttt aga gag ttt ata gtg aac caa atc gga 965
Asn Ser Arg Arg Val Gly Phe Arg Glu Phe Ile Val Asn Gln Ile Gly
300                 305                 310                 315

att ggg aag atg gag tag gcggcc 989
Ile Gly Lys Met Glu
                320

<210> 38
<211> 320
<212> PRT
<213> Arabidopsis thaliana

<400> 38

Met Ser Asn Pro Thr Arg Lys Asn Met Glu Arg Ile Lys Gly Pro Trp
1               5                   10                  15

Ser Pro Glu Glu Asp Asp Leu Leu Gln Arg Leu Val Gln Lys His Gly
            20                  25                  30

Pro Arg Asn Trp Ser Leu Ile Ser Lys Ser Ile Pro Gly Arg Ser Gly
        35                  40                  45

Lys Ser Cys Arg Leu Arg Trp Cys Asn Gln Leu Ser Pro Glu Val Glu
    50                  55                  60

His Arg Ala Phe Ser Gln Glu Glu Asp Glu Thr Ile Ile Arg Ala His
65                  70                  75                  80

Ala Arg Phe Gly Asn Lys Trp Ala Thr Ile Ser Arg Leu Leu Asn Gly
                85                  90                  95

Arg Thr Asp Asn Ala Ile Lys Asn His Trp Asn Ser Thr Leu Lys Arg
            100                 105                 110

Lys Cys Ser Val Glu Gly Gln Ser Cys Asp Phe Gly Gly Asn Gly Gly
        115                 120                 125

Tyr Asp Gly Asn Leu Gly Glu Glu Gln Pro Leu Lys Arg Thr Ala Ser
    130                 135                 140

Gly Gly Gly Gly Val Ser Thr Gly Leu Tyr Met Ser Pro Gly Ser Pro
145                 150                 155                 160

Ser Gly Ser Asp Val Ser Glu Gln Ser Ser Gly Gly Ala His Val Phe
                165                 170                 175

Lys Pro Thr Val Arg Ser Glu Val Thr Ala Ser Ser Ser Gly Glu Asp
        180                 185                 190

Pro Pro Thr Tyr Leu Ser Leu Ser Leu Pro Trp Thr Asp Glu Thr Val
        195                 200                 205

Arg Val Asn Glu Pro Val Gln Leu Asn Gln Asn Thr Val Met Asp Gly
    210                 215                 220

Gly Tyr Thr Ala Glu Leu Phe Pro Val Arg Lys Glu Glu Gln Val Glu
225                 230                 235                 240

Val Glu Glu Glu Glu Ala Lys Gly Ile Ser Gly Gly Phe Gly Gly Glu
                245                 250                 255

Phe Met Thr Val Val Gln Glu Met Ile Arg Thr Glu Val Arg Ser Tyr
            260                 265                 270

Met Ala Asp Leu Gln Arg Gly Asn Val Gly Gly Ser Ser Ser Gly Gly
        275                 280                 285

Gly Gly Gly Gly Ser Cys Met Pro Gln Ser Val Asn Ser Arg Arg Val
    290                 295                 300

Gly Phe Arg Glu Phe Ile Val Asn Gln Ile Gly Ile Gly Lys Met Glu
305                 310                 315                 320

<210>  39
<211>  994
<212>  DNA
<213>  Arabidopsis thaliana

<220>
<221>  CDS
<222>  (140)..(889)
<223>  G1307

<400>  39
   cccttattgg gcntnancgn ccncccggca ggtctagnnn tnancgcccg cgtccttctn      60
   ccattttacn cncttgcngc ccacccttgt atntcntttt ntnngtgntn tttttcntga     120
   gggggcaacg gaaaaaaga atg gga aga gca cca tgt tgt gag aaa atg ggg      172
                        Met Gly Arg Ala Pro Cys Cys Glu Lys Met Gly
                        1               5                   10
   gtg aag aga gga cca tgg act cct gaa gaa gat caa atc ttg atc aat      220
   Val Lys Arg Gly Pro Trp Thr Pro Glu Glu Asp Gln Ile Leu Ile Asn
               15                  20                  25
   tat att cat ctt tat ggt cat tct aat tgg cga gct ctc cca aaa cac      268
   Tyr Ile His Leu Tyr Gly His Ser Asn Trp Arg Ala Leu Pro Lys His
           30                  35                  40
   gca ggt tta ctt aga tgt ggg aaa agt tgc aga ctt ggt tgg atc aat      316
   Ala Gly Leu Leu Arg Cys Gly Lys Ser Cys Arg Leu Gly Trp Ile Asn
       45                  50                  55
   tat ctt aga cca gac att aaa cgt ggc aat ttc act cct caa gaa gaa      364
   Tyr Leu Arg Pro Asp Ile Lys Arg Gly Asn Phe Thr Pro Gln Glu Glu
   60                  65                  70                  75
   caa act att atc aat ctg cat gaa agc tta ggc aac aga tgg tct gcg      412
   Gln Thr Ile Ile Asn Leu His Glu Ser Leu Gly Asn Arg Trp Ser Ala
   80 85 90
   att gct gca aaa ttg ccg gga cga acc gac aat gaa ata aaa aat gtt 460
   Ile Ala Ala Lys Leu Pro Gly Arg Thr Asp Asn Glu Ile Lys Asn Val
   95 100 105
   tgg cac act cat ttg aag aaa aga ctc agc aaa aat cta aac aat ggc 508
   Trp His Thr His Leu Lys Lys Arg Leu Ser Lys Asn Leu Asn Asn Gly
   110 115 120
   gga gac acc aaa gac gtt aac gga att aac gag acc aca aat gaa gac 556
   Gly Asp Thr Lys Asp Val Asn Gly Ile Asn Glu Thr Thr Asn Glu Asp
   125 130 135
   aaa gga tct gtg ata gtc gac aca gcc tct tta caa caa ttt tct aat 604
   Lys Gly Ser Val Ile Val Asp Thr Ala Ser Leu Gln Gln Phe Ser Asn
   140 145 150 155
   agt att aca aca ttt gat att tca aat gat aac aag gac gat att atg 652
   Ser Ile Thr Thr Phe Asp Ile Ser Asn Asp Asn Lys Asp Asp Ile Met
   160 165 170
   tcg tac gag gat att tct gcc ttg ata gat gat agt ttt tgg tcg gac 700
   Ser Tyr Glu Asp Ile Ser Ala Leu Ile Asp Asp Ser Phe Trp Ser Asp
   175 180 185
   gtc ata tcg gta gat aat tcg aat aag aat gag aag aag ata gag gat 748
   Val Ile Ser Val Asp Asn Ser Asn Lys Asn Glu Lys Lys Ile Glu Asp
   190 195 200
   tgg gaa gga ttg atc gat aga aat agt aaa aaa tgt agc tat agt aat 796
   Trp Glu Gly Leu Ile Asp Arg Asn Ser Lys Lys Cys Ser Tyr Ser Asn
   205 210 215
   tct aag ttg tat aat gat gac atg gag ttt tgg ttt gat gtt ttc act 844
   Ser Lys Leu Tyr Asn Asp Asp Met Glu Phe Trp Phe Asp Val Phe Thr
   220 225 230 235
   agt aat cgt aga att gag gaa ttt tcc gac ata ccc gag ttt taa 889
   Ser Asn Arg Arg Ile Glu Glu Phe Ser Asp Ile Pro Glu Phe
   240 245
   ttttgatttt gattttgtgt tgtttttgtc gttaagactt tgaaagtctt tttgtaatcc 949
   aaatgaataa attccttttc tttttaaaaa aaaaaaaaaa aaaaa 994

<210> 40
<211> 249
<212> PRT
<213> Arabidopsis thaliana

<400> 40

Met Gly Arg Ala Pro Cys Cys Glu Lys Met Gly Val Lys Arg Gly Pro
1               5                   10                  15

Trp Thr Pro Glu Glu Asp Gln Ile Leu Ile Asn Tyr Ile His Leu Tyr
            20                  25                  30

Gly His Ser Asn Trp Arg Ala Leu Pro Lys His Ala Gly Leu Leu Arg
        35                  40                  45

Cys Gly Lys Ser Cys Arg Leu Gly Trp Ile Asn Tyr Leu Arg Pro Asp
    50                  55                  60

Ile Lys Arg Gly Asn Phe Thr Pro Gln Glu Glu Gln Thr Ile Ile Asn
65                  70                  75                  80

Leu His Glu Ser Leu Gly Asn Arg Trp Ser Ala Ile Ala Ala Lys Leu
                85                  90                  95

Pro Gly Arg Thr Asp Asn Glu Ile Lys Asn Val Trp His Thr His Leu
            100                 105                 110

Lys Lys Arg Leu Ser Lys Asn Leu Asn Asn Gly Gly Asp Thr Lys Asp
        115                 120                 125

Val Asn Gly Ile Asn Glu Thr Thr Asn Glu Asp Lys Gly Ser Val Ile
    130                 135                 140

Val Asp Thr Ala Ser Leu Gln Gln Phe Ser Asn Ser Ile Thr Thr Phe
145                 150                 155                 160

Asp Ile Ser Asn Asp Asn Lys Asp Asp Ile Met Ser Tyr Glu Asp Ile
                165                 170                 175

Ser Ala Leu Ile Asp Asp Ser Phe Trp Ser Asp Val Ile Ser Val Asp
            180                 185                 190

Asn Ser Asn Lys Asn Glu Lys Lys Ile Glu Asp Trp Glu Gly Leu Ile
        195                 200                 205

Asp Arg Asn Ser Lys Lys Cys Ser Tyr Ser Asn Ser Lys Leu Tyr Asn
    210                 215                 220

Asp Asp Met Glu Phe Trp Phe Asp Val Phe Thr Ser Asn Arg Arg Ile
225                 230                 235                 240

Glu Glu Phe Ser Asp Ile Pro Glu Phe
                245

<210> 41
<211> 891
<212> DNA
<213> Arabidopsis thaliana

<220>
<221> CDS
<222> (1)..(891)
<223> G1327

<400> 41
atg ggg aaa gga aga gca cca tgc tgc gac aag aac aaa gtg aag aga 48
Met Gly Lys Gly Arg Ala Pro Cys Cys Asp Lys Asn Lys Val Lys Arg
1               5                   10                  15

ggg cca tgg agc cct caa gaa gat ctc act ctc atc act ttt att caa 96
Gly Pro Trp Ser Pro Gln Glu Asp Leu Thr Leu Ile Thr Phe Ile Gln
            20                  25                  30

aaa cat ggc cat caa aac tgg aga tct ctt ccc aag ctt gct gga ttg 144
Lys His Gly His Gln Asn Trp Arg Ser Leu Pro Lys Leu Ala Gly Leu
        35                  40                  45

ttg aga tgt ggg aaa agt tgc cga cta aga tgg ata aac tat ctg aga 192
Leu Arg Cys Gly Lys Ser Cys Arg Leu Arg Trp Ile Asn Tyr Leu Arg
    50                  55                  60

ccg gac gtg aag cga ggc aac ttt agc aaa aag gag gaa gat gct atc 240
Pro Asp Val Lys Arg Gly Asn Phe Ser Lys Lys Glu Glu Asp Ala Ile
65                  70                  75                  80

att cac tac cat caa acc ctt gga aac aag tgg tca aag atc gcg tcc 288
Ile His Tyr His Gln Thr Leu Gly Asn Lys Trp Ser Lys Ile Ala Ser
                85                  90                  95

ttc ttg ccg gga aga act gac aac gag atc aaa aac gtg tgg aac acg 336
Phe Leu Pro Gly Arg Thr Asp Asn Glu Ile Lys Asn Val Trp Asn Thr
            100                 105                 110

cat ctc aag aaa cga ctc act cca tct tct tct tct tca tcc ctc tct 384
His Leu Lys Lys Arg Leu Thr Pro Ser Ser Ser Ser Ser Ser Leu Ser
        115                 120                 125

agc act cat gac caa agc aca aaa gca gat cat gac aag aac tgt gac 432
Ser Thr His Asp Gln Ser Thr Lys Ala Asp His Asp Lys Asn Cys Asp
    130                 135                 140

ggg gct caa gaa gaa ata cat tca ggg tta aat gag agc caa aac tca 480
Gly Ala Gln Glu Glu Ile His Ser Gly Leu Asn Glu Ser Gln Asn Ser
145                 150                 155                 160

gct act tcg tca cat cac caa ggc gag tgt atg cac aca aaa cca gag 528
Ala Thr Ser Ser His His Gln Gly Glu Cys Met His Thr Lys Pro Glu
                165                 170                 175

ctt cat gag gtt aat gga ctc aac gag atc cag ttc ctg ctc gac cat 576
Leu His Glu Val Asn Gly Leu Asn Glu Ile Gln Phe Leu Leu Asp His
            180                 185                 190

gat gac ttt gat gat ata acc tct gag ttt ctt cag gat aac gat atc 624
Asp Asp Phe Asp Asp Ile Thr Ser Glu Phe Leu Gln Asp Asn Asp Ile
        195                 200                 205

tta ttt ccg cta gac tct ctt ctt cat aac cac caa act cac att tca 672
Leu Phe Pro Leu Asp Ser Leu Leu His Asn His Gln Thr His Ile Ser
    210                 215                 220

acc caa gaa atg act cga gag gta acc aaa tcg caa tca ttt gat cat 720
Thr Gln Glu Met Thr Arg Glu Val Thr Lys Ser Gln Ser Phe Asp His
225                 230                 235                 240

cct caa ccg gat atc cca tgc gga ttt gaa gac aca aac gaa gaa tcc 768
Pro Gln Pro Asp Ile Pro Cys Gly Phe Glu Asp Thr Asn Glu Glu Ser
                245                 250                 255

gac ttg agg aga cag ctg gtt gaa tca acc aca cct aac aat gag tac 816
Asp Leu Arg Arg Gln Leu Val Glu Ser Thr Thr Pro Asn Asn Glu Tyr
            260                 265                 270

gac gag tgg ttc aac ttc att gac aac caa act tac ttt gat gat ttt 864
Asp Glu Trp Phe Asn Phe Ile Asp Asn Gln Thr Tyr Phe Asp Asp Phe
        275                 280                 285

aat ttc gtc gga gaa gta tgt cta tga 891
Asn Phe Val Gly Glu Val Cys Leu
    290                 295

<210> 42
<211> 296
<212> PRT
<213> Arabidopsis thaliana

<400> 42

Met Gly Lys Gly Arg Ala Pro Cys Cys Asp Lys Asn Lys Val Lys Arg
1               5                   10                  15

Gly Pro Trp Ser Pro Gln Glu Asp Leu Thr Leu Ile Thr Phe Ile Gln
            20                  25                  30

Lys His Gly His Gln Asn Trp Arg Ser Leu Pro Lys Leu Ala Gly Leu
        35                  40                  45

Leu Arg Cys Gly Lys Ser Cys Arg Leu Arg Trp Ile Asn Tyr Leu Arg
50                  55                  60

Pro Asp Val Lys Arg Gly Asn Phe Ser Lys Lys Glu Glu Asp Ala Ile
65                  70                  75                  80

Ile His Tyr His Gln Thr Leu Gly Asn Lys Trp Ser Lys Ile Ala Ser
                85                  90                  95

Phe Leu Pro Gly Arg Thr Asp Asn Glu Ile Lys Asn Val Trp Asn Thr
            100                 105                 110

His Leu Lys Lys Arg Leu Thr Pro Ser Ser Ser Ser Ser Ser Leu Ser
        115                 120                 125

Ser Thr His Asp Gln Ser Thr Lys Ala Asp His Asp Lys Asn Cys Asp
    130                 135                 140

Gly Ala Gln Glu Glu Ile His Ser Gly Leu Asn Glu Ser Gln Asn Ser
145                 150                 155                 160

Ala Thr Ser Ser His His Gln Gly Glu Cys Met His Thr Lys Pro Glu
                165                 170                 175

Leu His Glu Val Asn Gly Leu Asn Glu Ile Gln Phe Leu Leu Asp His
            180                 185                 190

Asp Asp Phe Asp Asp Ile Thr Ser Glu Phe Leu Gln Asp Asn Asp Ile
        195                 200                 205

Leu Phe Pro Leu Asp Ser Leu Leu His Asn His Gln Thr His Ile Ser
    210                 215                 220

Thr Gln Glu Met Thr Arg Glu Val Thr Lys Ser Gln Ser Phe Asp His
225                 230                 235                 240

Pro Gln Pro Asp Ile Pro Cys Gly Phe Glu Asp Thr Asn Glu Glu Ser
                245                 250                 255

Asp Leu Arg Arg Gln Leu Val Glu Ser Thr Thr Pro Asn Asn Glu Tyr
            260                 265                 270

Asp Glu Trp Phe Asn Phe Ile Asp Asn Gln Thr Tyr Phe Asp Asp Phe
        275                 280                 285

Asn Phe Val Gly Glu Val Cys Leu
    290                 295

<210>  43
<211>  1237
<212>  DNA
<213>  Arabidopsis thaliana

<220>
<221>  CDS
<222>  (73)..(954)
<223>  G673

<400> 43
   tctctctcta accccttctc tcttcagtct ctctctctct agacgatctc tatcttgaat 60
   aaaataccga ta atg acc tca acc aat ccg gtg gtc gcc gaa gta ata ccg 111
   Met Thr Ser Thr Asn Pro Val Val Ala Glu Val Ile Pro
   1 5 10
   gcg gaa act tct aca gat gct aca gag acg acg att gca acg acg gaa 159
   Ala Glu Thr Ser Thr Asp Ala Thr Glu Thr Thr Ile Ala Thr Thr Glu
   15 20 25
   gct ggt gaa gca ccg gag aag aag gtg agg aaa gct tat aca atc acc 207
   Ala Gly Glu Ala Pro Glu Lys Lys Val Arg Lys Ala Tyr Thr Ile Thr
   30 35 40 45
   aag tct aga gag agt tgg act gaa gga gaa cac gac aag ttt ctg gaa 255
   Lys Ser Arg Glu Ser Trp Thr Glu Gly Glu His Asp Lys Phe Leu Glu
   50 55 60
   gct ctt caa ttg ttt gat cgt gac tgg aaa aag ata gaa gat ttt gtt 303
   Ala Leu Gln Leu Phe Asp Arg Asp Trp Lys Lys Ile Glu Asp Phe Val
   65 70 75
   ggt tca aag aca gtt att cag atc agg agc cat gcc caa aaa tac ttt 351
   Gly Ser Lys Thr Val Ile Gln Ile Arg Ser His Ala Gln Lys Tyr Phe
   80 85 90
   cta aag gtc caa aaa aat ggg act tta gca cat gtt cca ccc cct agg 399
   Leu Lys Val Gln Lys Asn Gly Thr Leu Ala His Val Pro Pro Pro Arg
   95 100 105
   cct aag cgc aaa gct gct cat cca tat cct caa aag gca tcg aaa aat 447
   Pro Lys Arg Lys Ala Ala His Pro Tyr Pro Gln Lys Ala Ser Lys Asn
   110 115 120 125
   gct caa atg tcg ctt cac gtt tcc atg tcc ttt cct act caa ata aat 495
   Ala Gln Met Ser Leu His Val Ser Met Ser Phe Pro Thr Gln Ile Asn
   130 135 140
   aac ctg cct gga tat act cca tgg gat gat gat aca tct gca ttg tta 543
   Asn Leu Pro Gly Tyr Thr Pro Trp Asp Asp Asp Thr Ser Ala Leu Leu
   145 150 155
   aac att gct gta agt ggg gtt att cca cca gaa gat gaa ctt gat act 591
   Asn Ile Ala Val Ser Gly Val Ile Pro Pro Glu Asp Glu Leu Asp Thr
   160 165 170
   ctt tgt gga gca gaa gtt gat gtt gga tca aat gac atg ata agt gaa 639
   Leu Cys Gly Ala Glu Val Asp Val Gly Ser Asn Asp Met Ile Ser Glu
   175 180 185
   act agt cct tca gca tct ggt atc gga agc tca agc aga aca cta tca 687
   Thr Ser Pro Ser Ala Ser Gly Ile Gly Ser Ser Ser Arg Thr Leu Ser
   190 195 200 205
   gat tct aag ggt ttg aga ctg gcg aaa caa gct ccc tca atg cat ggt 735
   Asp Ser Lys Gly Leu Arg Leu Ala Lys Gln Ala Pro Ser Met His Gly
   210 215 220
   ctt cct gat ttt gct gag gtt tat aac ttc att ggg agt gtg ttc gat 783
   Leu Pro Asp Phe Ala Glu Val Tyr Asn Phe Ile Gly Ser Val Phe Asp
   225 230 235
   cct gac agc aaa ggc cgc atg aaa aag ctc aag gaa atg gat cct ata 831
   Pro Asp Ser Lys Gly Arg Met Lys Lys Leu Lys Glu Met Asp Pro Ile
   240 245 250
   aat ttc gaa act gtt ttg ctg ttg atg aga aac ctc aca gtg aac ttg 879
   Asn Phe Glu Thr Val Leu Leu Leu Met Arg Asn Leu Thr Val Asn Leu
   255 260 265
   tca aac cct gac ttt gaa cct act tct gaa tat gtt gat gct gca gag 927
   Ser Asn Pro Asp Phe Glu Pro Thr Ser Glu Tyr Val Asp Ala Ala Glu
   270                    275                    280                    285
   gaa ggt cat gaa cac tta agc tct tag ctgtttgtgc actcaacaag          974
   Glu Gly His Glu His Leu Ser Ser
                   290
   ttatatatct tcttgacgac ttcttgctcg caacaactct ctaccagcta tcaaatgcat   1034
   cgtacggttg ttgtctgagg agaacataac tgagtcgtcg tcacaaacaa gaggaacata   1094
   tgcagtttcg gtcagaacca gtcgtgtgaa tggtagatat atgtatgtgt gtgtagaaaa   1154
   tggttaccaa ttgtatcttc tttttgataa ttattttttc atgccttttg taatatgtaa   1214
   gtttctttaa aaaaaaaaaa aaa                                            1237

<210>  44
<211>  293
<212>  PRT
<213>  Arabidopsis thaliana

<400>  44

Met Thr Ser Thr Asn Pro Val Val Ala Glu Val Ile Pro Ala Glu Thr
1               5                   10                  15

Ser Thr Asp Ala Thr Glu Thr Thr Ile Ala Thr Thr Glu Ala Gly Glu
            20                  25                  30

Ala Pro Glu Lys Lys Val Arg Lys Ala Tyr Thr Ile Thr Lys Ser Arg
        35                  40                  45

Glu Ser Trp Thr Glu Gly Glu His Asp Lys Phe Leu Glu Ala Leu Gln
    50                  55                  60

Leu Phe Asp Arg Asp Trp Lys Lys Ile Glu Asp Phe Val Gly Ser Lys
65                  70                  75                  80

Thr Val Ile Gln Ile Arg Ser His Ala Gln Lys Tyr Phe Leu Lys Val
                85                  90                  95

Gln Lys Asn Gly Thr Leu Ala His Val Pro Pro Pro Arg Pro Lys Arg
            100                 105                 110

Lys Ala Ala His Pro Tyr Pro Gln Lys Ala Ser Lys Asn Ala Gln Met
        115                 120                 125

Ser Leu His Val Ser Met Ser Phe Pro Thr Gln Ile Asn Asn Leu Pro
    130                 135                 140

Gly Tyr Thr Pro Trp Asp Asp Asp Thr Ser Ala Leu Leu Asn Ile Ala
145                 150                 155                 160

Val Ser Gly Val Ile Pro Pro Glu Asp Glu Leu Asp Thr Leu Cys Gly
                165                 170                 175

Ala Glu Val Asp Val Gly Ser Asn Asp Met Ile Ser Glu Thr Ser Pro
            180                 185                 190

Ser Ala Ser Gly Ile Gly Ser Ser Ser Arg Thr Leu Ser Asp Ser Lys
        195                 200                 205

Gly Leu Arg Leu Ala Lys Gln Ala Pro Ser Met His Gly Leu Pro Asp
    210                 215                 220

Phe Ala Glu Val Tyr Asn Phe Ile Gly Ser Val Phe Asp Pro Asp Ser
225                 230                 235                 240

Lys Gly Arg Met Lys Lys Leu Lys Glu Met Asp Pro Ile Asn Phe Glu
                245                 250                 255

Thr Val Leu Leu Leu Met Arg Asn Leu Thr Val Asn Leu Ser Asn Pro
            260                 265                 270

Asp Phe Glu Pro Thr Ser Glu Tyr Val Asp Ala Ala Glu Glu Gly His
        275                 280                 285

Glu His Leu Ser Ser
    290

<210> 45
<211> 1764
<212> DNA
<213> Arabidopsis thaliana

<220>
<221> CDS
<222> (1)..(1764)
<223> G307

<400> 45
atg aag aga gat cat cac caa ttc caa ggt cga ttg tcc aac cac ggg 48
Met Lys Arg Asp His His Gln Phe Gln Gly Arg Leu Ser Asn His Gly
1               5                   10                  15

act tct tct tct tca tca tca atc tct aaa gat aag atg atg atg gtg 96
Thr Ser Ser Ser Ser Ser Ser Ile Ser Lys Asp Lys Met Met Met Val
            20                  25                  30

aaa aaa gaa gaa gac ggt gga ggt aac atg gac gac gag ctt ctc gct 144
Lys Lys Glu Glu Asp Gly Gly Gly Asn Met Asp Asp Glu Leu Leu Ala
        35                  40                  45

gtt tta ggt tac aaa gtt agg tca tcg gag atg gcg gag gtt gct ttg 192
Val Leu Gly Tyr Lys Val Arg Ser Ser Glu Met Ala Glu Val Ala Leu
    50                  55                  60

aaa ctc gaa caa tta gag acg atg atg agt aat gtt caa gaa gat ggt 240
Lys Leu Glu Gln Leu Glu Thr Met Met Ser Asn Val Gln Glu Asp Gly
65                  70                  75                  80

tta tct cat ctc gcg acg gat act gtt cat tat aat ccg tcg gag ctt 288
Leu Ser His Leu Ala Thr Asp Thr Val His Tyr Asn Pro Ser Glu Leu
                85                  90                  95

tat tct tgg ctt gat aat atg ctc tct gag ctt aat cct cct cct ctt 336
Tyr Ser Trp Leu Asp Asn Met Leu Ser Glu Leu Asn Pro Pro Pro Leu
            100                 105                 110

ccg gcg agt tct aac ggt tta gat ccg gtt ctt cct tcg ccg gag att 384
Pro Ala Ser Ser Asn Gly Leu Asp Pro Val Leu Pro Ser Pro Glu Ile
        115                 120                 125

tgt ggt ttt ccg gct tcg gat tat gac ctt aaa gtc att ccc gga aac 432
Cys Gly Phe Pro Ala Ser Asp Tyr Asp Leu Lys Val Ile Pro Gly Asn
    130                 135                 140

gcg att tat cag ttt ccg gcg att gat tct tcg tct tcg tcg aat aat 480
Ala Ile Tyr Gln Phe Pro Ala Ile Asp Ser Ser Ser Ser Ser Asn Asn

145                 150                 155                 160

cag aac aag cgt ttg aaa tca tgc tcg agt cct gat tct atg gtt aca      528
Gln Asn Lys Arg Leu Lys Ser Cys Ser Ser Pro Asp Ser Met Val Thr
                165                 170                 175

tcg act tcg acg ggt acg cag att ggt gga gtc ata gga acg acg gtg      576
Ser Thr Ser Thr Gly Thr Gln Ile Gly Gly Val Ile Gly Thr Thr Val
            180                 185                 190

acg aca acc acc acg aca acg acg gcg gcg gct gag tca act cgt tct      624
Thr Thr Thr Thr Thr Thr Thr Thr Ala Ala Ala Glu Ser Thr Arg Ser
        195                 200                 205

gtt atc ctg gtt gac tcg caa gag aac ggt gtt cgt tta gtc cac gcg      672
Val Ile Leu Val Asp Ser Gln Glu Asn Gly Val Arg Leu Val His Ala
    210                 215                 220

ctt atg gct tgt gca gaa gca atc cag cag aac aat ttg act cta gcg      720
Leu Met Ala Cys Ala Glu Ala Ile Gln Gln Asn Asn Leu Thr Leu Ala
225                 230                 235                 240

gaa gct ctt gtg aag caa atc gga tgc tta gct gtg tct caa gcc gga      768
Glu Ala Leu Val Lys Gln Ile Gly Cys Leu Ala Val Ser Gln Ala Gly
                245                 250                 255

gct atg aga aaa gtg gct act tac ttc gcc gaa gct tta gct cgg cgg      816
Ala Met Arg Lys Val Ala Thr Tyr Phe Ala Glu Ala Leu Ala Arg Arg
            260                 265                 270

atc tac cgt ctc tct ccg ccg cag aat cag atc gat cat tgt ctc tcc      864
Ile Tyr Arg Leu Ser Pro Pro Gln Asn Gln Ile Asp His Cys Leu Ser
        275                 280                 285

gat act ctt cag atg cac ttt tac gag act tgt cct tat ctt aaa ttc      912
Asp Thr Leu Gln Met His Phe Tyr Glu Thr Cys Pro Tyr Leu Lys Phe
    290                 295                 300

gct cac ttc acg gcg aac caa gcg att ctc gaa gct ttt gaa ggt aag      960
Ala His Phe Thr Ala Asn Gln Ala Ile Leu Glu Ala Phe Glu Gly Lys
305                 310                 315                 320

aag aga gta cac gtc att gat ttc tcg atg aac caa ggt ctt caa tgg      1008
Lys Arg Val His Val Ile Asp Phe Ser Met Asn Gln Gly Leu Gln Trp
                325                 330                 335

cct gcg ctt atg caa gct ctt gcg ctt cga gaa gga ggt cct cca act      1056
Pro Ala Leu Met Gln Ala Leu Ala Leu Arg Glu Gly Gly Pro Pro Thr
            340                 345                 350

ttc cgg tta acc gga att ggt cca ccg gcg ccg gat aat tct gat cat      1104
Phe Arg Leu Thr Gly Ile Gly Pro Pro Ala Pro Asp Asn Ser Asp His
        355                 360                 365

ctt cat gaa gtt ggt tgt aaa tta gct cag ctt gcg gag gcg att cac      1152
Leu His Glu Val Gly Cys Lys Leu Ala Gln Leu Ala Glu Ala Ile His
    370                 375                 380

gta gaa ttc gaa tac cgt gga ttc gtt gct aac agc tta gcc gat ctc      1200
Val Glu Phe Glu Tyr Arg Gly Phe Val Ala Asn Ser Leu Ala Asp Leu
385                 390                 395                 400

gat gct tcg atg ctt gag ctt aga ccg agc gat acg gaa gct gtt gcg      1248
Asp Ala Ser Met Leu Glu Leu Arg Pro Ser Asp Thr Glu Ala Val Ala
                405                 410                 415

gtg aac tct gtt ttt gag cta cat aag ctc tta ggt cgt ccc ggt ggg      1296
Val Asn Ser Val Phe Glu Leu His Lys Leu Leu Gly Arg Pro Gly Gly
            420                 425                 430

ata gag aaa gtt ctc ggc gtt gtg aaa cag att aaa ccg gtg att ttc      1344
Ile Glu Lys Val Leu Gly Val Val Lys Gln Ile Lys Pro Val Ile Phe
        435                 440                 445

acg gtg gtt gag caa gaa tcg aac cat aac gga ccg gtt ttc tta gac      1392

Thr Val Val Glu Gln Glu Ser Asn His Asn Gly Pro Val Phe Leu Asp
    450                 455                 460

cgg ttt act gaa tcg tta cat tat tat tcg act ctg ttt gat tcg ttg      1440
Arg Phe Thr Glu Ser Leu His Tyr Tyr Ser Thr Leu Phe Asp Ser Leu
465                 470                 475                 480

gaa gga gtt ccg aat agt caa gac aaa gtc atg tct gaa gtt tac tta      1488
Glu Gly Val Pro Asn Ser Gln Asp Lys Val Met Ser Glu Val Tyr Leu
                485                 490                 495

ggg aaa cag att tgt aat ctg gtg gct tgt gaa ggt cct gac aga gtc      1536
Gly Lys Gln Ile Cys Asn Leu Val Ala Cys Glu Gly Pro Asp Arg Val
            500                 505                 510

gag aga cac gaa acg ttg agt caa tgg gga aac cgg ttt ggt tcg tcc      1584
Glu Arg His Glu Thr Leu Ser Gln Trp Gly Asn Arg Phe Gly Ser Ser
        515                 520                 525

ggt tta gcg ccg gca cat ctt ggg tct aac gcg ttt aag caa gcg agt      1632
Gly Leu Ala Pro Ala His Leu Gly Ser Asn Ala Phe Lys Gln Ala Ser
    530                 535                 540

atg ctt ttg tct gtg ttt aat agt ggc caa ggt tat cgt gtg gag gag      1680
Met Leu Leu Ser Val Phe Asn Ser Gly Gln Gly Tyr Arg Val Glu Glu
545                 550                 555                 560

agt aat gga tgt ttg atg ttg ggt tgg cac act cgc cca ctc att acc      1728
Ser Asn Gly Cys Leu Met Leu Gly Trp His Thr Arg Pro Leu Ile Thr
                565                 570                 575

acc tcc gct tgg aaa ctc tcg acg gcg gcg cac tga                      1764
Thr Ser Ala Trp Lys Leu Ser Thr Ala Ala His
            580                 585

<210>  46
<211>  587
<212>  PRT
<213>  Arabidopsis thaliana

<400>  46

Met Lys Arg Asp His His Gln Phe Gln Gly Arg Leu Ser Asn His Gly
1               5                   10                  15

Thr Ser Ser Ser Ser Ser Ser Ile Ser Lys Asp Lys Met Met Met Val
            20                  25                  30

Lys Lys Glu Glu Asp Gly Gly Gly Asn Met Asp Asp Glu Leu Leu Ala
        35                  40                  45

Val Leu Gly Tyr Lys Val Arg Ser Ser Glu Met Ala Glu Val Ala Leu
    50                  55                  60

Lys Leu Glu Gln Leu Glu Thr Met Met Ser Asn Val Gln Glu Asp Gly
65                  70                  75                  80

Leu Ser His Leu Ala Thr Asp Thr Val His Tyr Asn Pro Ser Glu Leu
                85                  90                  95

Tyr Ser Trp Leu Asp Asn Met Leu Ser Glu Leu Asn Pro Pro Pro Leu
            100                 105                 110

Pro Ala Ser Ser Asn Gly Leu Asp Pro Val Leu Pro Ser Pro Glu Ile
        115                 120                 125

Cys Gly Phe Pro Ala Ser Asp Tyr Asp Leu Lys Val Ile Pro Gly Asn
    130                 135                 140

Ala Ile Tyr Gln Phe Pro Ala Ile Asp Ser Ser Ser Ser Ser Asn Asn
145                 150                 155                 160

Gln Asn Lys Arg Leu Lys Ser Cys Ser Ser Pro Asp Ser Met Val Thr
                165                 170                 175

Ser Thr Ser Thr Gly Thr Gln Ile Gly Gly Val Ile Gly Thr Thr Val
            180                 185                 190

Thr Thr Thr Thr Thr Thr Thr Thr Ala Ala Ala Glu Ser Thr Arg Ser
        195                 200                 205

Val Ile Leu Val Asp Ser Gln Glu Asn Gly Val Arg Leu Val His Ala
    210                 215                 220

Leu Met Ala Cys Ala Glu Ala Ile Gln Gln Asn Asn Leu Thr Leu Ala
225                 230                 235                 240

Glu Ala Leu Val Lys Gln Ile Gly Cys Leu Ala Val Ser Gln Ala Gly
                245                 250                 255

Ala Met Arg Lys Val Ala Thr Tyr Phe Ala Glu Ala Leu Ala Arg Arg
            260                 265                 270

Ile Tyr Arg Leu Ser Pro Pro Gln Asn Gln Ile Asp His Cys Leu Ser
        275                 280                 285

Asp Thr Leu Gln Met His Phe Tyr Glu Thr Cys Pro Tyr Leu Lys Phe
    290                 295                 300

Ala His Phe Thr Ala Asn Gln Ala Ile Leu Glu Ala Phe Glu Gly Lys
305                 310                 315                 320

Lys Arg Val His Val Ile Asp Phe Ser Met Asn Gln Gly Leu Gln Trp
                325                 330                 335

Pro Ala Leu Met Gln Ala Leu Ala Leu Arg Glu Gly Gly Pro Pro Thr
            340                 345                 350

Phe Arg Leu Thr Gly Ile Gly Pro Pro Ala Pro Asp Asn Ser Asp His
        355                 360                 365

Leu His Glu Val Gly Cys Lys Leu Ala Gln Leu Ala Glu Ala Ile His
    370                 375                 380

Val Glu Phe Glu Tyr Arg Gly Phe Val Ala Asn Ser Leu Ala Asp Leu
385                 390                 395                 400

Asp Ala Ser Met Leu Glu Leu Arg Pro Ser Asp Thr Glu Ala Val Ala
                405                 410                 415

Val Asn Ser Val Phe Glu Leu His Lys Leu Leu Gly Arg Pro Gly Gly
            420                 425                 430

Ile Glu Lys Val Leu Gly Val Val Lys Gln Ile Lys Pro Val Ile Phe
        435                 440                 445

Thr Val Val Glu Gln Glu Ser Asn His Asn Gly Pro Val Phe Leu Asp
    450                 455                 460

Arg Phe Thr Glu Ser Leu His Tyr Tyr Ser Thr Leu Phe Asp Ser Leu
465                 470                 475                 480

Glu Gly Val Pro Asn Ser Gln Asp Lys Val Met Ser Glu Val Tyr Leu
                485                 490                 495

Gly Lys Gln Ile Cys Asn Leu Val Ala Cys Glu Gly Pro Asp Arg Val
            500                 505                 510

Glu Arg His Glu Thr Leu Ser Gln Trp Gly Asn Arg Phe Gly Ser Ser
        515                 520                 525

Gly Leu Ala Pro Ala His Leu Gly Ser Asn Ala Phe Lys Gln Ala Ser
    530                 535                 540

Met Leu Leu Ser Val Phe Asn Ser Gly Gln Gly Tyr Arg Val Glu Glu
545                 550                 555                 560

Ser Asn Gly Cys Leu Met Leu Gly Trp His Thr Arg Pro Leu Ile Thr
                565                 570                 575

Thr Ser Ala Trp Lys Leu Ser Thr Ala Ala His
            580                 585

<210>  47
<211>  990
<212>  DNA
<213>  Arabidopsis thaliana

<220>
<221>  CDS
<222>  (7)..(813)
<223>  G529

<400>  47
gcaaag atg tct tct gat tcg tcc cgg gaa gag aat gtg tac ttg gcc      48
       Met Ser Ser Asp Ser Ser Arg Glu Glu Asn Val Tyr Leu Ala
       1               5                   10

aag tta gcc gag caa gct gag cgt tac gag gaa atg gtt gag ttc atg      96
Lys Leu Ala Glu Gln Ala Glu Arg Tyr Glu Glu Met Val Glu Phe Met
15                  20                  25                  30

gag aaa gtt gca aag acc gtg gag acc gag gaa ctt act gtt gaa gag      144
Glu Lys Val Ala Lys Thr Val Glu Thr Glu Glu Leu Thr Val Glu Glu
                35                  40                  45

agg aat ctc ttg tct gtt gct tac aag aac gtg att ggt gct agg aga      192
Arg Asn Leu Leu Ser Val Ala Tyr Lys Asn Val Ile Gly Ala Arg Arg
            50                  55                  60

gct tct tgg agg att atc tct tcc att gag cag aag gaa gat agc agg      240
Ala Ser Trp Arg Ile Ile Ser Ser Ile Glu Gln Lys Glu Asp Ser Arg
        65                  70                  75

ggc aac agt gat cat gtt tcg att atc aag gat tac aga ggc aag att      288
Gly Asn Ser Asp His Val Ser Ile Ile Lys Asp Tyr Arg Gly Lys Ile

80                  85                  90

gaa act gag ctc agc aag att tgt gat ggc att ttg aac ctt ctt gag     336
Glu Thr Glu Leu Ser Lys Ile Cys Asp Gly Ile Leu Asn Leu Leu Glu
95                  100                 105                 110

gct cat ctc att cct gct gct tct ttg gct gag tcc aaa gtt ttt tac     384
Ala His Leu Ile Pro Ala Ala Ser Leu Ala Glu Ser Lys Val Phe Tyr
                115                 120                 125

ctg aag atg aag gga gat tat cat cgg tac ctt gct gaa ttc aag act     432
Leu Lys Met Lys Gly Asp Tyr His Arg Tyr Leu Ala Glu Phe Lys Thr
            130                 135                 140

ggt gct gag agg aaa gaa gct gct gag agc act ctt gtt gcc tac aag     480
Gly Ala Glu Arg Lys Glu Ala Ala Glu Ser Thr Leu Val Ala Tyr Lys
        145                 150                 155

tct gct cag gat att gct ctt gct gat ctg gct ccc act cac cca atc     528
Ser Ala Gln Asp Ile Ala Leu Ala Asp Leu Ala Pro Thr His Pro Ile
    160                 165                 170

aga ctg ggg ctt gct ctt aac ttc tct gtt ttc tac tat gag att ctc     576
Arg Leu Gly Leu Ala Leu Asn Phe Ser Val Phe Tyr Tyr Glu Ile Leu
175                 180                 185                 190

aac tca tct gat cgt gcg tgt agt ctc gca aag cag gct ttt gat gag     624
Asn Ser Ser Asp Arg Ala Cys Ser Leu Ala Lys Gln Ala Phe Asp Glu
                195                 200                 205

gca atc tcg gag cta gac aca ttg gga gag gaa tca tac aag gac agt     672
Ala Ile Ser Glu Leu Asp Thr Leu Gly Glu Glu Ser Tyr Lys Asp Ser
            210                 215                 220

aca ttg atc atg cag ctt ctc cgt gac aat ctc acc ctc tgg act tct     720
Thr Leu Ile Met Gln Leu Leu Arg Asp Asn Leu Thr Leu Trp Thr Ser
        225                 230                 235

gac ctc aat gac gaa gct ggt gat gat atc aag gaa gcc ccg aaa gag     768
Asp Leu Asn Asp Glu Ala Gly Asp Asp Ile Lys Glu Ala Pro Lys Glu
    240                 245                 250

gtg cag aaa gtt gat gaa caa gcc caa cca cca cct tcg cag tga         813
Val Gln Lys Val Asp Glu Gln Ala Gln Pro Pro Pro Ser Gln
255                 260                 265

taaaatcaga tccatggaat gatttgcaga caaaaagata tatggcttgg ttctgtgttt   873

ttaaacagaa aaaaaccttg tagtttcctt aaacatgggc tgtagtttcc ttaaacatgg   933

atttgtagta gtaattgtag ctgcatgatt tggttatcga tggttaaaaa aaaaaaa      990

<210>  48
<211>  268
<212>  PRT
<213>  Arabidopsis thaliana

<400>  48

Met Ser Ser Asp Ser Ser Arg Glu Glu Asn Val Tyr Leu Ala Lys Leu
1               5                   10                  15

Ala Glu Gln Ala Glu Arg Tyr Glu Glu Met Val Glu Phe Met Glu Lys
            20                  25                  30

Val Ala Lys Thr Val Glu Thr Glu Glu Leu Thr Val Glu Glu Arg Asn
        35                  40                  45

Leu Leu Ser Val Ala Tyr Lys Asn Val Ile Gly Ala Arg Arg Ala Ser
    50                  55                  60

Trp Arg Ile Ile Ser Ser Ile Glu Gln Lys Glu Asp Ser Arg Gly Asn
65                  70                  75                  80

Ser Asp His Val Ser Ile Ile Lys Asp Tyr Arg Gly Lys Ile Glu Thr
                85                  90                  95

Glu Leu Ser Lys Ile Cys Asp Gly Ile Leu Asn Leu Leu Glu Ala His
            100                 105                 110

Leu Ile Pro Ala Ala Ser Leu Ala Glu Ser Lys Val Phe Tyr Leu Lys
        115                 120                 125

Met Lys Gly Asp Tyr His Arg Tyr Leu Ala Glu Phe Lys Thr Gly Ala
    130                 135                 140

Glu Arg Lys Glu Ala Ala Glu Ser Thr Leu Val Ala Tyr Lys Ser Ala
145                 150                 155                 160

Gln Asp Ile Ala Leu Ala Asp Leu Ala Pro Thr His Pro Ile Arg Leu
                165                 170                 175

Gly Leu Ala Leu Asn Phe Ser Val Phe Tyr Tyr Glu Ile Leu Asn Ser
            180                 185                 190

Ser Asp Arg Ala Cys Ser Leu Ala Lys Gln Ala Phe Asp Glu Ala Ile
        195                 200                 205

Ser Glu Leu Asp Thr Leu Gly Glu Glu Ser Tyr Lys Asp Ser Thr Leu
    210                 215                 220

Ile Met Gln Leu Leu Arg Asp Asn Leu Thr Leu Trp Thr Ser Asp Leu
225                 230                 235                 240

Asn Asp Glu Ala Gly Asp Asp Ile Lys Glu Ala Pro Lys Glu Val Gln
                245                 250                 255

Lys Val Asp Glu Gln Ala Gln Pro Pro Pro Ser Gln
            260                 265

<210>  49
<211>  1069
<212>  DNA
<213>  Arabidopsis thaliana

<220>
<221>  CDS
<222>  (70)..(867)
<223>  G531

<400>  49
ccacgcgtcc gcgacaaatc cctaattcca aaatcctctc ggatctaaaa gagtgtgtga      60

taacataag atg tcg tct tct cgg gaa gag aat gtg tac tta gcc aag tta      111
          Met Ser Ser Ser Arg Glu Glu Asn Val Tyr Leu Ala Lys Leu
          1               5                   10

gct gag caa gct gaa cgt tat gag gaa atg gtt gag ttc atg gag aaa      159
Ala Glu Gln Ala Glu Arg Tyr Glu Glu Met Val Glu Phe Met Glu Lys
15                  20                  25                  30

gtt gca aag act gtt gac acc gat gag ctt act gtc gaa gag aga aac 207
Val Ala Lys Thr Val Asp Thr Asp Glu Leu Thr Val Glu Glu Arg Asn
                35                  40                  45

ctc ttg tct gtt gct tac aag aac gtc att ggt gct agg aga gct tcc 255
Leu Leu Ser Val Ala Tyr Lys Asn Val Ile Gly Ala Arg Arg Ala Ser
            50                  55                  60

tgg agg atc ata tct tcc att gaa cag aag gaa gaa agc aga gga aac 303
Trp Arg Ile Ile Ser Ser Ile Glu Gln Lys Glu Glu Ser Arg Gly Asn
        65                  70                  75

gat gat cat gtt tcc att atc aag gac tac aga gga aag atc gaa act 351
Asp Asp His Val Ser Ile Ile Lys Asp Tyr Arg Gly Lys Ile Glu Thr
    80                  85                  90

gaa ctc agc aaa atc tgt gat gga ata ctc aat ctt ctg gat tct cac 399
Glu Leu Ser Lys Ile Cys Asp Gly Ile Leu Asn Leu Leu Asp Ser His
95                  100                 105                 110

ctt gtt ccc act gca tct ttg gcc gag tcc aaa gtc ttt tac ctc aaa 447
Leu Val Pro Thr Ala Ser Leu Ala Glu Ser Lys Val Phe Tyr Leu Lys
                115                 120                 125

atg aaa gga gat tac cac agg tac ctt gct gag ttt aag act gga gct 495
Met Lys Gly Asp Tyr His Arg Tyr Leu Ala Glu Phe Lys Thr Gly Ala
            130                 135                 140

gag agg aaa gaa gct gct gag agc act ctg gtt gct tac aag tca gct 543
Glu Arg Lys Glu Ala Ala Glu Ser Thr Leu Val Ala Tyr Lys Ser Ala
        145                 150                 155

cag gat att gca ctt gct gat tta gct cct act cat ccg att aga ctg 591
Gln Asp Ile Ala Leu Ala Asp Leu Ala Pro Thr His Pro Ile Arg Leu
    160                 165                 170

gga ctt gct ctt aac ttc tct gtc ttc tac tac gag att ctc aac tca 639
Gly Leu Ala Leu Asn Phe Ser Val Phe Tyr Tyr Glu Ile Leu Asn Ser
175                 180                 185                 190

cct gat cgt gcc tgc agt ctc gca aaa cag gct ttt gat gag gcc att 687
Pro Asp Arg Ala Cys Ser Leu Ala Lys Gln Ala Phe Asp Glu Ala Ile
                195                 200                 205

tct gag ctg gat aca tta gga gaa gaa tca tac aaa gac agt acg ttg 735
Ser Glu Leu Asp Thr Leu Gly Glu Glu Ser Tyr Lys Asp Ser Thr Leu
            210                 215                 220

ata atg caa ctt ctc cgt gac aat ctg acc ctt tgg aac tct gac atc 783
Ile Met Gln Leu Leu Arg Asp Asn Leu Thr Leu Trp Asn Ser Asp Ile
        225                 230                 235

aat gat gag gcg ggc ggt gat gag atc aag gag gcg tca aaa cat gag 831
Asn Asp Glu Ala Gly Gly Asp Glu Ile Lys Glu Ala Ser Lys His Glu
    240                 245                 250

ccg gaa gag ggg aaa cca gct gag aca ggg cag tga ccagagagag 877
Pro Glu Glu Gly Lys Pro Ala Glu Thr Gly Gln
255                 260                 265

agggagagac atttctaagt atgtatggta tggattttcc aaaaacatat gatatgatta 937

ggggatttgt agaagcagag agaaagatct ttatttgatt ttctccaaaa atctctgttc 997

cctttttttt ttattgggtt attaaagctg tttgagtcca aaaaaaaaaa aaaaaaaaaa 1057

aaaaaaaaaa aa 1069

<210> 50
<211> 265
<212> PRT
<213> Arabidopsis thaliana

<400> 50

Met Ser Ser Ser Arg Glu Glu Asn Val Tyr Leu Ala Lys Leu Ala Glu
1               5                   10                  15

Gln Ala Glu Arg Tyr Glu Glu Met Val Glu Phe Met Glu Lys Val Ala
            20                  25                  30

Lys Thr Val Asp Thr Asp Glu Leu Thr Val Glu Glu Arg Asn Leu Leu
        35                  40                  45

Ser Val Ala Tyr Lys Asn Val Ile Gly Ala Arg Arg Ala Ser Trp Arg
    50                  55                  60

Ile Ile Ser Ser Ile Glu Gln Lys Glu Glu Ser Arg Gly Asn Asp Asp
65                  70                  75                  80

His Val Ser Ile Ile Lys Asp Tyr Arg Gly Lys Ile Glu Thr Glu Leu
                85                  90                  95

Ser Lys Ile Cys Asp Gly Ile Leu Asn Leu Leu Asp Ser His Leu Val
            100                 105                 110

Pro Thr Ala Ser Leu Ala Glu Ser Lys Val Phe Tyr Leu Lys Met Lys
        115                 120                 125

Gly Asp Tyr His Arg Tyr Leu Ala Glu Phe Lys Thr Gly Ala Glu Arg
    130                 135                 140

Lys Glu Ala Ala Glu Ser Thr Leu Val Ala Tyr Lys Ser Ala Gln Asp
145                 150                 155                 160

Ile Ala Leu Ala Asp Leu Ala Pro Thr His Pro Ile Arg Leu Gly Leu
                165                 170                 175

Ala Leu Asn Phe Ser Val Phe Tyr Tyr Glu Ile Leu Asn Ser Pro Asp
            180                 185                 190

Arg Ala Cys Ser Leu Ala Lys Gln Ala Phe Asp Glu Ala Ile Ser Glu
        195                 200                 205

Leu Asp Thr Leu Gly Glu Glu Ser Tyr Lys Asp Ser Thr Leu Ile Met
    210                 215                 220

Gln Leu Leu Arg Asp Asn Leu Thr Leu Trp Asn Ser Asp Ile Asn Asp
225                 230                 235                 240

Glu Ala Gly Gly Asp Glu Ile Lys Glu Ala Ser Lys His Glu Pro Glu
                245                 250                 255

Glu Gly Lys Pro Ala Glu Thr Gly Gln
            260                 265

<210>  51
<211>  2240
<212>  DNA
<213>  Arabidopsis thaliana

<220>

<221> CDS
<222> (238)..(2064)
<223> G214

<400> 51
tgagatttct ccatttccgt agcttctggt ctcttttctt tgtttcattg atcaaaagca 60

aatcacttct tcttcttctt cttctcgatt tcttactgtt ttcttatcca acgaaatctg 120

gaattaaaaa tggaatcttt atcgaatcca agctgatttt gtttctttca ttgaatcatc 180

tctctaaagt ggaattttgt aaagagaaga tctgaagttg tgtagaggag cttagtg 237

atg gag aca aat tcg tct gga gaa gat ctg gtt att aag act cgg aag 285
Met Glu Thr Asn Ser Ser Gly Glu Asp Leu Val Ile Lys Thr Arg Lys
1               5                   10                  15

cca tat acg ata aca aag caa cgt gaa agg tgg act gag gaa gaa cat 333
Pro Tyr Thr Ile Thr Lys Gln Arg Glu Arg Trp Thr Glu Glu Glu His
            20                  25                  30

aat aga ttc att gaa gct ttg agg ctt tat ggt aga gca tgg cag aag 381
Asn Arg Phe Ile Glu Ala Leu Arg Leu Tyr Gly Arg Ala Trp Gln Lys
        35                  40                  45

att gaa gaa cat gta gca aca aaa act gct gtc cag ata aga agt cac 429
Ile Glu Glu His Val Ala Thr Lys Thr Ala Val Gln Ile Arg Ser His
    50                  55                  60

gct cag aaa ttt ttc tcc aag gta gag aaa gag gct gaa gct aaa ggt 477
Ala Gln Lys Phe Phe Ser Lys Val Glu Lys Glu Ala Glu Ala Lys Gly
65                  70                  75                  80

gta gct atg ggt caa gcg cta gac ata gct att cct cct cca cgg cct 525
Val Ala Met Gly Gln Ala Leu Asp Ile Ala Ile Pro Pro Pro Arg Pro
                85                  90                  95

aag cgt aaa cca aac aat cct tat cct cga aag acg gga agt gga acg 573
Lys Arg Lys Pro Asn Asn Pro Tyr Pro Arg Lys Thr Gly Ser Gly Thr
            100                 105                 110

atc ctt atg tca aaa acg ggt gtg aat gat gga aaa gag tcc ctt gga 621
Ile Leu Met Ser Lys Thr Gly Val Asn Asp Gly Lys Glu Ser Leu Gly
        115                 120                 125

tca gaa aaa gtg tcg cat cct gag atg gcc aat gaa gat cga caa caa 669
Ser Glu Lys Val Ser His Pro Glu Met Ala Asn Glu Asp Arg Gln Gln
    130                 135                 140

tca aag cct gaa gag aaa act ctg cag gaa gac aac tgt tca gat tgt 717
Ser Lys Pro Glu Glu Lys Thr Leu Gln Glu Asp Asn Cys Ser Asp Cys
145                 150                 155                 160

ttc act cat cag tat ctc tct gct gca tcc tcc atg aat aaa agt tgt 765
Phe Thr His Gln Tyr Leu Ser Ala Ala Ser Ser Met Asn Lys Ser Cys
                165                 170                 175

ata gag aca tca aac gca agc act ttc cgc gag ttc ttg cct tca cgg 813
Ile Glu Thr Ser Asn Ala Ser Thr Phe Arg Glu Phe Leu Pro Ser Arg
            180                 185                 190

gaa gag gga agt cag aat aac agg gta aga aag gag tca aac tca gat 861
Glu Glu Gly Ser Gln Asn Asn Arg Val Arg Lys Glu Ser Asn Ser Asp
        195                 200                 205

ttg aat gca aaa tct ctg gaa aac ggt aat gag caa gga cct cag act 909
Leu Asn Ala Lys Ser Leu Glu Asn Gly Asn Glu Gln Gly Pro Gln Thr
    210                 215                 220

tat ccg atg cat atc cct gtg cta gtg cca ttg ggg agc tca ata aca 957
Tyr Pro Met His Ile Pro Val Leu Val Pro Leu Gly Ser Ser Ile Thr
225                 230                 235                 240

agt tct cta tca cat cct cct tca gag cca gat agt cat ccc cac aca 1005

Ser Ser Leu Ser His Pro Pro Ser Glu Pro Asp Ser His Pro His Thr
                245                 250                 255

gtt gca gga gat tat cag tcg ttt cct aat cat ata atg tca acc ctt 1053
Val Ala Gly Asp Tyr Gln Ser Phe Pro Asn His Ile Met Ser Thr Leu
            260                 265                 270

tta caa aca ccg gct ctt tat act gcc gca act ttc gcc tca tca ttt 1101
Leu Gln Thr Pro Ala Leu Tyr Thr Ala Ala Thr Phe Ala Ser Ser Phe
        275                 280                 285

tgg cct ccc gat tct agt ggt ggc tca cct gtt cca ggg aac tca cct 1149
Trp Pro Pro Asp Ser Ser Gly Gly Ser Pro Val Pro Gly Asn Ser Pro
    290                 295                 300

ccg aat ctg gct gcc atg gcc gca gcc act gtt gca gct gct agt gct 1197
Pro Asn Leu Ala Ala Met Ala Ala Ala Thr Val Ala Ala Ala Ser Ala
305                 310                 315                 320

tgg tgg gct gcc aat gga tta tta cct tta tgt gct cct ctt agt tca 1245
Trp Trp Ala Ala Asn Gly Leu Leu Pro Leu Cys Ala Pro Leu Ser Ser
                325                 330                 335

ggt ggt ttc act agt cat cct cca tct act ttt gga cca tca tgt gat 1293
Gly Gly Phe Thr Ser His Pro Pro Ser Thr Phe Gly Pro Ser Cys Asp
            340                 345                 350

gta gag tac aca aaa gca agc act tta caa cat ggt tct gtg cag agc 1341
Val Glu Tyr Thr Lys Ala Ser Thr Leu Gln His Gly Ser Val Gln Ser
        355                 360                 365

cga gag caa gaa cac tcc gag gca tca aag gct cga tct tca ctg gac 1389
Arg Glu Gln Glu His Ser Glu Ala Ser Lys Ala Arg Ser Ser Leu Asp
    370                 375                 380

tca gag gat gtt gaa aat aag agt aaa cca gtt tgt cat gag cag cct 1437
Ser Glu Asp Val Glu Asn Lys Ser Lys Pro Val Cys His Glu Gln Pro
385                 390                 395                 400

tct gca aca cct gag agt gat gca aag ggt tca gat gga gca gga gac 1485
Ser Ala Thr Pro Glu Ser Asp Ala Lys Gly Ser Asp Gly Ala Gly Asp
                405                 410                 415

aga aaa caa gtt gac cgg tcc tcg tgt ggc tca aac act ccg tcg agt 1533
Arg Lys Gln Val Asp Arg Ser Ser Cys Gly Ser Asn Thr Pro Ser Ser
            420                 425                 430

agt gat gat gtt gag gcg gat gca tca gaa agg caa gag gat ggc acc 1581
Ser Asp Asp Val Glu Ala Asp Ala Ser Glu Arg Gln Glu Asp Gly Thr
        435                 440                 445

aat ggt gag gtg aaa gaa acg aat gaa gac act aat aaa cct caa act 1629
Asn Gly Glu Val Lys Glu Thr Asn Glu Asp Thr Asn Lys Pro Gln Thr
    450                 455                 460

tca gag tcc aat gca cgc cgc agt aga atc agc tcc aat ata acc gat 1677
Ser Glu Ser Asn Ala Arg Arg Ser Arg Ile Ser Ser Asn Ile Thr Asp
465                 470                 475                 480

cca tgg aag tct gtg tct gac gag ggt cga att gcc ttc caa gct ctc 1725
Pro Trp Lys Ser Val Ser Asp Glu Gly Arg Ile Ala Phe Gln Ala Leu
                485                 490                 495

ttc tcc aga gag gta ttg ccg caa agt ttt aca tat cga gaa gaa cac 1773
Phe Ser Arg Glu Val Leu Pro Gln Ser Phe Thr Tyr Arg Glu Glu His
            500                 505                 510

aga gag gaa gaa caa caa caa caa gaa caa aga tat cca atg gca ctt 1821
Arg Glu Glu Glu Gln Gln Gln Gln Glu Gln Arg Tyr Pro Met Ala Leu
        515                 520                 525

gat ctt aac ttc aca gct cag tta aca cca gtt gat gat caa gag gag 1869
Asp Leu Asn Phe Thr Ala Gln Leu Thr Pro Val Asp Asp Gln Glu Glu
    530                 535                 540

aag aga aac aca gga ttt ctt gga atc gga tta gat gct tca aag cta 1917
Lys Arg Asn Thr Gly Phe Leu Gly Ile Gly Leu Asp Ala Ser Lys Leu
545                 550                 555                 560

atg agt aga gga aga aca ggt ttt aaa cca tac aaa aga tgt tcc atg 1965
Met Ser Arg Gly Arg Thr Gly Phe Lys Pro Tyr Lys Arg Cys Ser Met
                565                 570                 575

gaa gcc aaa gaa agt aga atc ctc aac aac aat cct atc att cat gtg 2013
Glu Ala Lys Glu Ser Arg Ile Leu Asn Asn Asn Pro Ile Ile His Val
            580                 585                 590

gaa cag aaa gat ccc aaa cgg atg cgg ttg gaa act caa gct tcc aca 2061
Glu Gln Lys Asp Pro Lys Arg Met Arg Leu Glu Thr Gln Ala Ser Thr
        595                 600                 605

tga gactctattt tcatctgatc tgttgtttgt actctgtttt taagttttca 2114

agaccactgc tacattttct ttttcttttg aggcctttgt atttgtttcc ttgtccatag 2174

tcttcctgta acatttgact ctgtattatt caacaaatca taaactgttt aatctttttt 2234

tttcca 2240

<210> 52
<211> 608
<212> PRT
<213> Arabidopsis thaliana

<400> 52

Met Glu Thr Asn Ser Ser Gly Glu Asp Leu Val Ile Lys Thr Arg Lys
1               5                   10                  15

Pro Tyr Thr Ile Thr Lys Gln Arg Glu Arg Trp Thr Glu Glu Glu His
            20                  25                  30

Asn Arg Phe Ile Glu Ala Leu Arg Leu Tyr Gly Arg Ala Trp Gln Lys
        35                  40                  45

Ile Glu Glu His Val Ala Thr Lys Thr Ala Val Gln Ile Arg Ser His
    50                  55                  60

Ala Gln Lys Phe Phe Ser Lys Val Glu Lys Glu Ala Glu Ala Lys Gly
65                  70                  75                  80

Val Ala Met Gly Gln Ala Leu Asp Ile Ala Ile Pro Pro Pro Arg Pro
                85                  90                  95

Lys Arg Lys Pro Asn Asn Pro Tyr Pro Arg Lys Thr Gly Ser Gly Thr
            100                 105                 110

Ile Leu Met Ser Lys Thr Gly Val Asn Asp Gly Lys Glu Ser Leu Gly
        115                 120                 125

Ser Glu Lys Val Ser His Pro Glu Met Ala Asn Glu Asp Arg Gln Gln
    130                 135                 140

Ser Lys Pro Glu Glu Lys Thr Leu Gln Glu Asp Asn Cys Ser Asp Cys
145                 150                 155                 160

Phe Thr His Gln Tyr Leu Ser Ala Ala Ser Ser Met Asn Lys Ser Cys
                165                 170                 175

Ile Glu Thr Ser Asn Ala Ser Thr Phe Arg Glu Phe Leu Pro Ser Arg
            180                 185                 190

Glu Glu Gly Ser Gln Asn Asn Arg Val Arg Lys Glu Ser Asn Ser Asp
        195                 200                 205

Leu Asn Ala Lys Ser Leu Glu Asn Gly Asn Glu Gln Gly Pro Gln Thr
    210                 215                 220

Tyr Pro Met His Ile Pro Val Leu Val Pro Leu Gly Ser Ser Ile Thr
225                 230                 235                 240

Ser Ser Leu Ser His Pro Pro Ser Glu Pro Asp Ser His Pro His Thr
                245                 250                 255

Val Ala Gly Asp Tyr Gln Ser Phe Pro Asn His Ile Met Ser Thr Leu
            260                 265                 270

Leu Gln Thr Pro Ala Leu Tyr Thr Ala Ala Thr Phe Ala Ser Ser Phe
        275                 280                 285

Trp Pro Pro Asp Ser Ser Gly Gly Ser Pro Val Pro Gly Asn Ser Pro
    290                 295                 300

Pro Asn Leu Ala Ala Met Ala Ala Ala Thr Val Ala Ala Ala Ser Ala
305                 310                 315                 320

Trp Trp Ala Ala Asn Gly Leu Leu Pro Leu Cys Ala Pro Leu Ser Ser
                325                 330                 335

Gly Gly Phe Thr Ser His Pro Pro Ser Thr Phe Gly Pro Ser Cys Asp
            340                 345                 350

Val Glu Tyr Thr Lys Ala Ser Thr Leu Gln His Gly Ser Val Gln Ser
        355                 360                 365

Arg Glu Gln Glu His Ser Glu Ala Ser Lys Ala Arg Ser Ser Leu Asp
    370                 375                 380

Ser Glu Asp Val Glu Asn Lys Ser Lys Pro Val Cys His Glu Gln Pro
385                 390                 395                 400

Ser Ala Thr Pro Glu Ser Asp Ala Lys Gly Ser Asp Gly Ala Gly Asp
                405                 410                 415

Arg Lys Gln Val Asp Arg Ser Ser Cys Gly Ser Asn Thr Pro Ser Ser
            420                 425                 430

Ser Asp Asp Val Glu Ala Asp Ala Ser Glu Arg Gln Glu Asp Gly Thr
        435                 440                 445

Asn Gly Glu Val Lys Glu Thr Asn Glu Asp Thr Asn Lys Pro Gln Thr
    450                 455                 460

Ser Glu Ser Asn Ala Arg Arg Ser Arg Ile Ser Ser Asn Ile Thr Asp
465                 470                 475                 480

Pro Trp Lys Ser Val Ser Asp Glu Gly Arg Ile Ala Phe Gln Ala Leu
                485                 490                 495

Phe Ser Arg Glu Val Leu Pro Gln Ser Phe Thr Tyr Arg Glu Glu His
            500                 505                 510

Arg Glu Glu Glu Gln Gln Gln Gln Glu Gln Arg Tyr Pro Met Ala Leu
        515                 520                 525

Asp Leu Asn Phe Thr Ala Gln Leu Thr Pro Val Asp Asp Gln Glu Glu
    530                 535                 540

Lys Arg Asn Thr Gly Phe Leu Gly Ile Gly Leu Asp Ala Ser Lys Leu
545                 550                 555                 560

Met Ser Arg Gly Arg Thr Gly Phe Lys Pro Tyr Lys Arg Cys Ser Met
                565                 570                 575

Glu Ala Lys Glu Ser Arg Ile Leu Asn Asn Asn Pro Ile Ile His Val
            580                 585                 590

Glu Gln Lys Asp Pro Lys Arg Met Arg Leu Glu Thr Gln Ala Ser Thr
        595                 600                 605

<210> 53
<211> 1155
<212> DNA
<213> Arabidopsis thaliana

<220>
<221> CDS
<222> (76)..(1077)
<223> G1930

<400> 53
attcacatta ctaatctctc aagatttcac aattttcttg tgattttctc tcagtttctt 60

atttcgtttc ataac atg gat gcc atg agt agc gta gac gag agc tct aca 111
Met Asp Ala Met Ser Ser Val Asp Glu Ser Ser Thr
1               5                   10

act aca gat tcc att ccg gcg aga aag tca tcg tct ccg gcg agt tta 159
Thr Thr Asp Ser Ile Pro Ala Arg Lys Ser Ser Ser Pro Ala Ser Leu
        15                  20                  25

cta tat aga atg gga agc gga aca agc gtg gta ctt gat tca gag aac 207
Leu Tyr Arg Met Gly Ser Gly Thr Ser Val Val Leu Asp Ser Glu Asn
    30                  35                  40

ggt gtc gaa gtc gaa gtc gaa gcc gaa tca aga aag ctt cct tct tca 255
Gly Val Glu Val Glu Val Glu Ala Glu Ser Arg Lys Leu Pro Ser Ser
45                  50                  55                  60

aga ttc aaa ggt gtt gtt cct caa cca aat gga aga tgg gga gct cag 303
Arg Phe Lys Gly Val Val Pro Gln Pro Asn Gly Arg Trp Gly Ala Gln
                65                  70                  75

att tac gag aaa cat caa cgc gtg tgg ctt ggt act ttc aac gag gaa 351
Ile Tyr Glu Lys His Gln Arg Val Trp Leu Gly Thr Phe Asn Glu Glu
            80                  85                  90

gac gaa gca gct cgt gct tac gac gtc gcg gct cac cgt ttc cgt ggc 399
Asp Glu Ala Ala Arg Ala Tyr Asp Val Ala Ala His Arg Phe Arg Gly
        95                  100                 105

cgc gat gcc gtt act aat ttc aaa gac acg acg ttc gaa gaa gag gtt     447
Arg Asp Ala Val Thr Asn Phe Lys Asp Thr Thr Phe Glu Glu Glu Val
    110                 115                 120

gag ttc tta aac gcg cat tcg aaa tca gag atc gta gat atg ttg aga     495
Glu Phe Leu Asn Ala His Ser Lys Ser Glu Ile Val Asp Met Leu Arg
125                 130                 135                 140

aaa cac act tac aaa gaa gag tta gac caa agg aaa cgt aac cgt gac     543
Lys His Thr Tyr Lys Glu Glu Leu Asp Gln Arg Lys Arg Asn Arg Asp
                145                 150                 155

ggt aac gga aaa gag acg acg gcg ttt gct ttg gct tcg atg gtg gtt     591
Gly Asn Gly Lys Glu Thr Thr Ala Phe Ala Leu Ala Ser Met Val Val
            160                 165                 170

atg acg ggg ttt aaa acg gcg gag tta ctg ttt gag aaa acg gta acg     639
Met Thr Gly Phe Lys Thr Ala Glu Leu Leu Phe Glu Lys Thr Val Thr
        175                 180                 185

cca agt gac gtc ggg aaa cta aac cgt tta gtt ata cca aaa cac caa     687
Pro Ser Asp Val Gly Lys Leu Asn Arg Leu Val Ile Pro Lys His Gln
    190                 195                 200

gcg gag aaa cat ttt ccg tta ccg tta ggt aat aat aac gtc tcc gtt     735
Ala Glu Lys His Phe Pro Leu Pro Leu Gly Asn Asn Asn Val Ser Val
205                 210                 215                 220

aaa ggt atg ctg ttg aat ttc gaa gac gtt aac ggg aaa gtg tgg agg     783
Lys Gly Met Leu Leu Asn Phe Glu Asp Val Asn Gly Lys Val Trp Arg
                225                 230                 235

ttc cgt tac tct tat tgg aat agt agt caa agt tat gtg ttg acc aaa     831
Phe Arg Tyr Ser Tyr Trp Asn Ser Ser Gln Ser Tyr Val Leu Thr Lys
            240                 245                 250

ggt tgg agt aga ttc gtt aaa gag aag aga ctt tgt gct ggt gat ttg     879
Gly Trp Ser Arg Phe Val Lys Glu Lys Arg Leu Cys Ala Gly Asp Leu
        255                 260                 265

atc agt ttt aaa aga tcc aac gat caa gat caa aaa ttc ttt atc ggg     927
Ile Ser Phe Lys Arg Ser Asn Asp Gln Asp Gln Lys Phe Phe Ile Gly
    270                 275                 280

tgg aaa tcg aaa tcc ggg ttg gat cta gag acg ggt cgg gtt atg aga     975
Trp Lys Ser Lys Ser Gly Leu Asp Leu Glu Thr Gly Arg Val Met Arg
285                 290                 295                 300

ttg ttt ggg gtt gat att tct tta aac gcc gtc gtt gta gtg aag gaa     1023
Leu Phe Gly Val Asp Ile Ser Leu Asn Ala Val Val Val Val Lys Glu
                305                 310                 315

aca acg gag gtg tta atg tcg tcg tta agg tgt aag aag caa cga gtt     1071
Thr Thr Glu Val Leu Met Ser Ser Leu Arg Cys Lys Lys Gln Arg Val
            320                 325                 330

ttg taa taacaattta acaacttggg aaagaaaaaa aagctttttg attttaattt     1127
Leu

ctcttcaacg ttaatcttgc tgagatta                                    1155

<210>  54
<211>  333
<212>  PRT
<213>  Arabidopsis thaliana

<400>  54

Met Asp Ala Met Ser Ser Val Asp Glu Ser Ser Thr Thr Thr Asp Ser
1               5                   10                  15

Ile Pro Ala Arg Lys Ser Ser Ser Pro Ala Ser Leu Leu Tyr Arg Met
            20                  25                  30

Gly Ser Gly Thr Ser Val Val Leu Asp Ser Glu Asn Gly Val Glu Val
        35                  40                  45

Glu Val Glu Ala Glu Ser Arg Lys Leu Pro Ser Ser Arg Phe Lys Gly
    50                  55                  60

Val Val Pro Gln Pro Asn Gly Arg Trp Gly Ala Gln Ile Tyr Glu Lys
65                  70                  75                  80

His Gln Arg Val Trp Leu Gly Thr Phe Asn Glu Glu Asp Glu Ala Ala
                85                  90                  95

Arg Ala Tyr Asp Val Ala Ala His Arg Phe Arg Gly Arg Asp Ala Val
            100                 105                 110

Thr Asn Phe Lys Asp Thr Thr Phe Glu Glu Glu Val Glu Phe Leu Asn
        115                 120                 125

Ala His Ser Lys Ser Glu Ile Val Asp Met Leu Arg Lys His Thr Tyr
    130                 135                 140

Lys Glu Glu Leu Asp Gln Arg Lys Arg Asn Arg Asp Gly Asn Gly Lys
145                 150                 155                 160

Glu Thr Thr Ala Phe Ala Leu Ala Ser Met Val Val Met Thr Gly Phe
                165                 170                 175

Lys Thr Ala Glu Leu Leu Phe Glu Lys Thr Val Thr Pro Ser Asp Val
            180                 185                 190

Gly Lys Leu Asn Arg Leu Val Ile Pro Lys His Gln Ala Glu Lys His
        195                 200                 205

Phe Pro Leu Pro Leu Gly Asn Asn Asn Val Ser Val Lys Gly Met Leu
    210                 215                 220

Leu Asn Phe Glu Asp Val Asn Gly Lys Val Trp Arg Phe Arg Tyr Ser
225                 230                 235                 240

Tyr Trp Asn Ser Ser Gln Ser Tyr Val Leu Thr Lys Gly Trp Ser Arg
                245                 250                 255

Phe Val Lys Glu Lys Arg Leu Cys Ala Gly Asp Leu Ile Ser Phe Lys
            260                 265                 270

Arg Ser Asn Asp Gln Asp Gln Lys Phe Phe Ile Gly Trp Lys Ser Lys
        275                 280                 285

Ser Gly Leu Asp Leu Glu Thr Gly Arg Val Met Arg Leu Phe Gly Val
    290                 295                 300

Asp Ile Ser Leu Asn Ala Val Val Val Val Lys Glu Thr Thr Glu Val
305                 310                 315                 320

Leu Met Ser Ser Leu Arg Cys Lys Lys Gln Arg Val Leu
                325                 330

<210>  55
<211>  1246
<212>  DNA
<213>  Arabidopsis thaliana

<220>
<221>  CDS
<222>  (81)..(1139)
<223>  G9

<400>  55
gtgtttcttc tttctgctaa aaggttataa tttttgtttc ttggtttggt gagaatcttc      60

aagaaactga aacaaagaaa atg gat tct agt tgc ata gac gag ata agt tcc     113
                      Met Asp Ser Ser Cys Ile Asp Glu Ile Ser Ser
                      1               5                   10

tcc act tca gaa tct ttc tcc gcc acc acc gcc aag aag ctc tct cct     161
Ser Thr Ser Glu Ser Phe Ser Ala Thr Thr Ala Lys Lys Leu Ser Pro
            15                  20                  25

cct ccc gcg gcg gcg tta cgc ctc tac cgg atg gga agc ggc ggg agc     209
Pro Pro Ala Ala Ala Leu Arg Leu Tyr Arg Met Gly Ser Gly Gly Ser
        30                  35                  40

agc gtc gtg ttg gat ccc gag aac ggc cta gag acg gag tca cga aag     257
Ser Val Val Leu Asp Pro Glu Asn Gly Leu Glu Thr Glu Ser Arg Lys
    45                  50                  55

cta cca tct tca aaa tac aaa ggt gtt gtt cct cag cct aac gga aga     305
Leu Pro Ser Ser Lys Tyr Lys Gly Val Val Pro Gln Pro Asn Gly Arg
60                  65                  70                  75

tgg gga gct cag atc tac gag aag cac caa cga gta tgg ctc ggg act     353
Trp Gly Ala Gln Ile Tyr Glu Lys His Gln Arg Val Trp Leu Gly Thr
                80                  85                  90

ttc aac gag caa gaa gaa gct gct cgt tcc tac gac atc gca gct tgt     401
Phe Asn Glu Gln Glu Glu Ala Ala Arg Ser Tyr Asp Ile Ala Ala Cys
            95                  100                 105

aga ttc cgt ggc cgc gac gcc gtc gtc aac ttc aag aac gtt ctg gaa     449
Arg Phe Arg Gly Arg Asp Ala Val Val Asn Phe Lys Asn Val Leu Glu
        110                 115                 120

gac ggc gat tta gct ttt ctt gaa gct cac tca aag gcc gag atc gtc     497
Asp Gly Asp Leu Ala Phe Leu Glu Ala His Ser Lys Ala Glu Ile Val
    125                 130                 135

gac atg ttg aga aaa cac act tac gcc gac gag ctt gaa cag aac aat     545
Asp Met Leu Arg Lys His Thr Tyr Ala Asp Glu Leu Glu Gln Asn Asn
140                 145                 150                 155

aaa cgg cag ttg ttt ctc tcc gtc gac gct aac gga aaa cgt aac gga     593
Lys Arg Gln Leu Phe Leu Ser Val Asp Ala Asn Gly Lys Arg Asn Gly
                160                 165                 170

tcg agt act act caa aac gac aaa gtt tta aag acg tgt gaa gtt ctt     641
Ser Ser Thr Thr Gln Asn Asp Lys Val Leu Lys Thr Cys Glu Val Leu
            175                 180                 185

ttc gag aag gct gtt aca cct agc gac gtt ggg aag cta aac cgt ctc     689
Phe Glu Lys Ala Val Thr Pro Ser Asp Val Gly Lys Leu Asn Arg Leu
        190                 195                 200

gtg ata cct aaa caa cac gcc gag aaa cac ttt ccg tta ccg tca ccg     737
Val Ile Pro Lys Gln His Ala Glu Lys His Phe Pro Leu Pro Ser Pro
    205                 210                 215

tca ccg gca gtg act aaa gga gtt ttg atc aac ttc gaa gac gtt aac 785
Ser Pro Ala Val Thr Lys Gly Val Leu Ile Asn Phe Glu Asp Val Asn
220                 225                 230                 235

ggt aaa gtg tgg agg ttc cgt tac tca tac tgg aac agt agt caa agt 833
Gly Lys Val Trp Arg Phe Arg Tyr Ser Tyr Trp Asn Ser Ser Gln Ser
                240                 245                 250

tac gtg ttg acc aag gga tgg agt cga ttc gtc aag gag aag aat ctt 881
Tyr Val Leu Thr Lys Gly Trp Ser Arg Phe Val Lys Glu Lys Asn Leu
            255                 260                 265

cga gcc ggt gat gtt gtt act ttc gag aga tcg acc gga cta gag cgg 929
Arg Ala Gly Asp Val Val Thr Phe Glu Arg Ser Thr Gly Leu Glu Arg
        270                 275                 280

cag tta tat att gat tgg aaa gtt cgg tct ggt ccg aga gaa aac ccg 977
Gln Leu Tyr Ile Asp Trp Lys Val Arg Ser Gly Pro Arg Glu Asn Pro
    285                 290                 295

gtt cag gtg gtg gtt cgg ctt ttc gga gtt gat atc ttt aat gtg acc 1025
Val Gln Val Val Val Arg Leu Phe Gly Val Asp Ile Phe Asn Val Thr
300                 305                 310                 315

acc gtg aag cca aac gac gtc gtg gcc gtt tgc ggt gga aag aga tct 1073
Thr Val Lys Pro Asn Asp Val Val Ala Val Cys Gly Gly Lys Arg Ser
                320                 325                 330

cga gat gtt gat gat atg ttt gcg tta cgg tgt tcc aag aag cag gcg 1121
Arg Asp Val Asp Asp Met Phe Ala Leu Arg Cys Ser Lys Lys Gln Ala
            335                 340                 345

ata atc aat gct ttg tga catatttcct tttccgattt tatgctttcg 1169
Ile Ile Asn Ala Leu
        350

tttttaatt ttttttttg tcaagttgtg taggttgtga ttcatgctag gttgtattta 1229

ggaaaagaga taagacc 1246

<210> 56
<211> 352
<212> PRT
<213> Arabidopsis thaliana

<400> 56

Met Asp Ser Ser Cys Ile Asp Glu Ile Ser Ser Ser Thr Ser Glu Ser
1               5                   10                  15

Phe Ser Ala Thr Thr Ala Lys Lys Leu Ser Pro Pro Pro Ala Ala Ala
            20                  25                  30

Leu Arg Leu Tyr Arg Met Gly Ser Gly Gly Ser Ser Val Val Leu Asp
        35                  40                  45

Pro Glu Asn Gly Leu Glu Thr Glu Ser Arg Lys Leu Pro Ser Ser Lys
    50                  55                  60

Tyr Lys Gly Val Val Pro Gln Pro Asn Gly Arg Trp Gly Ala Gln Ile
65                  70                  75                  80

Tyr Glu Lys His Gln Arg Val Trp Leu Gly Thr Phe Asn Glu Gln Glu
                85                  90                  95

Glu Ala Ala Arg Ser Tyr Asp Ile Ala Ala Cys Arg Phe Arg Gly Arg
            100                 105                 110

Asp Ala Val Val Asn Phe Lys Asn Val Leu Glu Asp Gly Asp Leu Ala
        115                 120                 125

Phe Leu Glu Ala His Ser Lys Ala Glu Ile Val Asp Met Leu Arg Lys
    130                 135                 140

His Thr Tyr Ala Asp Glu Leu Glu Gln Asn Asn Lys Arg Gln Leu Phe
145                 150                 155                 160

Leu Ser Val Asp Ala Asn Gly Lys Arg Asn Gly Ser Ser Thr Thr Gln
                165                 170                 175

Asn Asp Lys Val Leu Lys Thr Cys Glu Val Leu Phe Glu Lys Ala Val
            180                 185                 190

Thr Pro Ser Asp Val Gly Lys Leu Asn Arg Leu Val Ile Pro Lys Gln
        195                 200                 205

His Ala Glu Lys His Phe Pro Leu Pro Ser Pro Ser Pro Ala Val Thr
    210                 215                 220

Lys Gly Val Leu Ile Asn Phe Glu Asp Val Asn Gly Lys Val Trp Arg
225                 230                 235                 240

Phe Arg Tyr Ser Tyr Trp Asn Ser Ser Gln Ser Tyr Val Leu Thr Lys
                245                 250                 255

Gly Trp Ser Arg Phe Val Lys Glu Lys Asn Leu Arg Ala Gly Asp Val
            260                 265                 270

Val Thr Phe Glu Arg Ser Thr Gly Leu Glu Arg Gln Leu Tyr Ile Asp
        275                 280                 285

Trp Lys Val Arg Ser Gly Pro Arg Glu Asn Pro Val Gln Val Val Val
    290                 295                 300

Arg Leu Phe Gly Val Asp Ile Phe Asn Val Thr Thr Val Lys Pro Asn
305                 310                 315                 320

Asp Val Val Ala Val Cys Gly Gly Lys Arg Ser Arg Asp Val Asp Asp
                325                 330                 335

Met Phe Ala Leu Arg Cys Ser Lys Lys Gln Ala Ile Ile Asn Ala Leu
            340                 345                 350

<210>  57
<211>  1239
<212>  DNA
<213>  Arabidopsis thaliana

<220>
<221>  CDS
<222>  (6)..(1091)
<223>  G993

<400>  57
caaat atg gaa tac agc tgt gta gac gac agt agt aca acg tca gaa tct      50
      Met Glu Tyr Ser Cys Val Asp Asp Ser Ser Thr Thr Ser Glu Ser

1                 5                   10                  15

ctc tcc atc tct act act cca aag ccg aca acg acg acg gag aag aaa      98
Leu Ser Ile Ser Thr Thr Pro Lys Pro Thr Thr Thr Thr Glu Lys Lys
                20                  25                  30

ctc tct tct ccg ccg gcg acg tcg atg cgt ctc tac aga atg gga agc      146
Leu Ser Ser Pro Pro Ala Thr Ser Met Arg Leu Tyr Arg Met Gly Ser
            35                  40                  45

ggc gga agc agc gtc gtt ttg gat tca gag aac ggc gtc gag acc gag      194
Gly Gly Ser Ser Val Val Leu Asp Ser Glu Asn Gly Val Glu Thr Glu
        50                  55                  60

tca cgt aag ctt cct tcg tcg aaa tat aaa ggc gtt gtg cct cag cct      242
Ser Arg Lys Leu Pro Ser Ser Lys Tyr Lys Gly Val Val Pro Gln Pro
    65                  70                  75

aac gga aga tgg gga gct cag att tac gag aag cat cag cga gtt tgg      290
Asn Gly Arg Trp Gly Ala Gln Ile Tyr Glu Lys His Gln Arg Val Trp
80                  85                  90                  95

ctc ggt act ttc aac gag gaa gaa gaa gct gcg tct tct tac gac atc      338
Leu Gly Thr Phe Asn Glu Glu Glu Glu Ala Ala Ser Ser Tyr Asp Ile
                100                 105                 110

gcc gtg agg aga ttc cgc ggc cgc gac gcc gtc act aac ttc aaa tct      386
Ala Val Arg Arg Phe Arg Gly Arg Asp Ala Val Thr Asn Phe Lys Ser
            115                 120                 125

caa gtt gat gga aac gac gcc gaa tcg gct ttt ctt gac gct cat tct      434
Gln Val Asp Gly Asn Asp Ala Glu Ser Ala Phe Leu Asp Ala His Ser
        130                 135                 140

aaa gct gag atc gtg gat atg ttg agg aaa cac act tac gcc gat gag      482
Lys Ala Glu Ile Val Asp Met Leu Arg Lys His Thr Tyr Ala Asp Glu
    145                 150                 155

ttt gag cag agt aga cgg aag ttt gtt aac ggc gac gga aaa cgc tct      530
Phe Glu Gln Ser Arg Arg Lys Phe Val Asn Gly Asp Gly Lys Arg Ser
160                 165                 170                 175

ggg ttg gag acg gcg acg tac gga aac gac gct gtt ttg aga gcg cgt      578
Gly Leu Glu Thr Ala Thr Tyr Gly Asn Asp Ala Val Leu Arg Ala Arg
                180                 185                 190

gag gtt ttg ttc gag aag act gtt acg ccg agc gac gtc ggg aag ctg      626
Glu Val Leu Phe Glu Lys Thr Val Thr Pro Ser Asp Val Gly Lys Leu
            195                 200                 205

aac cgt tta gtg ata ccg aaa caa cac gcg gag aag cat ttt ccg tta      674
Asn Arg Leu Val Ile Pro Lys Gln His Ala Glu Lys His Phe Pro Leu
        210                 215                 220

ccg gcg atg acg acg gcg atg ggg atg aat ccg tct ccg acg aaa ggc      722
Pro Ala Met Thr Thr Ala Met Gly Met Asn Pro Ser Pro Thr Lys Gly
    225                 230                 235

gtt ttg att aac ttg gaa gat aga aca ggg aaa gtg tgg cgg ttc cgt      770
Val Leu Ile Asn Leu Glu Asp Arg Thr Gly Lys Val Trp Arg Phe Arg
240                 245                 250                 255

tac agt tac tgg aac agc agt caa agt tac gtg ttg acc aag ggc tgg      818
Tyr Ser Tyr Trp Asn Ser Ser Gln Ser Tyr Val Leu Thr Lys Gly Trp
                260                 265                 270

agc cgg ttc gtt aaa gag aag aat ctt cga gcc ggt gat gtg gtt tgt      866
Ser Arg Phe Val Lys Glu Lys Asn Leu Arg Ala Gly Asp Val Val Cys
            275                 280                 285

ttc gag aga tca acc gga cca gac cgg caa ttg tat atc cac tgg aaa      914
Phe Glu Arg Ser Thr Gly Pro Asp Arg Gln Leu Tyr Ile His Trp Lys
        290                 295                 300

gtc cgg tct agt ccg gtt cag act gtg gtt agg cta ttc gga gtc aac      962

Val Arg Ser Ser Pro Val Gln Thr Val Val Arg Leu Phe Gly Val Asn
    305                 310                 315

att ttc aat gtg agt aac gag aaa cca aac gac gtc gca gta gag tgt 1010
Ile Phe Asn Val Ser Asn Glu Lys Pro Asn Asp Val Ala Val Glu Cys
320                 325                 330                 335

gtt ggc aag aag aga tct cgg gaa gat gat ttg ttt tcg tta ggg tgt 1058
Val Gly Lys Lys Arg Ser Arg Glu Asp Asp Leu Phe Ser Leu Gly Cys
                340                 345                 350

tcc aag aag cag gcg att atc aac atc ttg tga caaattcttt tttttggtt 1111
Ser Lys Lys Gln Ala Ile Ile Asn Ile Leu
            355                 360

tttttcttca atttgtttct cctttttcaa tattttgtat tgaaatgaca agttgtaaat 1171

taggacaaga caagaaaaaa tgacaactag acaaaatagt ttttgtttaa aaaaaaaaaa 1231

aaaaaaa 1239

<210> 58
<211> 361
<212> PRT
<213> Arabidopsis thaliana

<400> 58

Met Glu Tyr Ser Cys Val Asp Asp Ser Ser Thr Thr Ser Glu Ser Leu
1               5                   10                  15

Ser Ile Ser Thr Thr Pro Lys Pro Thr Thr Thr Thr Glu Lys Lys Leu
            20                  25                  30

Ser Ser Pro Pro Ala Thr Ser Met Arg Leu Tyr Arg Met Gly Ser Gly
        35                  40                  45

Gly Ser Ser Val Val Leu Asp Ser Glu Asn Gly Val Glu Thr Glu Ser
    50                  55                  60

Arg Lys Leu Pro Ser Ser Lys Tyr Lys Gly Val Val Pro Gln Pro Asn
65                  70                  75                  80

Gly Arg Trp Gly Ala Gln Ile Tyr Glu Lys His Gln Arg Val Trp Leu
                85                  90                  95

Gly Thr Phe Asn Glu Glu Glu Glu Ala Ala Ser Ser Tyr Asp Ile Ala
            100                 105                 110

Val Arg Arg Phe Arg Gly Arg Asp Ala Val Thr Asn Phe Lys Ser Gln
        115                 120                 125

Val Asp Gly Asn Asp Ala Glu Ser Ala Phe Leu Asp Ala His Ser Lys
    130                 135                 140

Ala Glu Ile Val Asp Met Leu Arg Lys His Thr Tyr Ala Asp Glu Phe
145                 150                 155                 160

Glu Gln Ser Arg Arg Lys Phe Val Asn Gly Asp Gly Lys Arg Ser Gly
                165                 170                 175

Leu Glu Thr Ala Thr Tyr Gly Asn Asp Ala Val Leu Arg Ala Arg Glu
            180                 185                 190

Val Leu Phe Glu Lys Thr Val Thr Pro Ser Asp Val Gly Lys Leu Asn
        195                 200                 205

Arg Leu Val Ile Pro Lys Gln His Ala Glu Lys His Phe Pro Leu Pro
    210                 215                 220

Ala Met Thr Thr Ala Met Gly Met Asn Pro Ser Pro Thr Lys Gly Val
225                 230                 235                 240

Leu Ile Asn Leu Glu Asp Arg Thr Gly Lys Val Trp Arg Phe Arg Tyr
                245                 250                 255

Ser Tyr Trp Asn Ser Ser Gln Ser Tyr Val Leu Thr Lys Gly Trp Ser
            260                 265                 270

Arg Phe Val Lys Glu Lys Asn Leu Arg Ala Gly Asp Val Val Cys Phe
        275                 280                 285

Glu Arg Ser Thr Gly Pro Asp Arg Gln Leu Tyr Ile His Trp Lys Val
    290                 295                 300

Arg Ser Ser Pro Val Gln Thr Val Val Arg Leu Phe Gly Val Asn Ile
305                 310                 315                 320

Phe Asn Val Ser Asn Glu Lys Pro Asn Asp Val Ala Val Glu Cys Val
                325                 330                 335

Gly Lys Lys Arg Ser Arg Glu Asp Asp Leu Phe Ser Leu Gly Cys Ser
            340                 345                 350

Lys Lys Gln Ala Ile Ile Asn Ile Leu
        355                 360

<210> 59
<211> 803
<212> DNA
<213> Arabidopsis thaliana

<220>
<221> CDS
<222> (35)..(658)
<223> G41

<400> 59
ctgatcaatg aactcatttt ctgccttttc tgaa atg ttt ggc tcc gat tac gag 55
Met Phe Gly Ser Asp Tyr Glu
1               5

tct ccg gtt tcc tca ggc ggt gat tac agt ccg aag ctt gcc acg agc 103
Ser Pro Val Ser Ser Gly Gly Asp Tyr Ser Pro Lys Leu Ala Thr Ser
        10                  15                  20

tgc ccc aag aaa cca gcg gga agg aag aag ttt cgt gag act cgt cac 151
Cys Pro Lys Lys Pro Ala Gly Arg Lys Lys Phe Arg Glu Thr Arg His
    25                  30                  35

cca att tac aga gga gtt cgt caa aga aac tcc ggt aag tgg gtg tgt 199
Pro Ile Tyr Arg Gly Val Arg Gln Arg Asn Ser Gly Lys Trp Val Cys
40                  45                  50                  55

gag ttg aga gag cca aac aag aaa acg agg att tgg ctc ggg act ttc 247

Glu Leu Arg Glu Pro Asn Lys Lys Thr Arg Ile Trp Leu Gly Thr Phe
                60                  65                  70

caa acc gct gag atg gca gct cgt gct cac gac gtc gcc gcc ata gct 295
Gln Thr Ala Glu Met Ala Ala Arg Ala His Asp Val Ala Ala Ile Ala
            75                  80                  85

ctc cgt ggc aga tct gcc tgt ctc aat ttc gct gac tcg gct tgg cgg 343
Leu Arg Gly Arg Ser Ala Cys Leu Asn Phe Ala Asp Ser Ala Trp Arg
        90                  95                  100

cta cga atc ccg gaa tca acc tgt gcc aag gaa atc caa aag gcg gcg 391
Leu Arg Ile Pro Glu Ser Thr Cys Ala Lys Glu Ile Gln Lys Ala Ala
    105                 110                 115

gct gaa gcc gcg ttg aat ttt caa gat gag atg tgt cat atg acg acg 439
Ala Glu Ala Ala Leu Asn Phe Gln Asp Glu Met Cys His Met Thr Thr
120                 125                 130                 135

gat gct cat ggt ctt gac atg gag gag acc ttg gtg gag gct att tat 487
Asp Ala His Gly Leu Asp Met Glu Glu Thr Leu Val Glu Ala Ile Tyr
                140                 145                 150

acg ccg gaa cag agc caa gat gcg ttt tat atg gat gaa gag gcg atg 535
Thr Pro Glu Gln Ser Gln Asp Ala Phe Tyr Met Asp Glu Glu Ala Met
            155                 160                 165

ttg ggg atg tct agt ttg ttg gat aac atg gcc gaa ggg atg ctt tta 583
Leu Gly Met Ser Ser Leu Leu Asp Asn Met Ala Glu Gly Met Leu Leu
        170                 175                 180

ccg tcg ccg tcg gtt caa tgg aac tat aat ttt gat gtc gag gga gat 631
Pro Ser Pro Ser Val Gln Trp Asn Tyr Asn Phe Asp Val Glu Gly Asp
    185                 190                 195

gat gac gtg tcc tta tgg agc tat taa aattcgattt ttatttccat 678
Asp Asp Val Ser Leu Trp Ser Tyr
200                 205

ttttggtatt atagcttttt atacatttga tcctttttta gaatggatct tcttcttttt 738

ttggttgtga gaaacgaatg taaatggtaa aagttgttgt caaatgcaaa tgtttttgag 798

tgcag 803

<210> 60
<211> 207
<212> PRT
<213> Arabidopsis thaliana

<400> 60

Met Phe Gly Ser Asp Tyr Glu Ser Pro Val Ser Ser Gly Gly Asp Tyr
1               5                   10                  15

Ser Pro Lys Leu Ala Thr Ser Cys Pro Lys Lys Pro Ala Gly Arg Lys
            20                  25                  30

Lys Phe Arg Glu Thr Arg His Pro Ile Tyr Arg Gly Val Arg Gln Arg
        35                  40                  45

Asn Ser Gly Lys Trp Val Cys Glu Leu Arg Glu Pro Asn Lys Lys Thr
    50                  55                  60

Arg Ile Trp Leu Gly Thr Phe Gln Thr Ala Glu Met Ala Ala Arg Ala
65                  70                  75                  80

His Asp Val Ala Ala Ile Ala Leu Arg Gly Arg Ser Ala Cys Leu Asn
                85                  90                  95

Phe Ala Asp Ser Ala Trp Arg Leu Arg Ile Pro Glu Ser Thr Cys Ala
            100                 105                 110

Lys Glu Ile Gln Lys Ala Ala Ala Glu Ala Ala Leu Asn Phe Gln Asp
        115                 120                 125

Glu Met Cys His Met Thr Thr Asp Ala His Gly Leu Asp Met Glu Glu
    130                 135                 140

Thr Leu Val Glu Ala Ile Tyr Thr Pro Glu Gln Ser Gln Asp Ala Phe
145                 150                 155                 160

Tyr Met Asp Glu Glu Ala Met Leu Gly Met Ser Ser Leu Leu Asp Asn
                165                 170                 175

Met Ala Glu Gly Met Leu Leu Pro Ser Pro Ser Val Gln Trp Asn Tyr
            180                 185                 190

Asn Phe Asp Val Glu Gly Asp Asp Asp Val Ser Leu Trp Ser Tyr
        195                 200                 205

<210> 61
<211> 929
<212> DNA
<213> Arabidopsis thaliana

<220>
<221> CDS
<222> (164)..(805)
<223> G40

<400> 61
cttgaaaaag aatctacctg aaaagaaaaa aaagagagag agatataaat agctttacca 60

agacagatat actatctttt attaatccaa aaagactgag aactctagta actacgtact 120

acttaaacct tatccagttt cttgaaacag agtactctga tca atg aac tca ttt 175
Met Asn Ser Phe
1

tca gct ttt tct gaa atg ttt ggc tcc gat tac gag cct caa ggc gga 223
Ser Ala Phe Ser Glu Met Phe Gly Ser Asp Tyr Glu Pro Gln Gly Gly
5                   10                  15                  20

gat tat tgt ccg acg ttg gcc acg agt tgt ccg aag aaa ccg gcg ggc 271
Asp Tyr Cys Pro Thr Leu Ala Thr Ser Cys Pro Lys Lys Pro Ala Gly
                25                  30                  35

cgt aag aag ttt cgt gag act cgt cac cca att tac aga gga gtt cgt 319
Arg Lys Lys Phe Arg Glu Thr Arg His Pro Ile Tyr Arg Gly Val Arg
            40                  45                  50

caa aga aac tcc ggt aag tgg gtt tct gaa gtg aga gag cca aac aag 367
Gln Arg Asn Ser Gly Lys Trp Val Ser Glu Val Arg Glu Pro Asn Lys
        55                  60                  65

aaa acc agg att tgg ctc ggg act ttc caa acc gct gag atg gca gct 415
Lys Thr Arg Ile Trp Leu Gly Thr Phe Gln Thr Ala Glu Met Ala Ala
    70                  75                  80

cgt gct cac gac gtc gct gca tta gcc ctc cgt ggc cga tca gca tgt 463
Arg Ala His Asp Val Ala Ala Leu Ala Leu Arg Gly Arg Ser Ala Cys
85                  90                  95                  100

ctc aac ttc gct gac tcg gct tgg cgg cta cga atc ccg gag tca aca 511

Leu Asn Phe Ala Asp Ser Ala Trp Arg Leu Arg Ile Pro Glu Ser Thr
                105                 110                 115

tgc gcc aag gat atc caa aaa gcg gct gct gaa gcg gcg ttg gct ttt 559
Cys Ala Lys Asp Ile Gln Lys Ala Ala Ala Glu Ala Ala Leu Ala Phe
            120                 125                 130

caa gat gag acg tgt gat acg acg acc acg aat cat ggc ctg gac atg 607
Gln Asp Glu Thr Cys Asp Thr Thr Thr Thr Asn His Gly Leu Asp Met
        135                 140                 145

gag gag acg atg gtg gaa gct att tat aca ccg gaa cag agc gaa ggt 655
Glu Glu Thr Met Val Glu Ala Ile Tyr Thr Pro Glu Gln Ser Glu Gly
    150                 155                 160

gcg ttt tat atg gat gag gag aca atg ttt ggg atg ccg act ttg ttg 703
Ala Phe Tyr Met Asp Glu Glu Thr Met Phe Gly Met Pro Thr Leu Leu
165                 170                 175                 180

gat aat atg gct gaa ggc atg ctt tta ccg ccg ccg tct gtt caa tgg 751
Asp Asn Met Ala Glu Gly Met Leu Leu Pro Pro Pro Ser Val Gln Trp
                185                 190                 195

aat cat aat tat gac ggc gaa gga gat ggt gac gtg tcg ctt tgg agt 799
Asn His Asn Tyr Asp Gly Glu Gly Asp Gly Asp Val Ser Leu Trp Ser
            200                 205                 210

tac taa tattcgatag tcgtttccat ttttgtacta tagtttgaaa atattctagt 855
Tyr

tccttttttt agaatggttc cttcatttta ttttatttta ttgttgtaga aacgagtgga 915

aaataattca atac 929

<210> 62
<211> 213
<212> PRT
<213> Arabidopsis thaliana

<400> 62

Met Asn Ser Phe Ser Ala Phe Ser Glu Met Phe Gly Ser Asp Tyr Glu
1               5                   10                  15

Pro Gln Gly Gly Asp Tyr Cys Pro Thr Leu Ala Thr Ser Cys Pro Lys
            20                  25                  30

Lys Pro Ala Gly Arg Lys Lys Phe Arg Glu Thr Arg His Pro Ile Tyr
        35                  40                  45

Arg Gly Val Arg Gln Arg Asn Ser Gly Lys Trp Val Ser Glu Val Arg
    50                  55                  60

Glu Pro Asn Lys Lys Thr Arg Ile Trp Leu Gly Thr Phe Gln Thr Ala
65                  70                  75                  80

Glu Met Ala Ala Arg Ala His Asp Val Ala Ala Leu Ala Leu Arg Gly
                85                  90                  95

Arg Ser Ala Cys Leu Asn Phe Ala Asp Ser Ala Trp Arg Leu Arg Ile
            100                 105                 110

Pro Glu Ser Thr Cys Ala Lys Asp Ile Gln Lys Ala Ala Ala Glu Ala
        115                 120                 125

Ala Leu Ala Phe Gln Asp Glu Thr Cys Asp Thr Thr Thr Thr Asn His
    130                 135                 140

Gly Leu Asp Met Glu Glu Thr Met Val Glu Ala Ile Tyr Thr Pro Glu
145                 150                 155                 160

Gln Ser Glu Gly Ala Phe Tyr Met Asp Glu Glu Thr Met Phe Gly Met
                165                 170                 175

Pro Thr Leu Leu Asp Asn Met Ala Glu Gly Met Leu Leu Pro Pro Pro
            180                 185                 190

Ser Val Gln Trp Asn His Asn Tyr Asp Gly Glu Gly Asp Gly Asp Val
        195                 200                 205

Ser Leu Trp Ser Tyr
    210

<210> 63
<211> 908
<212> DNA
<213> Arabidopsis thaliana

<220>
<221> CDS
<222> (119)..(769)
<223> G42

<400> 63
cctgaactag aacagaaaga gagagaaact attatttcag caaaccatac caacaaaaaa 60

gacagagatc ttttagttac cttatccagt ttcttgaaac agagtactct tctgatca 118

atg aac tca ttt tct gct ttt tct gaa atg ttt ggc tcc gat tac gag 166
Met Asn Ser Phe Ser Ala Phe Ser Glu Met Phe Gly Ser Asp Tyr Glu
1               5                   10                  15

tct tcg gtt tcc tca ggc ggt gat tat att ccg acg ctt gcg agc agc 214
Ser Ser Val Ser Ser Gly Gly Asp Tyr Ile Pro Thr Leu Ala Ser Ser
            20                  25                  30

tgc ccc aag aaa ccg gcg ggt cgt aag aag ttt cgt gag act cgt cac 262
Cys Pro Lys Lys Pro Ala Gly Arg Lys Lys Phe Arg Glu Thr Arg His
        35                  40                  45

cca ata tac aga gga gtt cgt cgg aga aac tcc ggt aag tgg gtt tgt 310
Pro Ile Tyr Arg Gly Val Arg Arg Arg Asn Ser Gly Lys Trp Val Cys
    50                  55                  60

gag gtt aga gaa cca aac aag aaa aca agg att tgg ctc gga aca ttt 358
Glu Val Arg Glu Pro Asn Lys Lys Thr Arg Ile Trp Leu Gly Thr Phe
65                  70                  75                  80

caa acc gct gag atg gca gct cga gct cac gac gtt gcc gct tta gcc 406
Gln Thr Ala Glu Met Ala Ala Arg Ala His Asp Val Ala Ala Leu Ala
                85                  90                  95

ctt cgt ggc cga tca gcc tgt ctc aat ttc gct gac tcg gct tgg aga 454
Leu Arg Gly Arg Ser Ala Cys Leu Asn Phe Ala Asp Ser Ala Trp Arg
            100                 105                 110

ctc cga atc ccg gaa tca act tgc gct aag gac atc caa aag gcg gcg 502
Leu Arg Ile Pro Glu Ser Thr Cys Ala Lys Asp Ile Gln Lys Ala Ala
        115                 120                 125

gct gaa gct gcg ttg gcg ttt cag gat gag atg tgt gat gcg acg acg 550
Ala Glu Ala Ala Leu Ala Phe Gln Asp Glu Met Cys Asp Ala Thr Thr
    130                 135                 140

gat cat ggc ttc gac atg gag gag acg ttg gtg gag gct att tac acg 598
Asp His Gly Phe Asp Met Glu Glu Thr Leu Val Glu Ala Ile Tyr Thr
145                 150                 155                 160

gcg gaa cag agc gaa aat gcg ttt tat atg cac gat gag gcg atg ttt 646
Ala Glu Gln Ser Glu Asn Ala Phe Tyr Met His Asp Glu Ala Met Phe
                165                 170                 175

gag atg ccg agt ttg ttg gct aat atg gca gaa ggg atg ctt ttg ccg 694
Glu Met Pro Ser Leu Leu Ala Asn Met Ala Glu Gly Met Leu Leu Pro
            180                 185                 190

ctt ccg tcc gta cag tgg aat cat aat cat gaa gtc gac ggc gat gat 742
Leu Pro Ser Val Gln Trp Asn His Asn His Glu Val Asp Gly Asp Asp
        195                 200                 205

gac gac gta tcg tta tgg agt tat taa aactcagatt attatttcca 789
Asp Asp Val Ser Leu Trp Ser Tyr
    210                 215

tttttagtac gatacttttt attttattat tatttttaga tcctttttta gaatggaatc 849

tncattatgt ttgtaaaact gagaaacgag tgtaaattaa attgattcag tttcagtat 908

<210> 64
<211> 216
<212> PRT
<213> Arabidopsis thaliana

<400> 64

Met Asn Ser Phe Ser Ala Phe Ser Glu Met Phe Gly Ser Asp Tyr Glu
1               5                   10                  15

Ser Ser Val Ser Ser Gly Gly Asp Tyr Ile Pro Thr Leu Ala Ser Ser
            20                  25                  30

Cys Pro Lys Lys Pro Ala Gly Arg Lys Lys Phe Arg Glu Thr Arg His
        35                  40                  45

Pro Ile Tyr Arg Gly Val Arg Arg Arg Asn Ser Gly Lys Trp Val Cys
    50                  55                  60

Glu Val Arg Glu Pro Asn Lys Lys Thr Arg Ile Trp Leu Gly Thr Phe
65                  70                  75                  80

Gln Thr Ala Glu Met Ala Ala Arg Ala His Asp Val Ala Ala Leu Ala
                85                  90                  95

Leu Arg Gly Arg Ser Ala Cys Leu Asn Phe Ala Asp Ser Ala Trp Arg
            100                 105                 110

Leu Arg Ile Pro Glu Ser Thr Cys Ala Lys Asp Ile Gln Lys Ala Ala
        115                 120                 125

Ala Glu Ala Ala Leu Ala Phe Gln Asp Glu Met Cys Asp Ala Thr Thr
    130                 135                 140

Asp His Gly Phe Asp Met Glu Glu Thr Leu Val Glu Ala Ile Tyr Thr
145                 150                 155                 160

Ala Glu Gln Ser Glu Asn Ala Phe Tyr Met His Asp Glu Ala Met Phe
                165                 170                 175

Glu Met Pro Ser Leu Leu Ala Asn Met Ala Glu Gly Met Leu Leu Pro
            180                 185                 190

Leu Pro Ser Val Gln Trp Asn His Asn His Glu Val Asp Gly Asp Asp
        195                 200                 205

Asp Asp Val Ser Leu Trp Ser Tyr
    210                 215

<210> 65
<211> 1407
<212> DNA
<213> Arabidopsis thaliana

<220>
<221> CDS
<222> (191)..(1351)
<223> G1127

<400> 65
gacagactct ctctgtatgt gtgcgagaag cgagaagcga gagagagaga gagagagttg 60

ttagctcaca cgctttctct attttctcgg aattcacaaa acagaaagtt tcatccttta 120

cgagaattaa gccgaaagaa acaatctttg agtttgattt cttcttcctt ccttctctct 180

ctctgctcta atg gat tcc aga gac atc cca ccg tca cat aac cag ctt 229
Met Asp Ser Arg Asp Ile Pro Pro Ser His Asn Gln Leu
1               5                   10

caa cca cca ccg gga atg tta atg tct cat tac cgt aac cct aac gcc 277
Gln Pro Pro Pro Gly Met Leu Met Ser His Tyr Arg Asn Pro Asn Ala
    15                  20                  25

gcc gct tca cca tta atg gtt ccc act tcc aca tct caa ccg att caa 325
Ala Ala Ser Pro Leu Met Val Pro Thr Ser Thr Ser Gln Pro Ile Gln
30                  35                  40                  45

cac cct cgt ctt cct ttt ggc aat caa caa caa tct caa acg ttt cat 373
His Pro Arg Leu Pro Phe Gly Asn Gln Gln Gln Ser Gln Thr Phe His
                50                  55                  60

cag cag caa caa caa caa atg gat cag aag act ctt gaa tct ctt gga 421
Gln Gln Gln Gln Gln Gln Met Asp Gln Lys Thr Leu Glu Ser Leu Gly
            65                  70                  75

ttt ggt gat gga tca cct tct tct caa ccg atg cga ttc ggg atc gat 469
Phe Gly Asp Gly Ser Pro Ser Ser Gln Pro Met Arg Phe Gly Ile Asp
        80                  85                  90

gat cag aat cag caa ctg caa gtg aag aag aag cga gga agg ccg aga 517
Asp Gln Asn Gln Gln Leu Gln Val Lys Lys Lys Arg Gly Arg Pro Arg
    95                  100                 105

aag tat act cct gat ggt agc att gct tta ggt tta gct cct acg tct 565
Lys Tyr Thr Pro Asp Gly Ser Ile Ala Leu Gly Leu Ala Pro Thr Ser
110                 115                 120                 125

cct ctt ctc tct gca gct tct aat tct tac ggt gag ggt ggt gtt gga 613
Pro Leu Leu Ser Ala Ala Ser Asn Ser Tyr Gly Glu Gly Gly Val Gly
                130                 135                 140

gat agt ggt gga aat gga aac tct gtt gat cca cct gtt aaa cgt aac 661
Asp Ser Gly Gly Asn Gly Asn Ser Val Asp Pro Pro Val Lys Arg Asn
            145                 150                 155

aga gga agg cct cct ggt tct agt aag aaa cag ctt gat gct tta gga 709
Arg Gly Arg Pro Pro Gly Ser Ser Lys Lys Gln Leu Asp Ala Leu Gly
        160                 165                 170

gga act tca gga gtt ggg ttt aca cct cat gtc att gaa gtg aac aca 757
Gly Thr Ser Gly Val Gly Phe Thr Pro His Val Ile Glu Val Asn Thr
    175                 180                 185

gga gag gac ata gcg tca aag gtg atg gct ttt tcg gat caa ggg tca 805
Gly Glu Asp Ile Ala Ser Lys Val Met Ala Phe Ser Asp Gln Gly Ser
190                 195                 200                 205

aga aca att tgt att ctc tct gca agt ggt gca gtt tct aga gtg atg 853
Arg Thr Ile Cys Ile Leu Ser Ala Ser Gly Ala Val Ser Arg Val Met
                210                 215                 220

ctt cgt caa gct tct cat tct agt gga atc gtt act tat gag gga cga 901
Leu Arg Gln Ala Ser His Ser Ser Gly Ile Val Thr Tyr Glu Gly Arg
            225                 230                 235

ttt gag atc att act ctc tca ggc tca gtc ttg aat tat gag gta aat 949
Phe Glu Ile Ile Thr Leu Ser Gly Ser Val Leu Asn Tyr Glu Val Asn
        240                 245                 250

ggt tcc acc aac aga agt ggt aac ttg agt gtg gct ttg gct gga cct 997
Gly Ser Thr Asn Arg Ser Gly Asn Leu Ser Val Ala Leu Ala Gly Pro
    255                 260                 265

gat ggc ggc atc gta ggt ggc agt gta gtt ggt aat cta gta gct gca 1045
Asp Gly Gly Ile Val Gly Gly Ser Val Val Gly Asn Leu Val Ala Ala
270                 275                 280                 285

aca caa gtc cag gtg ata gtg gga agc ttt gtt gca gaa gca aag aaa 1093
Thr Gln Val Gln Val Ile Val Gly Ser Phe Val Ala Glu Ala Lys Lys
                290                 295                 300

ccg aaa caa agt agt gtt aac att gct cgg ggg cag aat cct gaa ccg 1141
Pro Lys Gln Ser Ser Val Asn Ile Ala Arg Gly Gln Asn Pro Glu Pro
            305                 310                 315

gct tca gcg ccg gct aac atg ttg aac ttt gga tca gtc tct caa gga 1189
Ala Ser Ala Pro Ala Asn Met Leu Asn Phe Gly Ser Val Ser Gln Gly
        320                 325                 330

cca tcg agc gag tca tca gaa gag aat gag agc ggt tct cct gca atg 1237
Pro Ser Ser Glu Ser Ser Glu Glu Asn Glu Ser Gly Ser Pro Ala Met
    335                 340                 345

cac cgt gac aat aat aat ggg ata tat gga gct caa caa caa caa caa 1285
His Arg Asp Asn Asn Asn Gly Ile Tyr Gly Ala Gln Gln Gln Gln Gln
350                 355                 360                 365

caa caa cct ctt cat cct cat cag atg caa atg tac caa cat ctt tgg 1333
Gln Gln Pro Leu His Pro His Gln Met Gln Met Tyr Gln His Leu Trp
                370                 375                 380

tct aat cat ggt caa taa aatgaagcgg aaattaattt gtttccgttt 1381
Ser Asn His Gly Gln
            385

tggttacggt tatggtttga tttctt 1407

<210> 66
<211> 386
<212> PRT
<213> Arabidopsis thaliana

<400> 66

Met Asp Ser Arg Asp Ile Pro Pro Ser His Asn Gln Leu Gln Pro Pro
1               5                   10                  15

Pro Gly Met Leu Met Ser His Tyr Arg Asn Pro Asn Ala Ala Ala Ser
            20                  25                  30

Pro Leu Met Val Pro Thr Ser Thr Ser Gln Pro Ile Gln His Pro Arg
        35                  40                  45

Leu Pro Phe Gly Asn Gln Gln Gln Ser Gln Thr Phe His Gln Gln Gln
    50                  55                  60

Gln Gln Gln Met Asp Gln Lys Thr Leu Glu Ser Leu Gly Phe Gly Asp
65                  70                  75                  80

Gly Ser Pro Ser Ser Gln Pro Met Arg Phe Gly Ile Asp Asp Gln Asn
                85                  90                  95

Gln Gln Leu Gln Val Lys Lys Lys Arg Gly Arg Pro Arg Lys Tyr Thr
            100                 105                 110

Pro Asp Gly Ser Ile Ala Leu Gly Leu Ala Pro Thr Ser Pro Leu Leu
        115                 120                 125

Ser Ala Ala Ser Asn Ser Tyr Gly Glu Gly Gly Val Gly Asp Ser Gly
    130                 135                 140

Gly Asn Gly Asn Ser Val Asp Pro Pro Val Lys Arg Asn Arg Gly Arg
145                 150                 155                 160

Pro Pro Gly Ser Ser Lys Lys Gln Leu Asp Ala Leu Gly Gly Thr Ser
                165                 170                 175

Gly Val Gly Phe Thr Pro His Val Ile Glu Val Asn Thr Gly Glu Asp
            180                 185                 190

Ile Ala Ser Lys Val Met Ala Phe Ser Asp Gln Gly Ser Arg Thr Ile
        195                 200                 205

Cys Ile Leu Ser Ala Ser Gly Ala Val Ser Arg Val Met Leu Arg Gln
    210                 215                 220

Ala Ser His Ser Ser Gly Ile Val Thr Tyr Glu Gly Arg Phe Glu Ile
225                 230                 235                 240

Ile Thr Leu Ser Gly Ser Val Leu Asn Tyr Glu Val Asn Gly Ser Thr
                245                 250                 255

Asn Arg Ser Gly Asn Leu Ser Val Ala Leu Ala Gly Pro Asp Gly Gly
            260                 265                 270

Ile Val Gly Gly Ser Val Val Gly Asn Leu Val Ala Ala Thr Gln Val
        275                 280                 285

Gln Val Ile Val Gly Ser Phe Val Ala Glu Ala Lys Lys Pro Lys Gln
    290                 295                 300

Ser Ser Val Asn Ile Ala Arg Gly Gln Asn Pro Glu Pro Ala Ser Ala
305                 310                 315                 320

Pro Ala Asn Met Leu Asn Phe Gly Ser Val Ser Gln Gly Pro Ser Ser
                325                 330                 335

Glu Ser Ser Glu Glu Asn Glu Ser Gly Ser Pro Ala Met His Arg Asp
            340                 345                 350

Asn Asn Asn Gly Ile Tyr Gly Ala Gln Gln Gln Gln Gln Gln Gln Pro
        355                 360                 365

Leu His Pro His Gln Met Gln Met Tyr Gln His Leu Trp Ser Asn His
    370                 375                 380

Gly Gln
385

<210> 67
<211> 1020
<212> DNA
<213> Arabidopsis thaliana

<220>
<221> CDS
<222> (1)..(1020)
<223> G2657

<400> 67
atg gat cca gtt caa tct cat gga tca caa agc tct ctt cct cct cct 48
Met Asp Pro Val Gln Ser His Gly Ser Gln Ser Ser Leu Pro Pro Pro
1               5                   10                  15

ttc cat gct aga gat ttc caa tta cat ctt caa caa caa caa caa cat 96
Phe His Ala Arg Asp Phe Gln Leu His Leu Gln Gln Gln Gln Gln His
            20                  25                  30

caa caa caa cat caa caa caa caa caa caa cag ttc ttt ctc cac cat 144
Gln Gln Gln His Gln Gln Gln Gln Gln Gln Gln Phe Phe Leu His His
        35                  40                  45

cat cag caa cca caa aga aac ctt gat caa gat cac gag cag caa gga 192
His Gln Gln Pro Gln Arg Asn Leu Asp Gln Asp His Glu Gln Gln Gly
    50                  55                  60

ggg tca ata ttg aat aga tct atc aag atg gat cgc gaa gag aca agc 240
Gly Ser Ile Leu Asn Arg Ser Ile Lys Met Asp Arg Glu Glu Thr Ser
65                  70                  75                  80

gat aac atg gac aac atc gct aat acc aac agc ggt agc gaa ggt aaa 288
Asp Asn Met Asp Asn Ile Ala Asn Thr Asn Ser Gly Ser Glu Gly Lys
                85                  90                  95

gag atg agt tta cac gga gga gaa gga gga agc ggt ggt gga gga agt 336
Glu Met Ser Leu His Gly Gly Glu Gly Gly Ser Gly Gly Gly Gly Ser
            100                 105                 110

gga gaa cag atg aca aga agg cca aga gga aga cca gca gga tcc aag 384
Gly Glu Gln Met Thr Arg Arg Pro Arg Gly Arg Pro Ala Gly Ser Lys
        115                 120                 125

aac aaa cct aaa gct cca ata atc ata aca aga gac agc gca aac gcg 432
Asn Lys Pro Lys Ala Pro Ile Ile Ile Thr Arg Asp Ser Ala Asn Ala
    130                 135                 140

ctt cga act cac gtc atg gag ata gga gac gga tgt gac ata gtt gac 480
Leu Arg Thr His Val Met Glu Ile Gly Asp Gly Cys Asp Ile Val Asp
145                 150                 155                 160

tgt atg gct acg ttc gct aga cgc cgc caa aga ggc gtt tgc gtt atg 528
Cys Met Ala Thr Phe Ala Arg Arg Arg Gln Arg Gly Val Cys Val Met
                165                 170                 175

agc ggt aca gga agc gtt act aac gtc act ata cgt cag cct gga tcg 576
Ser Gly Thr Gly Ser Val Thr Asn Val Thr Ile Arg Gln Pro Gly Ser

180                 185                 190

cca cct ggc tcg gtg gtt agc ctt cac ggc cgg ttt gaa atc ctc tct     624
Pro Pro Gly Ser Val Val Ser Leu His Gly Arg Phe Glu Ile Leu Ser
    195                 200                 205

ctt tcg gga tct ttc ttg cct ccg cct gcg ccg cct gca gcc acc gga     672
Leu Ser Gly Ser Phe Leu Pro Pro Pro Ala Pro Pro Ala Ala Thr Gly
    210                 215                 220

cta agc gtt tac cta gcc gga gga caa ggg cag gtc gtt gga ggt agt     720
Leu Ser Val Tyr Leu Ala Gly Gly Gln Gly Gln Val Val Gly Gly Ser
225                 230                 235                 240

gtg gtg gga cct ttg ttg tgt tcg ggt cct gtg gtg gtt atg gcg gct     768
Val Val Gly Pro Leu Leu Cys Ser Gly Pro Val Val Val Met Ala Ala
                245                 250                 255

tct ttt agc aat gcg gcg tac gaa agg ctg cct ttg gaa gaa gat gag     816
Ser Phe Ser Asn Ala Ala Tyr Glu Arg Leu Pro Leu Glu Glu Asp Glu
            260                 265                 270

atg cag acg cca gtt caa gga ggc ggt gga gga gga gga ggt ggt ggt     864
Met Gln Thr Pro Val Gln Gly Gly Gly Gly Gly Gly Gly Gly Gly Gly
        275                 280                 285

gga atg gga tct ccc ccg atg atg gga cag caa caa gct atg gca gct     912
Gly Met Gly Ser Pro Pro Met Met Gly Gln Gln Gln Ala Met Ala Ala
    290                 295                 300

atg gcg gcg gct caa gga cta cca ccg aat ctt ctt ggt tcg gtt cag     960
Met Ala Ala Ala Gln Gly Leu Pro Pro Asn Leu Leu Gly Ser Val Gln
305                 310                 315                 320

ttg cca ccg cca caa cag aat gat cag cag tat tgg tct acg ggt cgg     1008
Leu Pro Pro Pro Gln Gln Asn Asp Gln Gln Tyr Trp Ser Thr Gly Arg
                325                 330                 335

cca ccg tat tga                                                     1020
Pro Pro Tyr

<210>  68
<211>  339
<212>  PRT
<213>  Arabidopsis thaliana

<400>  68
   Met Asp Pro Val Gln Ser His Gly Ser Gln Ser Ser Leu Pro Pro Pro
   1               5                   10                  15
   Phe His Ala Arg Asp Phe Gln Leu His Leu Gln Gln Gln Gln Gln His
               20                  25                  30
   Gln Gln Gln His Gln Gln Gln Gln Gln Gln Gln Phe Phe Leu His His
           35                  40                  45
   His Gln Gln Pro Gln Arg Asn Leu Asp Gln Asp His Glu Gln Gln Gly
       50                  55                  60
   Gly Ser Ile Leu Asn Arg Ser Ile Lys Met Asp Arg Glu Glu Thr Ser
   65                  70                  75                  80
   Asp Asn Met Asp Asn Ile Ala Asn Thr Asn Ser Gly Ser Glu Gly Lys
                   85                  90                  95
   Glu Met Ser Leu His Gly Gly Glu Gly Gly Ser Gly Gly Gly Gly Ser
   100 105 110
   Gly Glu Gln Met Thr Arg Arg Pro Arg Gly Arg Pro Ala Gly Ser Lys
   115 120 125
   Asn Lys Pro Lys Ala Pro Ile Ile Ile Thr Arg Asp Ser Ala Asn Ala
   130 135 140
   Leu Arg Thr His Val Met Glu Ile Gly Asp Gly Cys Asp Ile Val Asp
   145 150 155 160
   Cys Met Ala Thr Phe Ala Arg Arg Arg Gln Arg Gly Val Cys Val Met
   165 170 175
   Ser Gly Thr Gly Ser Val Thr Asn Val Thr Ile Arg Gln Pro Gly Ser
   180 185 190
   Pro Pro Gly Ser Val Val Ser Leu His Gly Arg Phe Glu Ile Leu Ser
   195 200 205
   Leu Ser Gly Ser Phe Leu Pro Pro Pro Ala Pro Pro Ala Ala Thr Gly
   210 215 220
   Leu Ser Val Tyr Leu Ala Gly Gly Gln Gly Gln Val Val Gly Gly Ser
   225 230 235 240
   Val Val Gly Pro Leu Leu Cys Ser Gly Pro Val Val Val Met Ala Ala
   245 250 255
   Ser Phe Ser Asn Ala Ala Tyr Glu Arg Leu Pro Leu Glu Glu Asp Glu
   260 265 270
   Met Gln Thr Pro Val Gln Gly Gly Gly Gly Gly Gly Gly Gly Gly Gly
   275 280 285
   Gly Met Gly Ser Pro Pro Met Met Gly Gln Gln Gln Ala Met Ala Ala
   290 295 300
   Met Ala Ala Ala Gln Gly Leu Pro Pro Asn Leu Leu Gly Ser Val Gln
   305 310 315 320
   Leu Pro Pro Pro Gln Gln Asn Asp Gln Gln Tyr Trp Ser Thr Gly Arg
   325 330 335
   Pro Pro Tyr

<210>  69
<211>  1558
<212>  DNA
<213>  Arabidopsis thaliana

<220>
<221>  CDS
<222>  (191)..(1396)
<223>  G326

<400>  69

caattaatga catcttcttc ttctcctttc actgcaaaac cgaaagcttg agactttgag 60

attatgtcta tgtcatcttc ttcttcttcc atcgatcact tcatcacctt tcgtcatctt 120

gatcttattc tccactgtat aaaatcagcg agattttaag ggattgtgaa ggtaccatct 180

taaacacaaa atg ggt act tct act aca gag agt gtg gtg gcg tgt gaa 229
Met Gly Thr Ser Thr Thr Glu Ser Val Val Ala Cys Glu
1               5                   10

ttt tgc ggc gag aga acg gcg gtt ctg ttt tgt aga gcc gat acg gcg 277
Phe Cys Gly Glu Arg Thr Ala Val Leu Phe Cys Arg Ala Asp Thr Ala
    15                  20                  25

aag ctt tgt ttg cct tgt gac cag cac gtg cac tcg gcg aac ctt ctc 325
Lys Leu Cys Leu Pro Cys Asp Gln His Val His Ser Ala Asn Leu Leu
30                  35                  40                  45

tcg agg aag cat gtt cgt tct cag atc tgt gat aac tgt agc aaa gag 373
Ser Arg Lys His Val Arg Ser Gln Ile Cys Asp Asn Cys Ser Lys Glu
                50                  55                  60

ccg gtg tcc gta cgt tgc ttc aca gat aat ctc gta ttg tgt cag gag 421
Pro Val Ser Val Arg Cys Phe Thr Asp Asn Leu Val Leu Cys Gln Glu
            65                  70                  75

tgt gat tgg gat gtt cac gga agc tgt tcc tcc tcc gcg acg cat gaa 469
Cys Asp Trp Asp Val His Gly Ser Cys Ser Ser Ser Ala Thr His Glu
        80                  85                  90

cgc tcc gcc gtg gaa ggg ttt tca ggt tgt cct tcg gtt ttg gag ctt 517
Arg Ser Ala Val Glu Gly Phe Ser Gly Cys Pro Ser Val Leu Glu Leu
    95                  100                 105

gct gct gtg tgg gga atc gat tta aag ggt aag aag aaa gaa gat gac 565
Ala Ala Val Trp Gly Ile Asp Leu Lys Gly Lys Lys Lys Glu Asp Asp
110                 115                 120                 125

gaa gac gaa ttg act aag aat ttt ggg atg ggg ttg gat tcg tgg ggt 613
Glu Asp Glu Leu Thr Lys Asn Phe Gly Met Gly Leu Asp Ser Trp Gly
                130                 135                 140

tct gga tct aac atc gtt caa gaa ctg att gtt cct tat gat gtg tct 661
Ser Gly Ser Asn Ile Val Gln Glu Leu Ile Val Pro Tyr Asp Val Ser
            145                 150                 155

tgc aaa aag caa agc ttt agc ttt ggg agg tct aag cag gta gtg ttt 709
Cys Lys Lys Gln Ser Phe Ser Phe Gly Arg Ser Lys Gln Val Val Phe
        160                 165                 170

gaa cag ctt gag tta ctg aag aga ggc ttc gtt gaa ggc gaa gga gag 757
Glu Gln Leu Glu Leu Leu Lys Arg Gly Phe Val Glu Gly Glu Gly Glu
    175                 180                 185

att atg gtt ccg gag gga atc aat ggc gga gga agc att tct cag cca 805
Ile Met Val Pro Glu Gly Ile Asn Gly Gly Gly Ser Ile Ser Gln Pro
190                 195                 200                 205

tct ccg acg acg tcg ttt act tct ttg ctt atg tct caa agt ctt tgt 853
Ser Pro Thr Thr Ser Phe Thr Ser Leu Leu Met Ser Gln Ser Leu Cys
                210                 215                 220

ggt aat ggt atg caa tgg aat gct act aat cat agc act ggc cag aac 901
Gly Asn Gly Met Gln Trp Asn Ala Thr Asn His Ser Thr Gly Gln Asn
            225                 230                 235

act cag ata tgg gat ttt aac ttg gga cag tcg agg aac cct gat gaa 949
Thr Gln Ile Trp Asp Phe Asn Leu Gly Gln Ser Arg Asn Pro Asp Glu
        240                 245                 250

cct agt cca gtc gaa act aaa ggc tct act ttc aca ttc aac aac gtt 997
Pro Ser Pro Val Glu Thr Lys Gly Ser Thr Phe Thr Phe Asn Asn Val
    255                 260                 265

act cat ctc aag aac gat acc cga acc acc aat atg aat gct ttc aaa 1045

Thr His Leu Lys Asn Asp Thr Arg Thr Thr Asn Met Asn Ala Phe Lys
270                 275                 280                 285

gag agt tac cag gag gat tcc gtc cac tca act tct acc aag gga cag      1093
Glu Ser Tyr Gln Glu Asp Ser Val His Ser Thr Ser Thr Lys Gly Gln
                290                 295                 300

gaa aca tct aag agc aac aat att cct gct gcc att cac tcg cat aaa      1141
Glu Thr Ser Lys Ser Asn Asn Ile Pro Ala Ala Ile His Ser His Lys
            305                 310                 315

agt tct aac gac tcc tgt ggc ttg cat tgc acg gaa cat att gct att      1189
Ser Ser Asn Asp Ser Cys Gly Leu His Cys Thr Glu His Ile Ala Ile
        320                 325                 330

act agt aat aga gcc aca aga ttg gtg gcg gta acg aat gct gat cta      1237
Thr Ser Asn Arg Ala Thr Arg Leu Val Ala Val Thr Asn Ala Asp Leu
    335                 340                 345

gag cag atg gca cag aac aga gat aat gct atg cag cgg tac aag gaa      1285
Glu Gln Met Ala Gln Asn Arg Asp Asn Ala Met Gln Arg Tyr Lys Glu
350                 355                 360                 365

aag aag aaa acg cgg aga tat gat aag acc ata aga tat gaa acg agg      1333
Lys Lys Lys Thr Arg Arg Tyr Asp Lys Thr Ile Arg Tyr Glu Thr Arg
                370                 375                 380

aag gcg aga gcc gag acc agg ttg cgt gtt aag ggc aga ttt gtg aaa      1381
Lys Ala Arg Ala Glu Thr Arg Leu Arg Val Lys Gly Arg Phe Val Lys
            385                 390                 395

gct aca gat cct tag atgtctctcc acgttaggtt ttacatttga gatcctaagt      1436
Ala Thr Asp Pro
        400

taggaacttt ttttgttttt tctactttca actaccttgt aaatgtaaat gatcgatctt      1496

cagctgcata atgtgtggcc agatttttgt aatttttacg tttaaccttc taaaaaaaaa      1556

aa                                                                    1558

<210>  70
<211>  401
<212>  PRT
<213>  Arabidopsis thaliana

<400>  70

Met Gly Thr Ser Thr Thr Glu Ser Val Val Ala Cys Glu Phe Cys Gly
1               5                   10                  15

Glu Arg Thr Ala Val Leu Phe Cys Arg Ala Asp Thr Ala Lys Leu Cys
            20                  25                  30

Leu Pro Cys Asp Gln His Val His Ser Ala Asn Leu Leu Ser Arg Lys
        35                  40                  45

His Val Arg Ser Gln Ile Cys Asp Asn Cys Ser Lys Glu Pro Val Ser
    50                  55                  60

Val Arg Cys Phe Thr Asp Asn Leu Val Leu Cys Gln Glu Cys Asp Trp
65                  70                  75                  80

Asp Val His Gly Ser Cys Ser Ser Ser Ala Thr His Glu Arg Ser Ala
                85                  90                  95

Val Glu Gly Phe Ser Gly Cys Pro Ser Val Leu Glu Leu Ala Ala Val
            100                 105                 110

Trp Gly Ile Asp Leu Lys Gly Lys Lys Lys Glu Asp Asp Glu Asp Glu
        115                 120                 125

Leu Thr Lys Asn Phe Gly Met Gly Leu Asp Ser Trp Gly Ser Gly Ser
    130                 135                 140

Asn Ile Val Gln Glu Leu Ile Val Pro Tyr Asp Val Ser Cys Lys Lys
145                 150                 155                 160

Gln Ser Phe Ser Phe Gly Arg Ser Lys Gln Val Val Phe Glu Gln Leu
                165                 170                 175

Glu Leu Leu Lys Arg Gly Phe Val Glu Gly Glu Gly Glu Ile Met Val
            180                 185                 190

Pro Glu Gly Ile Asn Gly Gly Gly Ser Ile Ser Gln Pro Ser Pro Thr
        195                 200                 205

Thr Ser Phe Thr Ser Leu Leu Met Ser Gln Ser Leu Cys Gly Asn Gly
    210                 215                 220

Met Gln Trp Asn Ala Thr Asn His Ser Thr Gly Gln Asn Thr Gln Ile
225                 230                 235                 240

Trp Asp Phe Asn Leu Gly Gln Ser Arg Asn Pro Asp Glu Pro Ser Pro
                245                 250                 255

Val Glu Thr Lys Gly Ser Thr Phe Thr Phe Asn Asn Val Thr His Leu
            260                 265                 270

Lys Asn Asp Thr Arg Thr Thr Asn Met Asn Ala Phe Lys Glu Ser Tyr
        275                 280                 285

Gln Glu Asp Ser Val His Ser Thr Ser Thr Lys Gly Gln Glu Thr Ser
    290                 295                 300

Lys Ser Asn Asn Ile Pro Ala Ala Ile His Ser His Lys Ser Ser Asn
305                 310                 315                 320

Asp Ser Cys Gly Leu His Cys Thr Glu His Ile Ala Ile Thr Ser Asn
                325                 330                 335

Arg Ala Thr Arg Leu Val Ala Val Thr Asn Ala Asp Leu Glu Gln Met
            340                 345                 350

Ala Gln Asn Arg Asp Asn Ala Met Gln Arg Tyr Lys Glu Lys Lys Lys
        355                 360                 365

Thr Arg Arg Tyr Asp Lys Thr Ile Arg Tyr Glu Thr Arg Lys Ala Arg
    370                 375                 380

Ala Glu Thr Arg Leu Arg Val Lys Gly Arg Phe Val Lys Ala Thr Asp
385                 390                 395                 400

Pro

## Description

### RELATED APPLICATION INFORMATION

The present invention claims the benefit from US Provisional Patent Application Serial Nos. 60/166,228 filed November 17, 1999 and 60/197,899 filed April 17, 2000 and "Plant Trait Modification III" filed August 22, 2000.

### FIELD OF THE INVENTION

This invention relates to the field of plant biology. More particularly, the present invention pertains to compositions and methods for phenotypically modifying a plant.

### BACKGROUND OF THE INVENTION

Because sugars are important signaling molecules, the ability to control either the concentration of a signaling sugar or how the plant perceives or responds to a signaling sugar can be used to control plant development, physiology or metabolism. For example, the flux of sucrose (a disaccharide sugar used for systemically transporting carbon and energy in most plants) has been shown to affect gene expression and alter storage compound accumulation in seeds (Wobus et al (1999) Biol. Chem. 380:937-944). Manipulation of the sucrose signaling pathway in seeds may therefore cause seeds to have more protein, oil or carbohydrate, depending on the type of manipulation. Similarly, in tubers, sucrose is converted to starch which is used as an energy store.

It is thought that sugar signaling pathways may partially determine the levels of starch synthesized in the tubers (Zrenner et al. (1996) Plant J. 9:671-681). The manipulation of sugar signaling in tubers could lead to tubers with a higher starch content. Thus, manipulating the sugar signal transduction pathway may lead to altered gene expression to produce plants with desirable traits. In particular, manipulation of sugar signal transduction pathways could be used to alter source-sink relationships in seeds, tubers, roots and other storage organs leading to an increase in yield.

The present invention provides novel transcription factors useful for modifying a plant's phenotype in desirable ways by modifying a plant's sugar-sensing characteristics and thereby, increasing the yield.

### SUMMARY OF THE INVENTION

In a first aspect, the invention relates to a recombinant polynucleotide comprising a nucleotide sequence selected from the group consisting of: (a) a nucleotide sequence encoding a polypeptide comprising a sequence selected from SEQ ID Nos. 2N, where N=1-35, or a complementary nucleotide sequence thereof; (b) a nucleotide sequence encoding a polypeptide comprising a conservatively substituted variant of a polypeptide of (a); (c) a nucleotide sequence comprising a sequence selected from those of SEQ ID Nos. 2N-1, where N=1-35, or a complementary nucleotide sequence thereof; (d) a nucleotide sequence comprising silent substitutions in a nucleotide sequence of (c); (e) a nucleotide sequence which hybridizes under stringent conditions over substantially the entire length of a nucleotide sequence of one or more of: (a), (b), (c), or (d); (f) a nucleotide sequence comprising at least 15 consecutive nucleotides of a sequence of any of (a)-(e); (g) a nucleotide sequence comprising a subsequence or fragment of any of (a)-(f), which subsequence or fragment encodes a polypeptide having a biological activity that modifies a plant's sugar-sensing characteristics; (h) a nucleotide sequence having at least 34% sequence identity to a nucleotide sequence of any of (a)-(g); (i) a nucleotide sequence having at least 60% identity sequence identity to a nucleotide sequence of any of (a)-(g); (j) a nucleotide sequence which encodes a polypeptide having at least 34% identity sequence identity to a polypeptide of SEQ ID Nos. 2N, where N=1-35; (k) a nucleotide sequence which encodes a polypeptide having at least 60% identity sequence identity to a polypeptide of SEQ ID Nos. 2N, where N=1-35; and (1) a nucleotide sequence which encodes a conserved domain of a polypeptide having at least 65% sequence identity to a conserved domain of a polypeptide of SEQ ID Nos. 2N, where N=1-35. The recombinant polynucleotide may further comprise a constitutive, inducible, or tissue-active promoter operably linked to the nucleotide sequence. The invention also relates to compositions comprising at least two of the above described polynucleotides.

In a second aspect, the invention is an isolated or recombinant polypeptide comprising a subsequence of at least about 15 contiguous amino acids encoded by the recombinant or isolated polynucleotide described above.

In another aspect, the invention is a transgenic plant comprising one or more of the above described recombinant polynucleotides. In yet another aspect, the invention is a plant with altered expression levels of a polynucleotide described above or a plant with altered expression or activity levels of an above described polypeptide. Further, the invention is a plant lacking a nucleotide sequence encoding a polypeptide described above. The plant may be a soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet com, tobacco, tomato, watermelon, rosaceous fruits, or vegetable brassicas plant.

In a further aspect, the invention relates to a cloning or expression vector comprising the isolated or recombinant polynucleotide described above or cells comprising the cloning or expression vector.

In yet a further aspect, the invention relates to a composition produced by incubating a polynucleotide of the invention with a nuclease, a restriction enzyme, a polymerase; a polymerase and a primer; a cloning vector, or with a cell.

Furthermore, the invention relates to a method for producing a plant having improved sugar-sensing traits. The method comprises altering the expression of an isolated or recombinant polynucleotide of the invention or altering the expression or activity of a polypeptide of the invention in a plant to produce a modified plant, and selecting the modified plant for modified sugar-sensing traits.

In another aspect, the invention relates to a method of identifying a factor that is modulated by or interacts with a polypeptide encoded by a polynucleotide of the invention. The method comprises expressing a polypeptide encoded by the polynucleotide in a plant; and identifying at least one factor that is modulated by or interacts with the polypeptide. In one embodiment the method for identifying modulating or interacting factors is by detecting binding by the polypeptide to a promoter sequence, or by detecting interactions between an additional protein and the polypeptide in a yeast two hybrid system, or by detecting expression of a factor by hybridization to a microarray, subtractive hybridization or differential display.

In yet another aspect, the invention is a method of identifying a molecule that modulates activity or expression of a polynucleotide or polypeptide of interest. The method comprises placing the molecule in contact with a plant comprising the polynucleotide or polypeptide encoded by the polynucleotide of the invention and monitoring one or more of the expression level of the polynucleotide in the plant, the expression level of the polypeptide in the plant, and modulation of an activity of the polypeptide in the plant.

In yet another aspect, the invention relates to an integrated system, computer or computer readable medium comprising one or more character strings corresponding to a polynucleotide of the invention, or to a polypeptide encoded by the polynucleotide. The integrated system, computer or computer readable medium may comprise a link between one or more sequence strings to a modified plant sugar-sensing trait.

In yet another aspect, the invention is a method for identifying a sequence similar or homologous to one or more polynucleotides of the invention, or one or more polypeptides encoded by the polynucleotides. The method comprises providing a sequence database; and, querying the sequence database with one or more target sequences corresponding to the one or more polynucleotides or to the one or more polypeptides to identify one or more sequence members of the database that display sequence similarity or homology to one or more of the one or more target sequences.

The method may further comprise of linking the one or more of the polynucleotides of the invention, or encoded polypeptides, to a modified plant sugar-sensing phenotype.

### BRIEF DESCRIPTION OF THE DRAWINGS

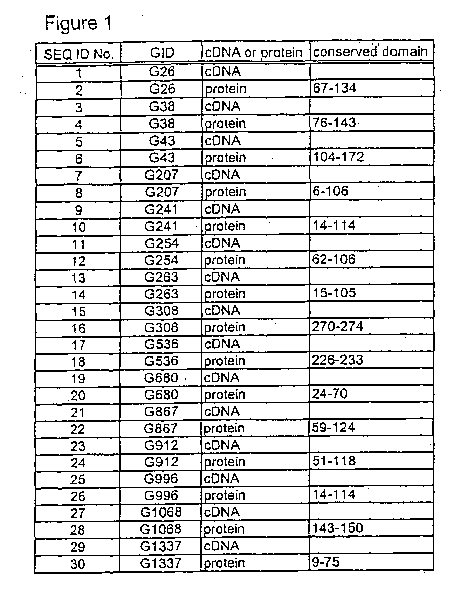

Figure 1 provides a table of exemplary polynucleotide and polypeptide sequences of the invention. The table includes from left to right for each sequence: the SEQ ID No., the internal code reference number (GID), whether the sequence is a polynucleotide or polypeptide sequence, and identification of any conserved domains for the polypeptide sequences.

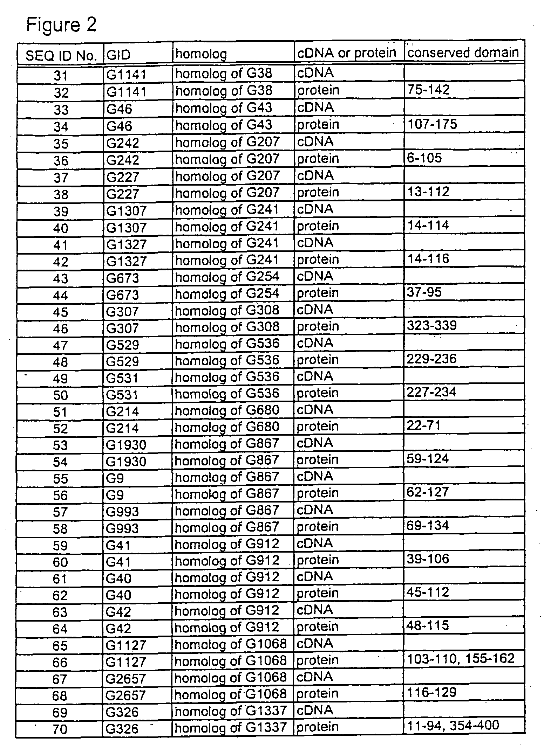

Figure 2 provides a table of exemplary sequences that are homologous to other sequences provided in the Sequence Listing and that are derived from *Arabidopsis thaliana.* The table includes from left to right: the SEQ ID No., the internal code reference number (GID), identification of the homologous sequence, whether the sequence is a polynucleotide or polypeptide sequence, and identification of any conserved domains for the polypeptide sequences.

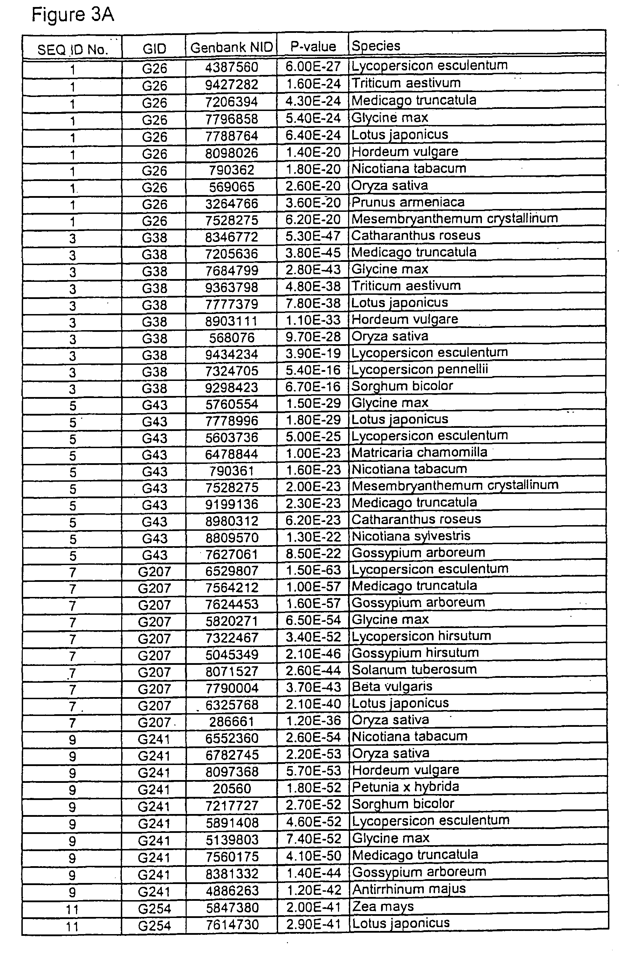

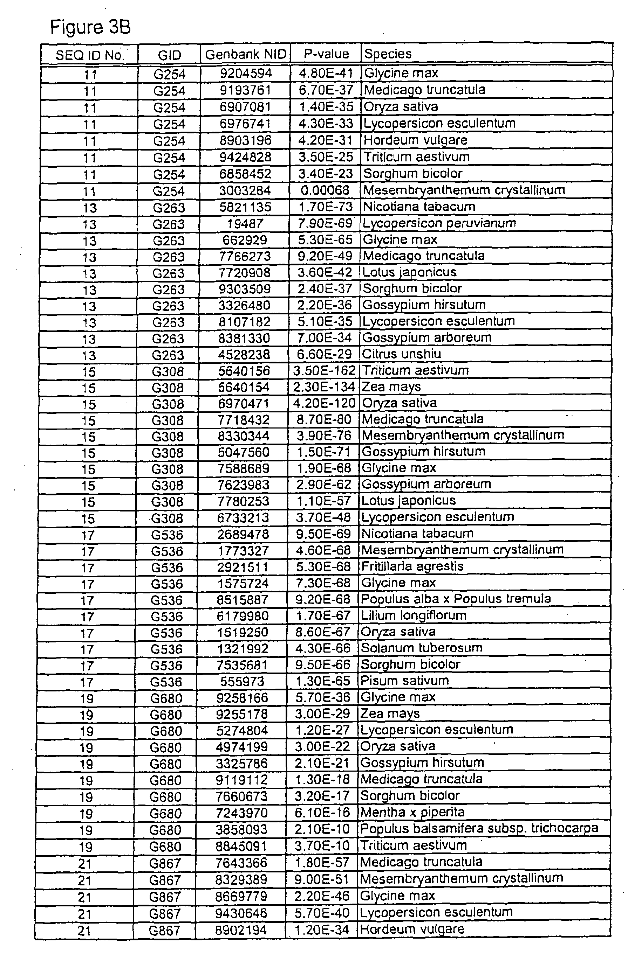

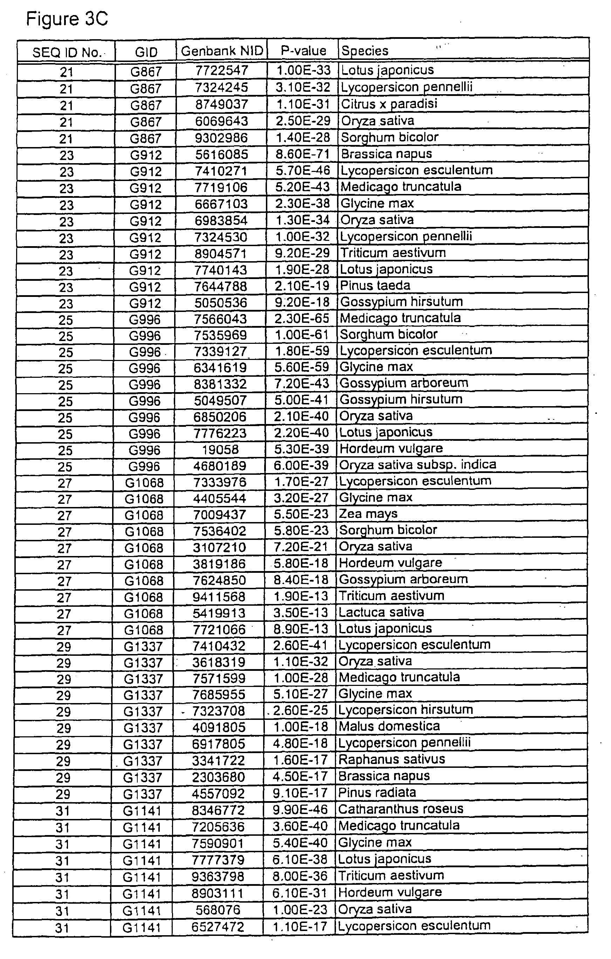

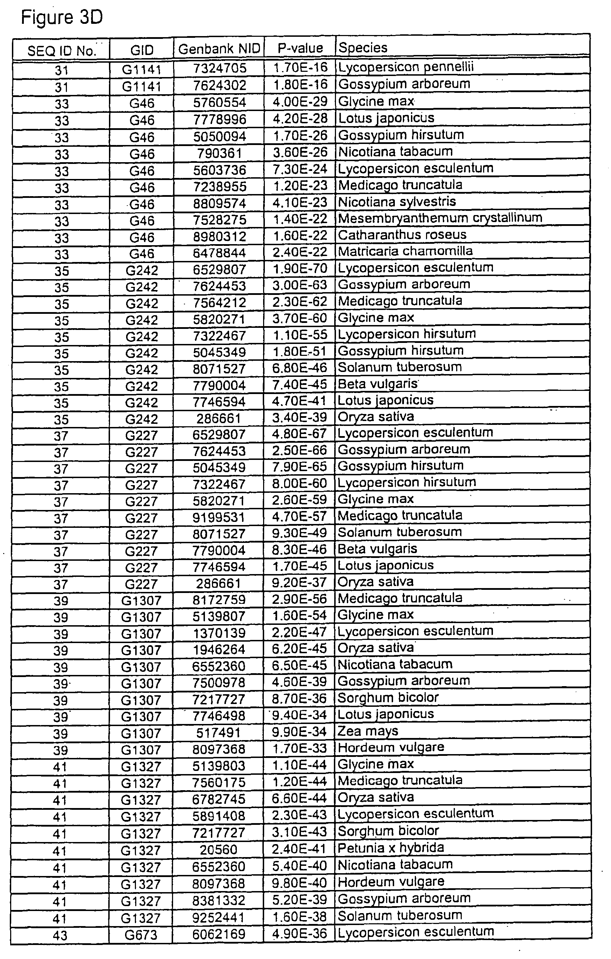

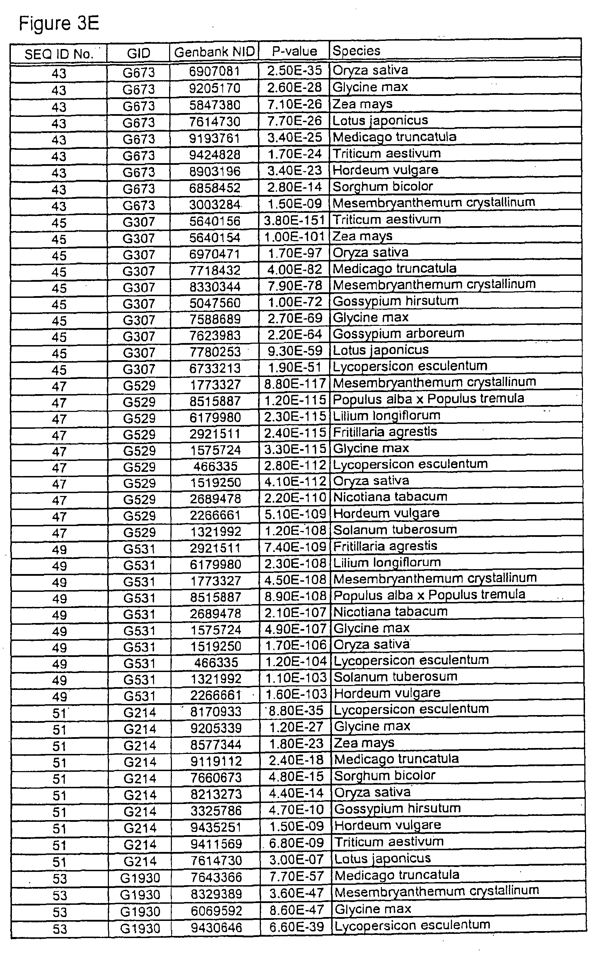

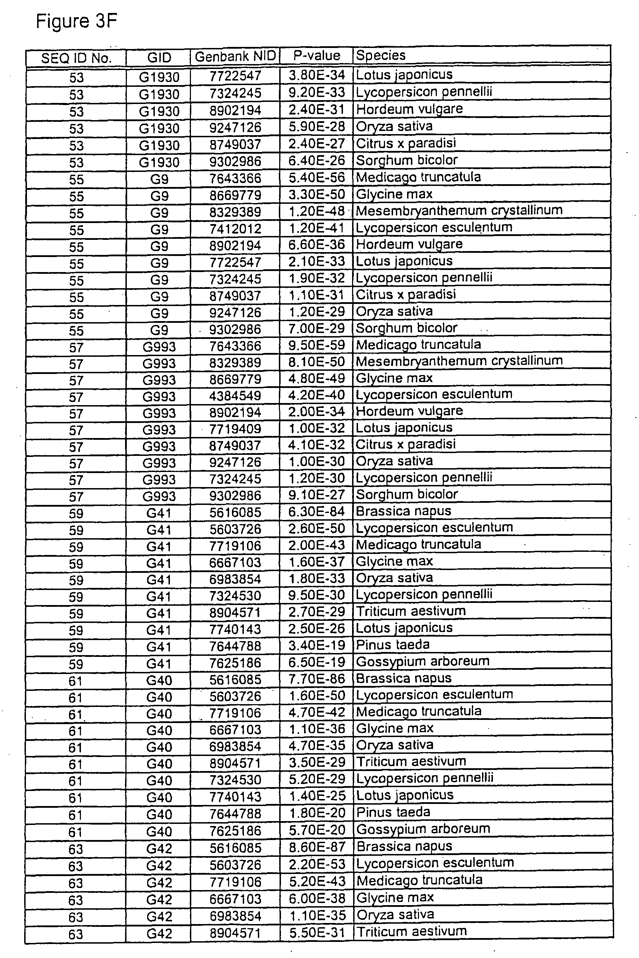

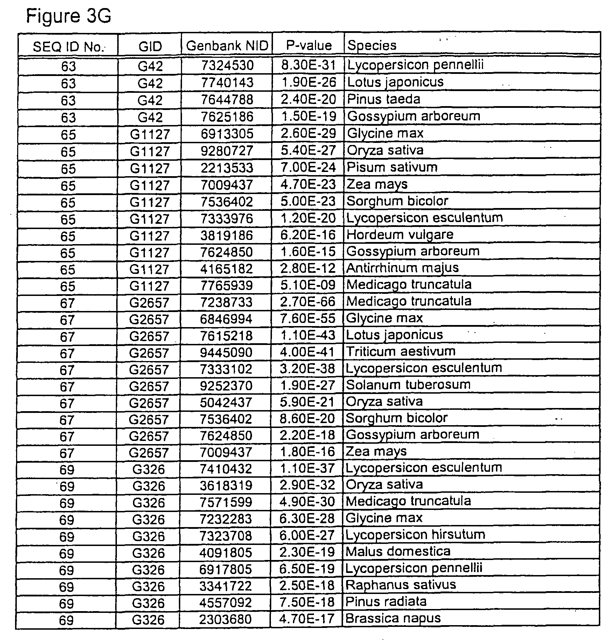

Figure 3 provides a table of exemplary sequences that are homologous to the sequences provided in Figures 1 and 2 and that are derived from plants other than *Arabidopsis thaliana.* The table includes from left to right: the SEQ ID No., the internal code reference number (GID), the unique GenBank sequence ID No. (NID), the probability that the comparison was generated by chance (P-value), and the species from which the homologous gene was identified.

### DETAILED DESCRIPTION

The present invention relates to polynucleotides and polypeptides, e.g. for modifying phenotypes of plants.

In particular, the polynucleotides or polypeptides are useful for modifying traits associated with a plant's sugar-sensing characteristics when the expression levels of the polynucleotides or expression levels or activity levels of the polypeptides are altered. Sugars are central regulatory molecules that control aspects of physiology, metabolism and development. Therefore, the polynucleotides and polypeptides are useful for modifying the growth and germination rates of plants, photosynthesis, glyoxylate metabolism, respiration, starch and sucrose synthesis and degradation, pathogen response, wounding response, cell cycle regulation, pigmentation, flowering and senescense of plants and for modifying sink-source relationships in seeds, tubers, roots and other storage organs leading to an increase in yield.

The polynucleotides of the invention encode plant transcription factors. The plant transcription factors are derived, e.g., from *Arabidopsis thaliana* and can belong, e.g., to one or more of the following transcription factor families: the AP2 (APETALA2) domain transcription factor family (Riechmann and Meyerowitz (1998) J. Biol. Chem. 379:633-646); the MYB transcription factor family (Martin and Paz-Ares (1997) Trends Genet. 13:67-73); the MADS domain transcription factor family (Riechmann and Meyerowitz (1997) J. Biol. Chem. 378:1079-1101); the WRKY protein family (Ishiguro and Nakamura (1994) Mol. Gen. Genet. 244:563-571); the ankyrin-repeat protein family (Zhang et al. (1992) Plant Cell 4:1575-1588); the miscellaneous protein (MISC) family (Kim et al. (1997) Plant J. 11:1237-1251); the zinc finger protein (Z) family (Klug and Schwabe (1995) FASEB J. *9*: 597-604); the homeobox (HB) protein family (Duboule (1994) Guidebook to the Homeobox Genes, Oxford University Press); the CAAT-element binding proteins (Forsburg and Guarente (1989) Genes Dev. 3:1166-1178); the squamosa promoter binding proteins (SPB) (Klein et al. (1996) Mol. Gen. Genet. 1996 250:7-16); the NAM protein family; the IAA/AUX proteins (Rouse et al. (1998) Science 279:1371-1373); the HLH/MYC protein family (Littlewood et al. (1994) Prot. Profile 1:639-709); the DNA-binding protein (DBP) family (Tucker et al. (1994) EMBO J. 13:2994-3002); the bZIP family of transcription factors (Foster et al. (1994) FASEB J. 8:192-200); the BPF-1 protein (Box P-binding factor) family (da Costa e Silva et al. (1993) Plant J. 4:125-135); and the golden protein (GLD) family (Hall et al. (1998) Plant Cell 10:925-936).

In addition to methods for modifying a plant phenotype by employing one or more polynucleotides and polypeptides of the invention described herein, the polynucleotides and polypeptides of the invention have a variety of additional uses. These uses include their use in the recombinant production (i.e, expression) of proteins; as regulators of plant gene expression, as diagnostic probes for the presence of complementary or partially complementary nucleic acids (including for detection of natural coding nucleic acids); as substrates for further reactions, e.g., mutation reactions, PCR reactions, or the like, of as substrates for cloning e.g., including digestion or ligation reactions, and for identifying exogenous or endogenous modulators of the transcription factors.

### DEFINITIONS

A "polynucleotide" is a nucleic acid sequence comprising a plurality of polymerized nucleotide residues, e.g., at least about 15 consecutive polymerized nucleotide residues, optionally at least about 30 consecutive nucleotides, at least about 50 consecutive nucleotides. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single stranded or double stranded DNA or RNA. The polynucleotide optionally comprises modified bases or a modified backbone. The polynucleotide can be, e.g., genomic DNA or RNA, a transcript (such as an mRNA), a cDNA, a PCR product, a cloned DNA, a synthetic DNA or RNA, or the like. The polynucleotide can comprise a sequence in either sense or antisense orientations.

A "recombinant polynucleotide" is a polynucleotide that is not in its native state, e.g., the polynucleotide comprises a nucleotide sequence not found in nature, or the polynucleotide is in a context other than that in which it is naturally found, e.g., separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid.

An "isolated polynucleotide" is a polynucleotide whether naturally occurring or recombinant, that is present outside the cell in which it is typically found in nature, whether purified or not. Optionally, an isolated polynucleotide is subject to one or more enrichment or purification procedures, e.g., cell lysis, extraction, centrifugation, precipitation, or the like.

A "recombinant polypeptide" is a polypeptide produced by translation of a recombinant polynucleotide. An "isolated polypeptide," whether a naturally occurring or a recombinant polypeptide, is more enriched in (or out of) a cell than the polypeptide in its natural state in a wild type cell, e.g., more than about 5% enriched, more than about 10% enriched, or more than about 20%, or more than about 50%, or more, enriched, i.e., alternatively denoted: 105%, 110%, 120%, 150% or more, enriched relative to wild type standardized at 100%. Such an enrichment is not the result of a natural response of a wild type plant. Alternatively, or additionally, the isolated polypeptide is separated from other cellular components with which it is typically associated, e.g., by any of the various protein purification methods herein.

The term "transgenic plant" refers to a plant that contains genetic material, not found in a wild type plant of the same species, variety or cultivar. The genetic material may include a transgene, an insertional mutagenesis event (such as by transposon or T-DNA insertional mutagenesis), an activation tagging sequence, a mutated sequence, a homologous recombination event or a sequence modified by chimeraplasty. Typically, the foreign genetic material has been introduced into the plant by human manipulation.

A transgenic plant may contain an expression vector or cassette. The expression cassette typically comprises a polypeptide-encoding sequence operably linked (i.e., under regulatory control of) to appropriate inducible or constitutive regulatory sequences that allow for the expression of polypeptide. The expression cassette can be introduced into a plant by transformation or by breeding after transformation of a parent plant. A plant refers to a whole plant as well as to a plant part, such as seed, fruit, leaf, or root, plant tissue, plant cells or any other plant material, e.g., a plant explant, as well as to progeny thereof, and to *in vitro* systems that mimic biochemical or cellular components or processes in a cell.

The phrase "ectopically expression or altered expression" in reference to a polynucleotide indicates that the pattern of expression in, e.g., a transgenic plant or plant tissue, is different from the expression pattern in a wild type plant or a reference plant of the same species. For example, the polynucleotide or polypeptide is expressed in a cell or tissue type other than a cell or tissue type in which the sequence is expressed in the wild type plant, or by expression at a time other than at the time the sequence is expressed in the wild type plant, or by a response to different inducible agents, such as hormones or environmental signals, or at different expression levels (either higher or lower) compared with those found in a wild type plant. The term also refers to altered expression patterns that are produced by lowering the levels of expression to below the detection level or completely abolishing expression. The resulting expression pattern can be transient or stable, constitutive or inducible. In reference to a polypeptide, the term "ectopic expression or altered expression" further may relate to altered activity levels resulting from the interactions of the polypeptides with exogenous or endogenous modulators or from interactions with factors or as a result of the chemical modification of the polypeptides.

The term "fragment" or "domain," with respect to a polypeptide, refers to a subsequence of the polypeptide. In some cases, the fragment or domain, is a subsequence of the polypeptide which performs at least one biological function of the intact polypeptide in substantially the same manner, or to a similar extent, as does the intact polypeptide. For example, a polypeptide fragment can comprise a recognizable structural motif or functional domain such as a DNA binding domain that binds to a DNA promoter region, an activation domain or a domain for protein-protein interactions. Fragments can vary in size from as few as 6 amino acids to the full length of the intact polypeptide, but are preferably at least about 30 amino acids in length and more preferably at least about 60 amino acids in length. In reference to a nucleotide sequence, "a fragment" refers to any subsequence of a polynucleotide, typically, of at least consecutive about 15 nucleotides, preferably at least about 30 nucleotides, more preferably at least about 50, of any of the sequences provided herein.

The term "trait" refers to a physiological, morphological, biochemical or physical characteristic of a plant or particular plant material or cell. In some instances, this characteristic is visible to the human eye, such as seed or plant size, or can be measured by available biochemical techniques, such as the protein, starch or oil content of seed or leaves or by the observation of the expression level of genes, e.g., by employing Northern analysis, RT-PCR, microarray gene expression assays or reporter gene expression systems, or by agricultural observations such as stress tolerance, yield or pathogen tolerance.

"Trait modification" refers to a detectable difference in a characteristic in a plant ectopically expressing a polynucleotide or polypeptide of the present invention relative to a plant not doing so, such as a wild type plant. In some cases, the trait modification can be evaluated quantitatively. For example, the trait modification can entail at least about a 2% increase or decrease in an observed trait (difference), at least a 5% difference, at least about a 10% difference, at least about a 20% difference, at least about a 30%, at least about a 50%, at least about a 70%, or at least about a 100%, or an even greater difference. It is known that there can be a natural variation in the modified trait. Therefore, the trait modification observed entails a change of the normal distribution of the trait in the plants compared with the distribution observed in wild type plant.

Trait modifications of particular interest include those to seed (such as embryo or endosperm), fruit, root, flower, leaf, stem, shoot, seedling or the like, including: enhanced tolerance to environmental conditions including freezing, chilling, heat, drought, water saturation, radiation and ozone; improved tolerance to microbial, fungal or viral diseases; improved tolerance to pest infestations, including nematodes, mollicutes, parasitic higher plants or the like; decreased herbicide sensitivity; improved tolerance of heavy metals or enhanced ability to take up heavy metals; improved growth under poor photoconditions (e.g., low light and/or short day length), or changes in expression levels of genes of interest. Other phenotype that can be modified relate to the production of plant metabolites, such as variations in the production of taxol, tocopherol, tocotrienol, sterols, phytosterols, vitamins, wax monomers, anti-oxidants, amino acids, lignins, cellulose, tannins, prenyllipids (such as chlorophylls and carotenoids), glucosinolates, and terpenoids, enhanced or compositionally altered protein or oil production (especially in seeds), or modified sugar (insoluble or soluble) and/or starch composition. Physical plant characteristics that can be modified include cell development (such as the number of trichomes), fruit and seed size and number, yields of plant parts such as stems, leaves and roots, the stability of the seeds during storage, characteristics of the seed pod (e.g., susceptibility to shattering), root hair length and quantity, intemode distances, or the quality of seed coat. Plant growth characteristics that can be modified include growth rate, germination rate of seeds, vigor of plants and seedlings, leaf and flower senescence, male sterility, apomixis, flowering time, flower abscission, rate of nitrogen uptake, biomass or transpiration characteristics, as well as plant architecture characteristics such as apical dominance, branching patterns, number of organs, organ identity, organ shape or size.

### POLYPEPTIDES AND POLYNUCLEOTIDES OF THE INVENTION

The present invention provides, among other things, transcription factors (TFs), and transcription factor homologue polypeptides, and isolated or recombinant polynucleotides encoding the polypeptides. These polypeptides and polynucleotides may be employed to modify a plant's sugar-sensing characteristics..

Exemplary polynucleotides encoding the polypeptides of the invention were identified in the *Arabidopsis thaliana* GenBank database using publicly available sequence analysis programs and parameters. Sequences initially identified were then further characterized to identify sequences comprising specified sequence strings corresponding to sequence motifs present in families of known transcription factors. Polynucleotide sequences meeting such criteria were confirmed as transcription factors.

Additional polynucleotides of the invention were identified by screening *Arabidopsis thaliana* and/or other plant cDNA libraries with probes corresponding to known transcription factors under low stringency hybridization conditions. Additional sequences, including full length coding sequences were subsequently recovered by the rapid amplification of cDNA ends (RACE) procedure, using a commercially available kit according to the manufacturer's instructions. Where necessary, multiple rounds of RACE are performed to isolate 5' and 3' ends. The full length cDNA was then recovered by a routine end-to-end polymerase chain reaction (PCR) using primers specific to the isolated 5' and 3' ends. Exemplary sequences are provided in the Sequence Listing.

The polynucleotides of the invention were ectopically expressed in overexpressor or knockout plants and changes in the sugar-sensing characteristics of the plants were observed. Therefore, the polynucleotides and polypeptides can be employed to improve the sugar-sensing characteristics of plants.

### Making polynucleotides

The polynucleotides of the invention include sequences that encode transcription factors and transcription factor homologue polypeptides and sequences complementary thereto, as well as unique fragments of coding sequence, or sequence complementary thereto. Such polynucleotides can be, e.g., DNA or RNA, e.g., mRNA, cRNA, synthetic RNA, genomic DNA, cDNA synthetic DNA, oligonucleotides, etc. The polynucleotides are either double-stranded or single-stranded, and include either, or both sense (i.e., coding) sequences and antisense (i.e., non-coding, complementary) sequences. The polynucleotides include the coding sequence of a transcription factor, or transcription factor homologue polypeptide, in isolation, in combination with additional coding sequences (e.g., a purification tag, a localization signal, as a fusion-protein, as a pre-protein, or the like), in combination with non-coding sequences (e.g., introns or inteins, regulatory elements such as promoters, enhancers, terminators, and the like), and/or in a vector or host environment in which the polynucleotide encoding a transcription factor or transcription factor homologue polypeptide is an endogenous or exogenous gene.

A variety of methods exist for producing the polynucleotides of the invention. Procedures for identifying and isolating DNA clones are well known to those of skill in the art, and are described in, e.g., Berger and Kimmel, Guide to Molecular Cloning Techniques, Methods in Enzymology volume 152 Academic Press, Inc., San Diego, CA ("Berger"); Sambrook et al., Molecular Cloning - A Laboratory Manual (2nd Ed.), Vol. 1-3, Cold Spring Harbor Laboratory, Cold Spring Harbor, New York, 1989 ("Sambrook") and Current Protocols in Molecular Biology F.M. Ausubel et al., eds., Current Protocols, a joint venture between Greene Publishing Associates, Inc. and John Wiley & Sons, Inc., (supplemented through 2000) ("Ausubel").

Alternatively, polynucleotides of the invention, can be produced by a variety of in vitro amplification methods adapted to the present invention by appropriate selection of specific or degenerate primers. Examples of protocols sufficient to direct persons of skill through in vitro amplification methods, including the polymerase chain reaction (PCR) the ligase chain reaction (LCR), Qbeta-replicase amplification and other RNA polymerase mediated techniques (e.g., NASBA), e.g., for the production of the homologous nucleic acids of the invention are found in Berger, Sambrook, and Ausubel, as well as Mullis et al., (1987) PCR Protocols A Guide to Methods and Applications (Innis et al. eds) Academic Press Inc. San Diego, CA (1990) (Innis). Improved methods for cloning in vitro amplified nucleic acids are described in Wallace et al., U.S. Pat. No. 5,426,039. Improved methods for amplifying large nucleic acids by PCR are summarized in Cheng et al. (1994) Nature 369: 684-685 and the references cited therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. See, e.g., Ausubel, Sambrook and Berger, *all supra.*

Alternatively, polynucleotides and oligonucleotides of the invention can be assembled from fragments produced by solid-phase synthesis methods. Typically, fragments of up to approximately 100 bases are individually synthesized and then enzymatically or chemically ligated to produce a desired sequence, e.g., a polynucletotide encoding all or part of a transcription factor. For example, chemical synthesis using the phosphoramidite method is described, e.g., by Beaucage et al. (1981) Tetrahedron Letters 22:1859-69; and Matthes et al. (1984) EMBO J. 3:801-5. According to such methods, oligonucleotides are synthesized, purified, annealed to their complementary strand, ligated and then optionally cloned into suitable vectors. And if so desired, the polynucleotides and polypeptides of the invention can be custom ordered from any of a number of commercial suppliers.

### HOMOLOGOUS SEQUENCES

Sequences homologous, i.e., that share significant sequence identity or similarity, to those provided in the Sequence Listing, derived from *Arabidopsis thaliana* or from other plants of choice are also an aspect of the invention. Homologous sequences can be derived from any plant including monocots and dicots and in particular agriculturally important plant species, including but not limited to, crops such as soybean, wheat, com, potato, cotton, rice, oilseed rape (including canola), sunflower, alfalfa, sugarcane and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits (such as apple, peach, pear, cherry and plum) and vegetable brassicas (such as broccoli, cabbage, cauliflower, brussel sprouts and kohlrabi). Other crops, fruits and vegetables whose phenotype can be changed include barley, rye, millet; sorghum, currant, avocado, citrus fruits such as oranges, lemons, grapefruit and tangerines, artichoke, cherries, nuts such as the walnut and peanut, endive, leek, roots, such as arrowroot, beet, cassava, turnip, radish, yam, and sweet potato, and beans. The homologous sequences may also be derived from woody species, such pine, poplar and eucalyptus.

Transcription factors that are homologous to the listed sequences will typically share at least about 34% amino acid sequence identity. More closely related transcription factors can share at least about 50%, about 60%, about 65%, about 70%, about 75% or about 80% or about 90% or about 95% or about 98% or more sequence identity with the listed sequences. Factors that are most closely related to the listed sequences share, e.g., at least about 85%, about 90% or about 95% or more % sequence identity to the listed sequences. At the nucleotide level, the sequences will typically share at least about 40% nucleotide sequence identity, preferably at least about 50%, about 60%, about 70% or about 80% sequence identity, and more preferably about 85%, about 90%, about 95% or about 97% or more sequence identity to one or more of the listed sequences. The degeneracy of the genetic code enables major variations in the nucleotide sequence of a polynucleotide while maintaining the amino acid sequence of the encoded protein. Conserved domains within a transcription factor family may exhibit a higher degree of sequence homology, such as at least 65% sequence identity including conservative substitutions, and preferably at least 80% sequence identity.

### Identifying Nucleic Acids by Hybridization

Polynucleotides homologous to the sequences illustrated in the Sequence Listing can be identified, e.g., by hybridization to each other under stringent or under highly stringent conditions. Single stranded polynucleotides hybridize when they associate based on a variety of well characterized physico-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number), as described in more detail in the references cited above.

An example of stringent hybridization conditions for hybridization of complementary nucleic acids which have more than 100 complementary residues on a filter in a Southern or northern blot is about 5°C to 20°C lower than the thermal melting point (Tm) for the specific sequence at a defined ionic strength and pH. The Tₘ is the temperature (under defined ionic strength and pH) at which 50% of the target sequence hybridizes to a perfectly matched probe. Nucleic acid molecules that hybridize under stringent conditions will typically hybridize to a probe based on either the entire cDNA or selected portions, e.g., to a unique subsequence, of the cDNA under wash conditions of 0.2x SSC to 2.0 x SSC, 0.1% SDS at 50-65° C, for example 0.2 x SSC, 0.1% SDS at 65° C. For identification of less closely related homologues washes can be performed at a lower temperature, e.g., 50° C. In general, stringency is increased by raising the wash temperature and/or decreasing the concentration of SSC.

As another example, stringent conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. Conditions can be selected such that a higher signal to noise ratio is observed in the particular assay which is used, e.g., about 15x, 25x, 35x, 50x or more. Accordingly, the subject nucleic acid hybridizes to the unique coding oligonucleotide with at least a 2x higher signal to noise ratio as compared to hybridization of the coding oligonucleotide to a nucleic acid encoding known polypeptide. Again, higher signal to noise ratios can be selected, e.g., about 5x, 10x, 25x, 35x, 50x or more. The particular signal will depend on the label used in the relevant assay, e.g., a fluorescent label, a colorimetric label, a radio active label, or the like.

Alternatively, transcription factor homologue polypeptides can be obtained by screening an expression library using antibodies specific for one or more transcription factors. With the provision herein of the disclosed transcription factor, and transcription factor homologue nucleic acid sequences, the encoded polypeptide(s) can be expressed and purified in a heterologous expression system (e.g., *E. coli)* and used to raise antibodies (monoclonal or polyclonal) specific for the polypeptide(s) in question. Antibodies can also be raised against synthetic peptides derived from transcription factor, or transcription factor homologue, amino acid sequences. Methods of raising antibodies are well known in the art and are described in Harlow and Lane (1988) Antibodies: A Laboratory Manual, Cold Spring Harbor Laboratory, New York. Such antibodies can then be used to screen an expression library produced from the plant from which it is desired to clone additional transcription factor homologues, using the methods described above. The selected cDNAs can be confirmed by sequencing and enzymatic activity.

### SEQUENCE VARIATIONS

It will readily be appreciated by those of skill in the art, that any of a variety of polynucleotide sequences are capable of encoding the transcription factors and transcription factor homologue polypeptides of the invention. Due to the degeneracy of the genetic code, many different polynucleotides can encode identical and/or substantially similar polypeptides in addition to those sequences illustrated in the Sequence Listing.

For example, Table 1 illustrates, e.g., that the codons AGC, AGT, TCA, TCC, TCG, and TCT all encode the same amino acid: serine. Accordingly, at each position in the sequence where there is a codon encoding serine, any of the above trinucleotide sequences can be used without altering the encoded polypeptide.

**Table 1**

| Amino acids | | | Codon | | | | | |
|---|---|---|---|---|---|---|---|---|
| Alanine | Ala | A | GCA | GCC | GCG | GCU | | |
| Cysteine | Cys | C | TGC | TGT | | | | |
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | AGT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

Sequence alterations that do not change the amino acid sequence encoded by the polynucleotide are termed "silent" variations. With the exception of the codons ATG and TGG, encoding methionine and tryptophan, respectively, any of the possible codons for the same amino acid can be substituted by a variety of techniques, e.g., site-directed mutagenesis, available in the art. Accordingly, any and all such variations of a sequence selected from the above table are a feature of the invention.

In addition to silent variations, other conservative variations that alter one, or a few amino acids in the encoded polypeptide, can be made without altering the function of the polypeptide, these conservative variants are, likewise, a feature of the invention.

For example, substitutions, deletions and insertions introduced into the sequences provided in the Sequence Listing are also envisioned by the invention. Such sequence modifications can be engineered into a sequence by site-directed mutagenesis (Wu (ed.) Meth. Enzymol. (1993) vol. 217, Academic Press) or the other methods noted below. Amino acid substitutions are typically of single residues; insertions usually will be on the order of about from 1 to 10 amino acid residues; and deletions will range about from 1 to 39, residues. In preferred embodiments, deletions or insertions are made in adjacent pairs, e.g., a deletion of two residues or insertion of two residues. Substitutions, deletions, insertions or any combination thereof can be combined to arrive at a sequence. The mutations that are made in the polynucleotide encoding the transcription factor should not place the sequence out of reading frame and should not create complementary regions that could produce secondary mRNA structure. Preferably, the polypeptide encoded by the DNA performs the desired function.

Conservative substitutions are those in which at least one residue in the amino acid sequence has been removed and a different residue inserted in its place. Such substitutions generally are made in accordance with the Table 2 when it is desired to maintain the activity of the protein. Table 2 shows amino acids which can be substituted for an amino acid in a protein and which are typically regarded as conservative substitutions.

**Table 2**

| **Residue** | **Conservative Substitutions** |
|---|---|
| Ala | Ser |
| Arg | Lys |
| Asn | Gln; His |
| Asp | Glu |
| Gln | Asn |
| Cys | Ser |
| Glu | Asp |
| Gly | Pro |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr; Gly |
| Thr | Ser;Val |
| Trp | Tyr |
| Tyr | Trp; Phe |
| Val | Ile; Leu |

Substitutions that are less conservative than those in Table 2 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

### FURTHER MODIFYING SEQUENCES OF THE INVENTION-MUTATION/ FORCED EVOLUTION

In addition to generating silent or conservative substitutions as noted, above, the present invention optionally includes methods of modifying the sequences of the Sequence Listing. In the methods, nucleic acid or protein modification methods are used to alter the given sequences to produce new sequences and/or to chemically or enzymatically modify given sequences to change the properties of the nucleic acids or proteins.

Thus, in one embodiment, given nucleic acid sequences are modified, e.g., according to standard mutagenesis or artificial evolution methods to produce modified sequences. For example, Ausubel, *supra,* provides additional details on mutagenesis methods. Artificial forced evolution methods are described, e.g., by Stemmer (1994) Nature 370:389-391, and Stemmer (1994) Proc. Natl. Acad. Sci. USA 91:10747-10751. Many other mutation and evolution methods are also available and expected to be within the skill of the practitioner.

Similarly, chemical or enzymatic alteration of expressed nucleic acids and polypeptides can be performed by standard methods. For example, sequence can be modified by addition of lipids, sugars, peptides, organic or inorganic compounds, by the inclusion of modified nucleotides or amino acids, or the like. For example, protein modification techniques are illustrated in Ausubel, *supra.* Further details on chemical and enzymatic modifications can be found herein. These modification methods can be used to modify any given sequence, or to modify any sequence produced by the various mutation and artificial evolution modification methods noted herein.

Accordingly, the invention provides for modification of any given nucleic acid by mutation, evolution, chemical or enzymatic modification, or other available methods, as well as for the products produced by practicing such methods, e.g., using the sequences herein as a starting substrate for the various modification approaches.

For example, optimized coding sequence containing codons preferred by a particular prokaryotic or eukaryotic host can be used e.g., to increase the rate of translation or to produce recombinant RNA transcripts having desirable properties, such as a longer half-life, as compared with transcripts produced using a non-optimized sequence. Translation stop codons can also be modified to reflect host preference. For example, preferred stop codons for *S. cerevisiae* and mammals are TAA and TGA, respectively. The preferred stop codon for monocotyledonous plants is TGA, whereas insects and *E. coli* prefer to use TAA as the stop codon.

The polynucleotide sequences of the present invention can also be engineered in order to alter a coding sequence for a variety of reasons, including but not limited to, alterations which modify the sequence to facilitate cloning, processing and/or expression of the gene product. For example, alterations are optionally introduced using techniques which are well known in the art, e.g., site-directed mutagenesis, to insert new restriction sites, to alter glycosylation patterns, to change codon preference, to introduce splice sites, etc.

Furthermore, a fragment or domain derived from any of the polypeptides of the invention can be combined with domains derived from other transcription factors or synthetic domains to modify the biological activity of a transcription factor. For instance, a DNA binding domain derived from a transcription factor of the invention can be combined with the activation domain of another transcription factor or with a synthetic activation domain. A transcription activation domain assists in initiating transcription from a DNA binding site. Examples include the transcription activation region of VP16 or GAL4 (Moore et al. (1998) Proc. Natl. Acad. Sci. USA 95: 376-381; and Aoyama et al. (1995) Plant Cell 7:1773-1785), peptides derived from bacterial sequences (Ma and Ptashne (1987) Cell 51; 113-119) and synthetic peptides (Giniger and Ptashne, (1987) Nature 330:670-672).

### EXPRESSION AND MODIFICATION OF POLYPEPTIDES

Typically, polynucleotide sequences of the invention are incorporated into recombinant DNA (or RNA) molecules that direct expression of polypeptides of the invention in appropriate host cells, transgenic plants, in vitro translation systems, or the like. Due to the inherent degeneracy of the genetic code, nucleic acid sequences which encode substantially the same or a functionally equivalent amino acid sequence can be substituted for any listed sequence to provide for cloning and expressing the relevant homologue.

### Vectors, Promoters and Expression Systems

The present invention includes recombinant constructs comprising one or more of the nucleic acid sequences herein. The constructs typically comprise a vector, such as a plasmid, a cosmid, a phage, a virus (e.g., a plant virus), a bacterial artificial chromosome (BAC), a yeast artificial chromosome (YAC), or the like, into which a nucleic acid sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available.

General texts which describe molecular biological techniques useful herein, including the use and production of vectors, promoters and many other relevant topics, include Berger, Sambrook and Ausubel, *supra*. Any of the identified sequences can be incorporated into a cassette or vector, e.g., for expression in plants. A number of expression vectors suitable for stable transformation of plant cells or for the establishment of transgenic plants have been described including those described in Weissbach and Weissbach, (1989) Methods for Plant Molecular Biology, Academic Press, and Gelvin et al., (1990) Plant Molecular Biology Manual, Kluwer Academic Publishers. Specific examples include those derived from a Ti plasmid of *Agrobacterium tumefaciens,* as well as those disclosed by Herrera-Estrella et al. (1983) Nature 303: 209, Bevan (1984) Nucl Acid Res. 12: 8711-8721, Klee (1985) Bio/Technology 3:637-642, for dicotyledonous plants.

Alternatively, non-Ti vectors can be used to transfer the DNA into monocotyledonous plants and cells by using free DNA delivery techniques. Such methods can involve, for example, the use of liposomes, electroporation, microprojectile bombardment, silicon carbide whiskers, and viruses. By using these methods transgenic plants such as wheat, rice (Christou (1991) Bio/technology 9: 957-962) and com (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol 102: 1077-1084; Vasil (1993) Bio/Technology 10: 667-674; Wan and Lemeaux (1994) Plant Physiol 104: 37-48, and for Agrobacterium-mediated DNA transfer (Ishida et al. (1996) Nature Biotech 14: 745-750).

Typically, plant transformation vectors include one or more cloned plant coding sequence (genomic or cDNA) under the transcriptional control of 5' and 3' regulatory sequences and a dominant selectable marker. Such plant transformation vectors typically also contain a promoter (e.g., a regulatory region controlling inducible or constitutive, environmentally-or developmentally-regulated, or cell- or tissue-specific expression), a transcription initiation start site, an RNA processing signal (such as intron splice sites), a transcription termination site, and/or a polyadenylation signal.

Examples of constitutive plant promoters which can be useful for expressing the TF sequence include: the cauliflower mosaic virus (CaMV) 35S promoter, which confers constitutive, high-level expression in most plant tissues (*see,* e.g., Odel et al. (1985) Nature 313:810); the nopaline synthase promoter (An et al. (1988) Plant Physiol 88:547); and the octopine synthase promoter (Fromm et al. (1989) Plant Cell 1: 977).

A variety of plant gene promoters that regulate gene expression in response to environmental, hormonal, chemical, developmental signals, and in a tissue-active manner can be used for expression of a TF sequence in plants. Choice of a promoter is based largely on the phenotype of interest and is determined by such factors as tissue (e.g., seed, fruit, root, pollen, vascular tissue, flower, carpel, etc.), inducibility (e.g., in response to wounding, heat, cold, drought, light, pathogens, etc.), timing, developmental stage, and the like. Numerous known promoters have been characterized and can favorable be employed to promote expression of a polynucleotide of the invention in a transgenic plant or cell of interest. For example, tissue specific promoters include: seed-specific promoters (such as the napin, phaseolin or DC3 promoter described in US Pat. No. 5,773,697), fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) Plant Mol Biol 11:651), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) Plant Mol Biol 37:977-988), flower-specific (Kaiser et al, (1995) Plant Mol Biol 28:231-243), pollen (Baerson et al. (1994) Plant Mol Biol 26:1947-1959), carpels (Ohl et al. (1990) Plant Cell 2:837-848), pollen and ovules (Baerson et al. (1993) Plant Mol Biol 22:255-267), auxin-inducible promoters (such as that described in van der Kop et al. (1999) Plant Mol Biol 39:979-990 or Baumann et al. (1999) Plant Cell 11:323-334), cytokinin-inducible promoter (Guevara-Garcia (1998) Plant Mol Biol 38:743-753), promoters responsive to gibberellin (Shi et al. (1998) Plant Mol Biol 38:1053-1060, Willmott et al. (1998) 38:817-825) and the like. Additional promoters are those that elicit expression in response to heat (Ainley et al. (1993) Plant Mol Biol 22: 13-23), light (e.g., the pea rbcS-3A promoter, Kuhlemeier et al. (1989) Plant Cell 1:471, and the maize rbcS promoter, Schaffner and Sheen (1991) Plant Cell 3: 997); wounding (e.g., *wunI,* Siebertz et al. (1989) Plant Cell 1: 961); pathogens (such as the PR-1 promoter described in Buchel et al. (1999) Plant Mol. Biol. 40:387-396, and the PDF1.2 promoter described in Manners et al. (1998) Plant Mol. Biol. 38:1071-80), and chemicals such as methyl jasmonate or salicylic acid (Gatz et al. (1997) Plant Mol Biol 48: 89-108). In addition, the timing of the expression can be controlled by using promoters such as those acting at senescence (An and Amazon (1995) Science 270: 1986-1988); or late seed development (Odell et al. (1994) Plant Physiol 106:447-45 8).

Plant expression vectors can also include RNA processing signals that can be positioned within, upstream or downstream of the coding sequence. In addition, the expression vectors can include additional regulatory sequences from the 3'-untranslated region of plant genes, e.g., a 3' terminator region to increase mRNA stability of the mRNA, such as the PI-II terminator region of potato or the octopine or nopaline synthase 3' terminator regions.

### Additional Expression Elements

Specific initiation signals can aid in efficient translation of coding sequences. These signals can include, e.g., the ATG initiation codon and adjacent sequences. In cases where a coding sequence, its initiation codon and upstream sequences are inserted into the appropriate expression vector, no additional translational control signals may be needed. However, in cases where only coding sequence (e.g., a mature protein coding sequence), or a portion thereof, is inserted, exogenous transcriptional control signals including the ATG initiation codon can be separately provided. The initiation codon is provided in the correct reading frame to facilitate transcription. Exogenous transcriptional elements and initiation codons can be of various origins, both natural and synthetic. The efficiency of expression can be enhanced by the inclusion of enhancers appropriate to the cell system in use.

### Expression Hosts

The present invention also relates to host cells which are transduced with vectors of the invention, and the production of polypeptides of the invention (including fragments thereof) by recombinant techniques. Host cells are genetically engineered (i.e, nucleic acids are introduced, e.g., transduced, transformed or transfected) with the vectors of this invention, which may be, for example, a cloning vector or an expression vector comprising the relevant nucleic acids herein. The vector is optionally a plasmid, a viral particle, a phage, a naked nucleic acids, *etc.* The engineered host cells can be cultured in conventional nutrient media modified as appropriate for activating promoters, selecting transformants, or amplifying the relevant gene. The culture conditions, such as temperature, pH and the like, are those previously used with the host cell selected for expression, and will be apparent to those skilled in the art and in the references cited herein, including, Sambrook and Ausubel.

The host cell can be a eukaryotic cell, such as a yeast cell, or a plant cell, or the host cell can be a prokaryotic cell, such as a bacterial cell. Plant protoplasts are also suitable for some applications. For example, the DNA fragments are introduced into plant tissues, cultured plant cells or plant protoplasts by standard methods including electroporation (Fromm et al., (1985) Proc. Natl. Acad. Sci. USA 82, 5824, infection by viral vectors such as cauliflower mosaic virus (CaMV) (Hohn et al., (1982) Molecular Biology of Plant Tumors, (Academic Press, New York) pp. 549-560; US 4,407,956), high velocity ballistic penetration by small particles with the nucleic acid either within the matrix of small beads or particles, or on the surface (Klein et al., (1987) Nature 327, 70-73), use of pollen as vector (WO 85/01856), or use of *Agrobacterium* *tumefaciens* or *A. rhizogenes* carrying a T-DNA plasmid in which DNA fragments are cloned. The T-DNA plasmid is transmitted to plant cells upon infection by *Agrobacterium tumefaciens,* and a portion is stably integrated into the plant genome (Horsch et al. (1984) Science 233:496-498; Fraley et al. (1983) Proc. Natl. Acad. Sci. USA 80, 4803).

The cell can include a nucleic acid of the invention which encodes a polypeptide, wherein the cells expresses a polypeptide of the invention. The cell can also include vector sequences, or the like. Furthermore, cells and transgenic plants which include any polypeptide or nucleic acid above or throughout this specification, e.g., produced by transduction of a vector of the invention, are an additional feature of the invention.

For long-term, high-yield production of recombinant proteins, stable expression can be used. Host cells transformed with a nucleotide sequence encoding a polypeptide of the invention are optionally cultured under conditions suitable for the expression and recovery of the encoded protein from cell culture. The protein or fragment thereof produced by a recombinant cell may be secreted, membrane-bound, or contained intracellularly, depending on the sequence and/or the vector used. As will be understood by those of skill in the art, expression vectors containing polynucleotides encoding mature proteins of the invention can be designed with signal sequences which direct secretion of the mature polypeptides through a prokaryotic or eukaryotic cell membrane.

### Modified Amino Acids

Polypeptides of the invention may contain one or more modified amino acids. The presence of modified amino acids may be advantageous in, for example, increasing polypeptide half-life, reducing polypeptide antigenicity or toxicity, increasing polypeptide storage stability, or the like. Amino acid(s) are modified, for example, co-translationally or post-translationally during recombinant production or modified by synthetic or chemical means.

Non-limiting examples of a modified amino acid include incorporation or other use of acetylated amino acids, glycosylated amino acids, sulfated amino acids, prenylated (e.g., farnesylated, geranylgeranylated) amino acids, PEG modified (e.g., "PEGylated") amino acids, biotinylated amino acids, carboxylated amino acids, phosphorylated amino acids, etc. References adequate to guide one of skill in the modification of amino acids are replete throughout the literature.

### IDENTIFICATION OF ADDITIONAL FACTORS

A transcription factor provided by the present invention can also be used to identify additional endogenous or exogenous molecules that can affect a phentoype or trait of interest. On the one hand, such molecules include organic (small or large molecules) and/or inorganic compounds that affect expression of (i.e., regulate) a particular transcription factor. Alternatively, such molecules include endogenous molecules that are acted upon either at a transcriptional level by a transcription factor of the invention to modify a phenotype as desired. For example, the transcription factors can be employed to identify one or more downstream gene with which is subject to a regulatory effect of the transcription factor. In one approach, a transcription factor or transcription factor homologue of the invention is expressed in a host cell, e.g, a transgenic plant cell, tissue or explant, and expression products, either RNA or protein, of likely or random targets are monitored, e.g., by hybridization to a microarray of nucleic acid probes corresponding to genes expressed in a tissue or cell type of interest, by two-dimensional gel electrophoresis of protein products, or by any other method known in the art for assessing expression of gene products at the level of RNA or protein. Alternatively, a transcription factor of the invention can be used to identify promoter sequences (i.e., binding sites) involved in the regulation of a downstream target. After identifying a promoter sequence, interactions between the transcription factor and the promoter sequence can be modified by changing specific nucleotides in the promoter sequence or specific amino acids in the transcription factor that interact with the promoter sequence to alter a plant trait. Typically, transcription factor DNA binding sites are identified by gel shift assays. After identifying the promoter regions, the promoter region sequences can be employed in double-stranded DNA arrays to identify molecules that affect the interactions of the transcription factors with their promoters (Bulyk et al. (1999) Nature Biotechnology 17:573-577).

The identified transcription factors are also useful to identify proteins that modify the activity of the transcription factor. Such modification can occur by covalent modification, such as by phosphorylation, or by protein-protein (homo or-heteropolymer) interactions. Any method suitable for detecting protein-protein interactions can be employed. Among the methods that can be employed are co-immunoprecipitation, cross-linking and co-purification through gradients or chromatographic columns, and the two-hybrid yeast system.

The two-hybrid system detects protein interactions in vivo and is described in Chien, et al., (1991), Proc. Natl. Acad. Sci. USA 88, 9578-9582 and is commercially available from Clontech (Palo Alto, Calif.). In such a system, plasmids are constructed that encode two hybrid proteins: one consists of the DNA-binding domain of a transcription activator protein fused to the TF polypeptide and the other consists of the transcription activator protein's activation domain fused to an unknown protein that is encoded by a cDNA that has been recombined into the plasmid as part of a cDNA library. The DNA-binding domain fusion plasmid and the cDNA library are transformed into a strain of the yeast *Saccharomyces cerevisiae* that contains a reporter gene (e.g., lacZ) whose regulatory region contains the transcription activator's binding site. Either hybrid protein alone cannot activate transcription of the reporter gene. Interaction of the two hybrid proteins reconstitutes the functional activator protein and results in expression of the reporter gene, which is detected by an assay for the reporter gene product. Then, the library plasmids responsible for reporter gene expression are isolated and sequenced to identify the proteins encoded by the library plasmids. After identifying proteins that interact with the transcription factors, assays for compounds that interfere with the TF protein-protein interactions can be preformed.

### IDENTIFICATION OF MODULATORS

In addition to the intracellular molecules described above, extracellular molecules that alter activity or expression of a transcription factor, either directly or indirectly, can be identified. For example, the methods can entail first placing a candidate molecule in contact with a plant or plant cell. The molecule can be introduced by topical administration, such as spraying or soaking of a plant, and then the molecule's effect on the expression or activity of the TF polypeptide or the expression of the polynucleotide monitored. Changes in the expression of the TF polypeptide can be monitored by use of polyclonal or monoclonal antibodies, gel electrophoresis or the like. Changes in the expression of the corresponding polynucleotide sequence can be detected by use of microarrays, Northerns, quantitative PCR, or any other technique for monitoring changes in mRNA expression. These techniques are exemplified in Ausubel et al. (eds) Current Protocols in Molecular Biology, John Wiley & Sons (1998). Such changes in the expression levels can be correlated with modified plant traits and thus identified molecules can be useful for soaking or spraying on fruit, vegetable and grain crops to modify traits in plants.

Essentially any available composition can be tested for modulatory activity of expression or activity of any nucleic acid or polypeptide herein. Thus, available libraries of compounds such as chemicals, polypeptides, nucleic acids and the like can be tested for modulatory activity. Often, potential modulator compounds can be dissolved in aqueous or organic (e.g., DMSO-based) solutions for easy delivery to the cell or plant of interest in which the activity of the modulator is to be tested. Optionally, the assays are designed to screen large modulator composition libraries by automating the assay steps and providing compounds from any convenient source to assays, which are typically run in parallel (e.g., in microtiter formats on microtiter plates in robotic assays).

In one embodiment, high throughput screening methods involve providing a combinatorial library containing a large number of potential compounds, (potential modulator compounds). Such "combinatorial chemical libraries" are then screened in one or more assays, as described herein, to identify those library members (particular chemical species or subclasses) that display a desired characteristic activity. The compounds thus identified can serve as target compounds.

A combinatorial chemical library can be, e.g., a collection of diverse chemical compounds generated by chemical synthesis or biological synthesis. For example, a combinatorial chemical library such as a polypeptide library is formed by combining a set of chemical building blocks (e.g., in one example, amino acids) in every possible way for a given compound length (i.e., the number of amino acids in a polypeptide compound of a set length). Exemplary libraries include peptide libraries, nucleic acid libraries, antibody libraries (see, e.g., Vaughn et al. (1996) Nature Biotechnology, 14(3):309-314 and PCT/CTS96/10287), carbohydrate libraries (see, e.g., Liang et al. Science (1996) 274:1520-1522 and U.S. Patent 5,593,853), peptide nucleic acid libraries (see, e.g., U.S. Patent 5,539,083), and small organic molecule libraries (see, e.g., benzodiazepines, Baum C&EN Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337) and the like.

Preparation and screening of combinatorial or other libraries is well known to those of skill in the art. Such combinatorial chemical libraries include, but are not limited to, peptide libraries (see, e.g., U.S. Patent 5,010,175, Furka, Int. J. Pept. Prot. Res. 37:487-193 (1991) and Houghton et al. Nature 354:84-88 (1991)). Other chemistries for generating chemical diversity libraries can also be used.

In addition, as noted, compound screening equipment for high-throughput screening is generally available, e.g., using any of a number of well known robotic systems that have also been developed for solution phase chemistries useful in assay systems. These systems include automated workstations including an automated synthesis apparatus and robotic systems utilizing robotic arms. Any of the above devices are suitable for use with the present invention, e.g., for high-throughput screening of potential modulators. The nature and implementation of modifications to these devices (if any) so that they can operate as discussed herein will be apparent to persons skilled in the relevant art.

Indeed, entire high throughput screening systems are commercially available. These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. Similarly, microfluidic implementations of screening are also commercially available.

The manufacturers of such systems provide detailed protocols the various high throughput. Thus, for example, Zymark Corp. provides technical bulletins describing screening systems for detecting the modulation of gene transcription, ligand binding, and the like. The integrated systems herein, in addition to providing for sequence alignment and, optionally, synthesis of relevant nucleic acids, can include such screening apparatus to identify modulators that have an effect on one or more polynucleotides or polypeptides according to the present invention.

In some assays it is desirable to have positive controls to ensure that the components of the assays are working properly. At least two types of positive controls are appropriate. That is, known transcriptional activators or inhibitors can be incubated with cells/plants/ etc. in one sample of the assay, and the resulting increase/decrease in transcription can be detected by measuring the resulting increase in RNA/ protein expression, etc., according to the methods herein. It will be appreciated that modulators can also be combined with transcriptional activators or inhibitors to find modulators which inhibit transcriptional activation or transcriptional repression. Either expression of the nucleic acids and proteins herein or any additional nucleic acids or proteins activated by the nucleic acids or proteins herein, or both, can be monitored.

In an embodiment, the invention provides a method for identifying compositions that modulate the activity or expression of a polynucleotide or polypeptide of the invention. For example, a test compound, whether a small or large molecule, is placed in contact with a cell, plant (or plant tissue or explant), or composition comprising the polynucleotide or polypeptide of interest and a resulting effect on the cell, plant, (or tissue or explant) or composition is evaluated by monitoring, either directly or indirectly, one or more of: expression level of the polynucleotide or polypeptide, activity (or modulation of the activity) of the polynucleotide or polypeptide. In some cases, an alteration in a plant phenotype can be detected following contact of a plant (or plant cell, or tissue or explant) with the putative modulator, e.g., by modulation of expression or activity of a polynucleotide or polypeptide of the invention.

### SUBSEQUENCES

Also contemplated are uses of polynucleotides, also referred to herein as oligonucleotides, typically having at least 12 bases, preferably at least 15, more preferably at least 20, 30, or 50 bases, which hybridize under at least highly stringent (or ultra-high stringent or ultra-ultra- high stringent conditions) conditions to a polynucleotide sequence described above. The polynucleotides may be used as probes, primers, sense and antisense agents, and the like, according to methods as noted *supra.*

Subsequences of the polynucleotides of the invention, including polynucleotide fragments and oligonucleotides are useful as nucleic acid probes and primers. An oligonucleotide suitable for use as a probe or primer is at least about 15 nucleotides in length, more often at least about 18 nucleotides, often at least about 21 nucleotides, frequently at least about 30 nucleotides, or about 40 nucleotides, or more in length. A nucleic acid probe is useful in hybridization protocols, e.g., to identify additional polypeptide homologues of the invention, including protocols for microarray experiments. Primers can be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR) or other nucleic-acid amplification methods. See Sambrook and Ausubel, *supra.*

In addition, the invention includes an isolated or recombinant polypeptide including a subsequence of at least about 15 contiguous amino acids encoded by the recombinant or isolated polynucleotides of the invention. For example, such polypeptides, or domains or fragments thereof, can be used as immunogens, e.g., to produce antibodies specific for the polypeptide sequence, or as probes for detecting a sequence of interest. A subsequence can range in size from about 15 amino acids in length up to and including the full length of the polypeptide.

### PRODUCTION OF TRANSGENIC PLANTS

### Modification of Traits

The polynucleotides of the invention are favorably employed to produce transgenic plants with various traits, or characteristics, that have been modified in a desirable manner, e.g., to improve the seed characteristics of a plant. For example, alteration of expression levels or patterns (e.g., spatial or temporal expression patterns) of one or more of the transcription factors (or transcription factor homologues) of the invention, as compared with the levels of the same protein found in a wild type plant, can be used to modify a plant's traits. An illustrative example of trait modification, improved sugar-sensing characteristics, by altering expression levels of a particular transcription factor is described further in the Examples and the Sequence Listing.

### Antisense and Cosuoppression Approaches

In addition to expression of the nucleic acids of the invention as gene replacement or plant phenotype modification nucleic acids, the nucleic acids are also useful for sense and anti-sense suppression of expression, e.g., to down-regulate expression of a nucleic acid of the invention, e.g., as a further mechanism for modulating plant phenotype. That is, the nucleic acids of the invention, or subsequences or anti-sense sequences thereof, can be used to block expression of naturally occurring homologous nucleic acids. A variety of sense and anti-sense technologies are known in the art, e.g., as set forth in Lichtenstein and Nellen (1997) Antisense Technology: A Practical Approach IRL Press at Oxford University, Oxford, England. In general, sense or anti-sense sequences are introduced into a cell, where they are optionally amplified, e.g., by transcription. Such sequences include both simple oligonucleotide sequences and catalytic sequences such as ribozymes.

For example, a reduction or elimination of expression (i.e., a "knock-out") of a transcription factor or transcription factor homologue polypeptide in a transgenic plant, e.g., to modify a plant trait, can be obtained by introducing an antisense construct corresponding to the polypeptide of interest as a cDNA. For antisense suppression, the transcription factor or homologue cDNA is arranged in reverse orientation (with respect to the coding sequence) relative to the promoter sequence in the expression vector. The introduced sequence need not be the full length cDNA or gene, and need not be identical to the cDNA or gene found in the plant type to be transformed. Typically, the antisense sequence need only be capable of hybridizing to the target gene or RNA of interest. Thus, where the introduced sequence is of shorter length, a higher degree of homology to the endogenous transcription factor sequence will be needed for effective antisense suppression. While antisense sequences of various lengths can be utilized, preferably, the introduced antisense sequence in the vector will be at least 30 nucleotides in length, and improved antisense suppression will typically be observed as the length of the antisense sequence increases. Preferably, the length of the antisense sequence in the vector will be greater than 100 nucleotides. Transcription of an antisense construct as described results in the production of RNA molecules that are the reverse complement of mRNA molecules transcribed from the endogenous transcription factor gene in the plant cell.

Suppression of endogenous transcription factor gene expression can also be achieved using a ribozyme. Ribozymes are RNA molecules that possess highly specific endoribonuclease activity. The production and use of ribozymes are disclosed in U.S. Patent No. 4,987,071 and U.S. Patent No. 5,543,508. Synthetic ribozyme sequences including antisense RNAs can be used to confer RNA cleaving activity on the antisense RNA, such that endogenous mRNA molecules that hybridize to the antisense RNA are cleaved, which in turn leads to an enhanced antisense inhibition of endogenous gene expression.

Vectors in which RNA encoded by a transcription factor or transcription factor homologue cDNA is over-expressed can also be used to obtain co-suppression of a corresponding endogenous gene, e.g., in the manner described in U.S. Patent No. 5,231,020 to Jorgensen. Such co-suppression (also termed sense suppression) does not require that the entire transcription factor cDNA be introduced into the plant cells, nor does it require that the introduced sequence be exactly identical to the endogenous transcription factor gene of interest. However, as with antisense suppression, the suppressive efficiency will be enhanced as specificity of hybridization is increased, e.g., as the introduced sequence is lengthened, and/or as the sequence similarity between the introduced sequence and the endogenous transcription factor gene is increased.

Vectors expressing an untranslatable form of the transcription factor mRNA, e.g., sequences comprising one or more stop codon, or nonsense mutation) can also be used to suppress expression of an endogenous transcription factor, thereby reducing or eliminating it's activity and modifying one or more traits. Methods for producing such constructs are described in U.S. Patent No. 5,583,021. Preferably, such constructs are made by introducing a premature stop codon into the transcription factor gene. Alternatively, a plant trait can be modified by gene silencing using double-strand RNA (Sharp (1999) Genes and Development 13: 139-141).

Another method for abolishing the expression of a gene is by insertion mutagenesis using the T-DNA of *Agrobacterium tumefaciens.* After generating the insertion mutants, the mutants can be screened to identify those containing the insertion in a transcription factor or transcription factor homologue gene. Plants containing a single transgene insertion event at the desired gene can be crossed to generate homozygous plants for the mutation (Koncz et al. (1992) Methods in Arabidopsis Research, World Scientific).

Alternatively, a plant phenotype can be altered by eliminating an endogenous gene, such as a transcription factor or transcription factor homologue, e.g., by homologous recombination (Kempin et al. (1997) Nature 389:802).

A plant trait can also be modified by using the cre-lox system (for example, as described in US Pat. No. 5,658,772). A plant genome can be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides of this invention can also be expressed in a plant in the absence of an expression cassette by manipulating the activity or expression level of the endogenous gene by other means. For example, by ectopically expressing a gene by T-DNA activation tagging (Ichikawa et al. (1997) Nature 390 698-701; Kakimoto et al. (1996) Science 274: 982-985). This method entails transforming a plant with a gene tag containing multiple transcriptional enhancers and once the tag has inserted into the genome, expression of a flanking gene coding sequence becomes deregulated. In another example, the transcriptional machinery in a plant can be modified so as to increase transcription levels of a polynucleotide of the invention (*See,* e.g., PCT Publications WO 96/06166 and WO 98/53057 which describe the modification of the DNA binding specificity of zinc finger proteins by changing particular amino acids in the DNA binding motif).

The transgenic plant can also include the machinery necessary for expressing or altering the activity of a polypeptide encoded by an endogenous gene, for example by altering the phosphorylation state of the polypeptide to maintain it in an activated state.

Transgenic plants (or plant cells, or plant explants, or plant tissues) incorporating the polynucleotides of the invention and/or expressing the polypeptides of the invention can be produced by a variety of well established techniques as described above. Following construction of a vector, most typically an expression cassette, including a polynucleotide, e.g., encoding a transcription factor or transcription factor homologue, of the invention, standard techniques can be used to introduce the polynucleotide into a plant, a plant cell, a plant explant or a plant tissue of interest. Optionally, the plant cell, explant or tissue can be regenerated to produce a transgenic plant.

The plant can be any higher plant, including gymnosperms, monocotyledonous and dicotyledenous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melons and cucumber), *Gramineae* (wheat, com, rice, barley, millet, etc.), *Solanaceae* (potato, tomato, tobacco, peppers, etc.), and various other crops. See protocols described in Ammirato et al. (1984) Handbook of Plant Cell Culture -Crop Species. Macmillan Publ. Co. Shimamoto et al. (1989) Nature 338:274-276; Fromm et al. (1990) Bio/Technology 8:833-839; and Vasil et al. (1990) Bio/Technology 8:429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumeficiens* mediated transformation. Transformation means introducing a nucleotide sequence in a plant in a manner to cause stable or transient expression of the sequence.

Successful examples of the modification of plant characteristics by transformation with cloned sequences which serve to illustrate the current knowledge in this field of technology, and which are herein incorporated by reference, include: U.S. Patent Nos. 5,571,706; 5,677,175; 5,510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modified trait can be any of those traits described above. Additionally, to confirm that the modified trait is due to changes in expression levels or activity of the polypeptide or polynucleotide of the invention can be determined by analyzing mRNA expression using Northern blots, RT-PCR or microarrays, or protein expression using immunoblots or Western blots or gel shift assays.

### INTEGRATED SYSTEMS-SEQUENCE IDENTITY

Additionally, the present invention may be an integrated system, computer or computer readable medium that comprises an instruction set for determining the identity of one or more sequences in a database. In addition, the instruction set can be used to generate or identify sequences that meet any specified criteria. Furthermore, the instruction set may be used to associate or link certain functional benefits, such improved sugar-sensing characteristics, with one or more identified sequence.

For example, the instruction set can include, e.g., a sequence comparison or other alignment program, e.g., an available program such as, for example, the Wisconsin Package Version 10.0, such as BLAST, FASTA, PILEUP, FINDPATTERNS or the like (GCG, Madision, WI). Public sequence databases such as GenBank, EMBL, Swiss-Prot and PIR or private sequence databases such as PhytoSeq (Incyte Pharmaceuticals, Palo Alto, CA) can be searched.

Alignment of sequences for comparison can be conducted by the local homology algorithm of Smith and Waterman (1981) Adv. Appl. Math. 2:482, by the homology alignment algorithm ofNeedleman and Wunsch (1970) J. Mol. Biol. 48:443, by the search for similarity method of Pearson and Lipman (1988) Proc. Natl. Acad. Sci. U.S.A. 85: 2444, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window can be a segment of at least about 20 contiguous positions, usually about 50 to about 200, more usually about 100 to about 150 contiguous positions. A description of the method is provided in Ausubel et al., *supra.*

A variety of methods of determining sequence relationships can be used, including manual alignment and computer assisted sequence alignment and analysis. This later approach is a preferred approach in the present invention, due to the increased throughput afforded by computer assisted methods. As noted above, a variety of computer programs for performing sequence alignment are available, or can be produced by one of skill.

One example algorithm that is suitable for determining percent sequence identity and sequence similarity is the BLAST algorithm, which is described in Altschul et al. J. Mol. Biol 215:403-410 (1990). Software for performing BLAST analyses is publicly available, e.g., through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff (1989) Proc. Natl. Acad. Sci. USA 89:10915).

In addition to calculating percent sequence identity, the BLAST algorithm also performs a statistical analysis of the similarity between two sequences (*see,* e.g., Karlin & Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001. An additional example of a useful sequence alignment algorithm is PILEUP. PILEUP creates a multiple sequence alignment from a group of related sequences using progressive, pairwise alignments. The program can align, e.g., up to 300 sequences of a maximum length of 5,000 letters.

The integrated system, or computer typically includes a user input interface allowing a user to selectively view one or more sequence records corresponding to the one or more character strings, as well as an instruction set which aligns the one or more character strings with each other or with an additional character string to identify one or more region of sequence similarity. The system may include a link of one or more character strings with a particular phenotype or gene function. Typically, the system includes a user readable output element which displays an alignment produced by the alignment instruction set.

The methods of this invention can be implemented in a localized or distributed computing environment. In a distributed environment, the methods may implemented on a single computer comprising multiple processors or on a multiplicity of computers. The computers can be linked, e.g. through a common bus, but more preferably the computer(s) are nodes on a network. The network can be a generalized or a dedicated local or wide-area network and, in certain preferred embodiments, the computers may be components of an intra-net or an internet.

Thus, the invention provides methods for identifying a sequence similar or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an inter or intra net) and a query is made against the sequence database using the relevant sequences herein and associated plant phenotypes or gene functions.

Any sequence herein can be entered into the database, before or after querying the database. This provides for both expansion of the database and, if done before the querying step, for insertion of control sequences into the database. The control sequences can be detected by the query to ensure the general integrity of both the database and the query. As noted, the query can be performed using a web browser based interface. For example, the database can be a centralized public database such as those noted herein, and the querying can be done from a remote terminal or computer across an internet or intranet.

### EXAMPLES

The following examples are intended to illustrate but not limit the present invention.

### EXAMPLE I. FULL LENGTH GENE IDENTIFICATION AND CLONING

Putative transcription factor sequences (genomic or ESTs) related to known transcription factors were identified in the *Arabidopsis thaliana* GenBank database using the tblastn sequence analysis program using default parameters and a P-value cutoff threshold of -4 or -5 or lower, depending on the length of the query sequence. Putative transcription factor sequence hits were then screened to identify those containing particular sequence strings. If the sequence hits contained such sequence strings, the sequences were confirmed as transcription factors.

Alternatively, Arabidopsis *thaliana* cDNA libraries derived from different tissues or treatments, or genomic libraries were screened to identify novel members of a transcription family using a low stringency hybridization approach. Probes were synthesized using gene specific primers in a standard PCR reaction (annealing temperature 60° C) and labeled with ³²P dCTP using the High Prime DNA Labeling Kit (Boehringer Mannheim). Purified radiolabelled probes were added to filters immersed in Church hybridization medium (0.5 M NaPO₄ pH 7.0, 7% SDS, 1 % w/v bovine serum albumin) and hybridized overnight at 60 °C with shaking. Filters were washed two times for 45 to 60 minutes with 1xSCC, 1% SDS at 60° C.

To identify additional sequence 5' or 3' of a partial cDNA sequence in a cDNA library, 5' and 3' rapid amplification of cDNA ends (RACE) was performed using the Marathon™ cDNA amplification kit (Clontech, Palo Alto, CA). Generally, the method entailed first isolating poly(A) mRNA, performing first and second strand cDNA synthesis to generate double stranded cDNA, blunting cDNA ends, followed by ligation of the Marathon™ Adaptor to the cDNA to form a library of adaptor-ligated ds cDNA.

Gene-specific primers were designed to be used along with adaptor specific primers for both 5' and 3' RACE reactions. Nested primers, rather than single primers, were used to increase PCR specificity. Using 5' and 3' RACE reactions, 5' and 3' RACE fragments were obtained, sequenced and cloned. The process can be repeated until 5' and 3' ends of the full-length gene were identified. Then the full-length cDNA was generated by PCR using primers specific to 5' and 3' ends of the gene by end-to-end PCR.

### EXAMPLE II. CONSTRUCTION OF EXPRESSION VECTORS

The sequence was amplified from a genomic or cDNA library using primers specific to sequences upstream and downstream of the coding region. The expression vector was pMEN20 or pMEN65, which are both derived from pMON316 (Sanders et al, (1987) Nucleic Acids Research 15:1543-58) and contain the CaMV 35S promoter to express transgenes. To clone the sequence into the vector, both pMEN20 and the amplified DNA fragment were digested separately with SalI and NotI restriction enzymes at 37° C for 2 hours. The digestion products were subject to electrophoresis in a 0.8% agarose gel and visualized by ethidium bromide. staining. The DNA fragments containing the sequence and the linearized plasmid were excised and purified by using a Qiaquick gel extraction kit (Qiagen, CA). The fragments of interest were ligated at a ratio of 3:1 (vector to insert). Ligation reactions using T4 DNA ligase (New England Biolabs, MA) were carried out at 16° C for 16 hours. The ligated DNAs were transformed into competent cells of the *E. coli* strain DH5alpha by using the heat shock method. The transformations were plated on LB plates containing 50 mg/l kanamycin (Sigma).

Individual colonies were grown overnight in five milliliters of LB broth containing 50 mg/l kanamycin at 37° C. Plasmid DNA was purified by using Qiaquick Mini Prep kits (Qiagen, CA).

### EXAMPLE III. TRANSFORMATION OF AGROBACTERIUM WITH THE EXPRESSION VECTOR

After the plasmid vector containing the gene was constructed, the vector was used to transform *Agrobacterium tumefaciens* cells expressing the gene products. The stock of *Agrobacterium tumefaciens* cells for transformation were made as described by Nagel et al. (1990) FEMS Microbiol Letts. 67: 325-328. *Agrobacterium* strain ABI was grown in 250 ml LB medium (Sigma) overnight at 28°C with shaking until an absorbance (A₆₀₀) of 0.5 - 1.0 was reached. Cells were harvested by centrifugation at 4,000 x g for 15 min at 4° C. Cells were then resuspended in 250 µl chilled buffer (1 mM HEPES, pH adjusted to 7.0 with KOH). Cells were centrifuged again as described above and resuspended in 125 µl chilled buffer. Cells were then centrifuged and resuspended two more times in the same HEPES buffer as described above at a volume of 100 µl and 750 µl, respectively. Resuspended cells were then distributed into 40 µl aliquots, quickly frozen in liquid nitrogen, and stored at -80° C.

*Agrobacterium* cells were transformed with plasmids prepared as described above following the protocol described by Nagel et al. For each DNA construct to be transformed, 50 - 100 ng DNA (generally resuspended in 10 mM Tris-HCl, 1 mM EDTA, pH 8.0) was mixed with 40 µl of *Agrobacterium* cells. The DNA/cell mixture was then transferred to a chilled cuvette with a 2mm electrode gap and subject to a 2.5 kV charge dissipated at 25 µF and 200 µF using a Gene Pulser II apparatus (Bio-Rad). After electroporation, cells were immediately resuspended in 1.0 ml LB and allowed to recover without antibiotic selection for 2 - 4 hours at 28° C in a shaking incubator. After recovery, cells were plated onto selective medium of LB broth containing 100 µg/ml spectinomycin (Sigma) and incubated for 24-48 hours at 28° C. Single colonies were then picked and inoculated in fresh medium. The presence of the plasmid construct was verified by PCR amplification and sequence analysis.

### EXAMPLE IV. TRANSFORMATION OF ARABIDOPSIS PLANTS WITH AGROBACTERIUM TUMEFACIENS WITH EXPRESSION VECTOR

After transformation of *Agrobacterium tumefaciens* with plasmid vectors containing the gene, single *Agrobacterium* colonies were identified, propagated, and used to transform *Arabidopsis* plants. Briefly, 500 ml cultures of LB medium containing 50 mg/l kanamycin were inoculated with the colonies and grown at 28° C with shaking for 2 days until an absorbance (A₆₀₀) of > 2.0 is reached. Cells were then harvested by centrifugation at 4,000 x g for 10 min, and resuspended in infiltration medium (1/2 X Murashige and Skoog salts (Sigma), 1 X Gamborg's B-5 vitamins (Sigma), 5.0% (w/v) sucrose (Sigma), 0.044 µM benzylamino purine (Sigma), 200 µl/L Silwet L-77 (Lehle Seeds) until an absorbance (A₆₀₀) of 0.8 was reached.

Prior to transformation, *Arabidopsis thaliana* seeds (ecotype Columbia) were sown at a density of~10 plants per 4" pot onto Pro-Mix BX potting medium (Hummert International) covered with fiberglass mesh (18 mm X 16 mm). Plants were grown under continuous illumination (50-75 µE/m²/sec) at 22-23° C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a 1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

### EXAMPLE V. IDENTIFICATION OF ARABIDOPSIS PRIMARY TRANSFORMANTS

Seeds collected from the transformation pots were sterilized essentially as follows. Seeds were dispersed into in a solution containing 0.1% (v/v) Triton X-100 (Sigma) and sterile H₂O and washed by shaking the suspension for 20 min. The wash solution was then drained and replaced with fresh wash solution to wash the seeds for 20 min with shaking. After removal of the second wash solution, a solution containing 0.1% (v/v) Triton X-100 and 70% ethanol (Equistar) was added to the seeds and the suspension was shaken for 5 min. After removal of the ethanol/detergent solution, a solution containing 0.1% (v/v) Triton X-100 and 30% (v/v) bleach (Clorox) was added to the seeds, and the suspension was shaken for 10 min. After removal of the bleach/detergent solution, seeds were then washed five times in sterile distilled H₂O. The seeds were stored in the last wash water at 4° C for 2 days in the dark before being plated onto antibiotic selection medium (1 X Murashige and Skoog salts (pH adjusted to 5.7 with 1M KOH), 1 X Gamborg's B-5 vitamins, 0.9% phytagar (Life Technologies), and 50 mg/l kanamycin). Seeds were germinated under continuous illumination (50-75 µE/m²/sec) at 22-23° C. After 7-10 days of growth under these conditions, kanamycin resistant primary transformants (T₁ generation) were visible and obtained. These seedlings were transferred first to fresh selection plates where the seedlings continued to grow for 3-5 more days, and then to soil (Pro-Mix BX potting medium).

Primary transformants were crossed and progeny seeds (T₂) collected; kanamycin resistant seedlings were selected and analyzed. The expression levels of the recombinant polynucleotides in the transformants varies from about a 5% expression level increase to a least a 100% expression level increase. Similar observations are made with respect to polypeptide level expression.

### EXAMPLE VI. IDENTIFICATION OF ARABIDOPSIS PLANTS WITH TRANSCRIPTION FACTOR GENE KNOCKOUTS

The screening of insertion mutagenized *Arabidopsis* collections for null mutants in a known target gene was essentially as described in Krysan et al (1999) Plant Cell 11:2283-2290. Briefly, gene-specific primers, nested by 5-250 base pairs to each other, were designed from the 5' and 3' regions of a known target gene. Similarly, nested sets of primers were also created specific to each of the T-DNA or transposon ends (the "right" and "left" borders). All possible combinations of gene specific and T-DNA/transposon primers were used to detect by PCR an insertion event within or close to the target gene. The amplified DNA fragments were then sequenced which allows the precise determination of the T-DNA/transposon insertion point relative to the target gene. Insertion events within the coding or intervening sequence of the genes were deconvoluted from a pool comprising a plurality of insertion events to a single unique mutant plant for functional characterization. The method is described in more detail in Yu and Adam, US Application Serial No. 09/177,733 filed October 23, 1998.

### EXAMPLE VII. IDENTIFICATION OF SUGAR-SENSING CHARACTERISTICS PHENOTYPE IN OVEREXPRESSOR OR GENE KNOCKOUT PLANTS

Experiments were performed to identify those transformants or knockouts that exhibited modified sugar-sensing . For such studies, seeds from transformants were germinated on media containing 5% glucose or 9.4% sucrose which normally partially restrict hypocotyl elongation. Plants with altered sugar sensing may have either longer or shorter hypocotyls than normal plants when grown on this media. Additionally, other plant traits may be varied such as root mass.

Table 3 shows the phenotypes observed for particular overexpressor or knockout plants and provides the SEQ ID No., the internal reference code (GID), whether a knockout or overexpressor plant was analyzed and the observed phenotype.

**Table 3**

| SEQ ID No. | GID | Knockout (OE) or overexpressor KO) | Phenotype observed |
|---|---|---|---|
| 1 | G26 | OE | Decreased germination and growth on glucose medium |
| 3 | G38 | OE | Reduced germination on glucose medium |
| 5 | G43 | OE | Decreased germination and growth on glucose medium |
| 7 | G207 | OE | Decreased germination on glucose medium |
| 9 | G241 | OE | Decreased germination and growth on glucose medium |
| 11 | G254 | OE | Decreased germination and growth on glucose medium |
| 13 | G263 | OE | Decreased root growth on sucrose medium |
| 15 | G308 | OE | No germination on glucose medium |
| 17 | G536 | OE | Decreased germination and growth on glucose medium |
| 19 | G680 | OE | Reduced germination on glucose medium |
| 21 | G867 | OE | Better seedling vigor on sucrose medium |
| 23 | G912 | OE | Reduced cotyledon expansion in glucose |
| 25 | G996 | OE | Reduced germination on glucose medium |
| 27 | G1068 | OE | Reduced cotyledon expansion in glucose |
| 29 | G1337 | OE | Decreased germination on sucrose medium |

For a particular overexpressor that shows a less beneficial sugar-sensing characteristic, it may be more useful to select a plant with a decreased expression of the particular transcription factor. For a particular knockout that shows a less beneficial sugar-sensing characteristic, it may be more useful to select a plant with an increased expression of the particular transcription factor.

### EXAMPLE VIII. IDENTIFICATION OF HOMOLOGOUS SEQUENCES

Homologous sequences from *Arabidopsis* and plant species other than *Arabidopsis* were identified using database sequence search tools, such as the Basic Local Alignment Search Tool (BLAST) (Altschul et al. (1990) J. Mol. Biol. 215:403-410; and Altschul et al. (1997) Nucl. Acid Res. 25: 3389-3402). The tblastx sequence analysis programs were employed using the BLOSUM-62 scoring matrix (Henikoff, S. and Henikoff, J. G. (1992) Proc. Natl. Acad. Sci. USA 89: 10915-10919).

Identified *Arabidopsis* homologous sequences are provided in Figure 2 and included in the Sequence Listing. The percent sequence identity among these sequences is as low as 47% sequence identity. Additionally, the entire NCBI GenBank database was filtered for sequences from all plants except *Arabidopsis thaliana* by selecting all entries in the NCBI GenBank database associated with NCBI taxonomic ID 33090 (Viridiplantae; all plants) and excluding entries associated with taxonomic ID 3701 (*Arabidopsis thaliana*)*.* These sequences were compared to sequences representing genes of SEQ IDs Nos. 1-54 on 9/26/2000 using the Washington University TBLASTX algorithm (version 2.0a19MP). For each gene of SEQ IDs Nos. 1-54, individual comparisons were ordered by probability score (P-value), where the score reflects the probability that a particular alignment occurred by chance. For example, a score of 3.6e-40 is 3.6 x 10⁻⁴⁰. For up to ten species, the gene with the lowest P-value (and therefore the most likely homolog) is listed in Figure 3.

In addition to P-values, comparisons were also scored by percentage identity. Percentage identity reflects the degree to which two segments of DNA or protein are identical over a particular length. The ranges of percent identity between the non-Arabidopsis genes shown in Figure 3 and the Arabidopsis genes in the sequence listing are: SEQ ID No. 1: 44%-79%; SEQ ID No. 3: 36%-72%; SEQ ID No. 5: 42%-67%; SEQ ID No. 7: 55%-82%; SEQ ID No. 9: 69%-84%; SEQ ID No. 11: 57%-90%; SEQ ID No. 13: 48%-85%; SEQ ID No. 15: 38%-85%; SEQ ID No. 17: 77%-87%; SEQ ID No. 19: 42%-88%; SEQ ID No. 21: 54%-69%; SEQ ID No. 23: 34%-71%; SEQ ID No. 25: 55%-95%; SEQ ID No. 27: 54%-95%; SEQ ID No. 29: 37%-58%; SEQ ID No. 31: 42%-70%; SEQ ID No. 33: 46%-62%; SEQ ID No. 35: 64%-84%; SEQ ID No. 37: 57%-87%; SEQ ID No. 39: 40%-80%; SEQ ID No. 41: 56%-82%; SEQ ID No. 43: 64%-93%; SEQ ID No. 45: 35%-86%; SEQ ID No. 47: 84%-91%; SEQ ID No. 49: 85%-91%; SEQ ID No. 51: 38%-89%; SEQ ID No. 53: 53%-75%; SEQ ID No. 55: 57%-72%; SEQ ID No. 57: 57%-69%; SEQ ID No. 59: 49%-86%; SEQ ID No. 61: 49%-78%; SEQ ID No. 63: 51%-86%; SEQ ID No. 65: 42%-72%; SEQ ID No. 67: 35%-69%; and SEQ ID No. 69: 36%-64%.

The polynucleotides and polypeptides in the Sequence Listing and the identified homologous sequences may be stored in a computer system and have associated or linked with the sequences a function, such as that the polynucleotides and polypeptides are useful for modifying the sugar-sensing characteristics of a plant.

All references, publications, patents and other documents herein are incorporated by reference in their entirety for all purposes. Although the invention has been described with reference to the embodiments and examples above, it should be understood that various modifications can be made without departing from the spirit of the invention.

A transgenic plant with modified sugar-sensing characteristics, which plant comprises a recombinant polynucleotide comprising a nucleotide sequence selected from the group consisting of:
(a) a nucleotide sequence encoding a polypeptide comprising a sequence selected from SEQ ID Nos. 2N, where N=1-35, or a complementary nucleotide sequence thereof;
(b) a nucleotide sequence encoding a polypeptide comprising a conservatively substituted variant of a polypeptide of (a);
(c) a nucleotide sequence comprising a sequence selected from those of SEQ ID Nos. 2N-1, where N=1-35, or a complementary nucleotide sequence thereof;
(d) a nucleotide sequence comprising silent substitutions in a nucleotide sequence of (c);
(e) a nucleotide sequence which hybridizes under stringent conditions to a nucleotide sequence of one or more of: (a), (b), (c), or (d);
(f) a nucleotide sequence comprising at least 15 consecutive nucleotides of a sequence of any of (a)-(e);
(g) a nucleotide sequence comprising a subsequence or fragment of any of (a)-(f), which subsequence or fragment encodes a polypeptide that modifies a plant's sugar-sensing characteristics;
(h) a nucleotide sequence having at least 34% sequence identity to a nucleotide sequence of any of (a)-(g);
(i) a nucleotide sequence having at least 60% identity sequence identity to a nucleotide sequence of any of (a)-(g);
(j) a nucleotide sequence which encodes polypeptide having at least 34% identity sequence identity to a polypeptide of SEQ ID Nos. 2N, where N=1-35;
(k) a nucleotide sequence which encodes a polypeptide having at least 60% identity sequence identity to a polypeptide of SEQ ID Nos. 2N, where N=1-35; and
(I) a nucleotide sequence which encodes a polypeptide having at least 65% sequence identity to a conserved domain of a polypeptide of SEQ ID Nos. 2N, where N=1-35.

The transgenic plant as defined above, further comprising a constitutive, inducible, or tissue-active promoter operably linked to said nucleotide sequence.

The transgenic plant as defined above, wherein the plant is selected from the group consisting of: soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet com, tobacco, tomato, watermelon, rosaceous fruits, and vegetable brassicas.

An isolated or recombinant polynucleotide comprising a nucleotide sequence selected from the group consisting of:
(a) a nucleotide sequence encoding a polypeptide comprising a sequence selected from SEQ ID Nos. 2N, where N=1-35, or a complementary nucleotide sequence thereof;
(b) a nucleotide sequence encoding a polypeptide comprising a conservatively substituted variant of a polypeptide of (a);
(c) a nucleotide sequence comprising a sequence selected from those of SEQ ID Nos. 2N-1, where N=1-35, or a complementary nucleotide sequence thereof;
(d) a nucleotide sequence comprising silent substitutions in a nucleotide sequence of (c);
(e) a nucleotide sequence which hybridizes under stringent conditions to a nucleotide sequence of one or more of: (a), (b), (c), or (d);
(f) a nucleotide sequence comprising at least 15 consecutive nucleotides of a sequence of any of (a)-(e);
(g) a nucleotide sequence comprising a subsequence or fragment of any of (a)-(f), which subsequence or fragment encodes a polypeptide that modifies a plant's sugar-sensing characteristics;
(h) a nucleotide sequence having at least 34% sequence identity to a nucleotide sequence of any of (a)-(g);
(i) a nucleotide sequence having at least 60% identity sequence identity to a nucleotide sequence of any of (a)-(g);
(j) a nucleotide sequence which encodes a polypeptide having at least 34% identity sequence identity to a polypeptide of SEQ m Nos. 2N, where N=1-35;
(k) a nucleotide sequence which encodes a polypeptide having at least 60% identity sequence identity to a polypeptide of SEQ ID Nos. 2N, where N=1-35; and
(l) a nucleotide sequence which encodes a conserved domain of a polypeptide having at least 65% sequence identity to a conserved domain of a polypeptide of SEQ ID Nos. 2N, where N=1-35.

The isolated or recombinant polynucleotide as defined above, further comprising a constitutive, inducible, or tissue-active promoter operably linked to the nucleotide sequence.

A cloning or expression vector comprising the isolated or recombinant polynucleotide as defined above.

A cell comprising the cloning or expression vector as defined above .

A transgenic plant comprising the isolated or recombinant polynucleotide as defined above.

A composition produced by one or more of:
(a) incubating one or more polynucleotide as defined above with a nuclease;
(b) incubating one or more polynucleotide as defined above with a restriction enzyme;
(c) incubating one or more polynucleotide as defined above with a polymerase;
(d) incubating one or more polynucleotide as defined above with a polymerase and a primer;
(e) incubating one or more polynucleotide as defined above with a cloning vector, or
(f) incubating one or more polynucleotide as defined above with a cell.

A composition comprising two or more different polynucleotides a s defined above.

An isolated or recombinant polypeptide comprising a subsequence of at least about 15 contiguous amino acids encoded by the recombinant or isolated polynucleotide as defined above .

A plant ectopically expressing an isolated polypeptide as defined above.

A method for producing a plant having modified sugar-sensing characteristics, the method comprising altering the expression of the isolated or recombinant polynucleotide as defined above or the expression levels or activity of a polypeptide as defined above in a plant, thereby producing a modified plant, and selecting the modified plant for improved sugar-sensing characteristics thereby providing the modified plant with a modified sugar-sensing characteristics.

The method as defined above, wherein the polynucleotide is a polynucleotide as defined above.

A method of identifying a factor that is modulated by or interacts with a polypeptide encoded by a polynucleotide as defined above, the method comprising:
(a) expressing a polypeptide encoded by the polynucleotide in a plant; and
(b) identifying at least one factor that is modulated by or interacts with the polypeptide.

The method as defined above, wherein the identifying is performed by detecting binding by the polypeptide to a promoter sequence, or detecting interactions between an additional protein and the polypeptide in a yeast two hybrid system.

The method as defined above, wherein the identifying is performed by detecting expression of a factor by hybridization to a microarray, subtractive hybridization or differential display.

A method of identifying a molecule that modulates activity or expression of a polynucleotide or polypeptide of interest, the method comprising:
(a) placing the molecule in contact with a plant comprising the polynucleotide or polypeptide encoded by the polynucleotide as defined above; and,
(b) monitoring one or more of:
   (i) expression level of the polynucleotide in the plant;
   (ii) expression level of the polypeptide in the plant;
   (iii) modulation of an activity of the polypeptide in the plant; or
   (iv) modulation of an activity of the polynucleotide in the plant.

An integrated system, computer or computer readable medium comprising one or more character strings corresponding to a polynucleotide as defined above, or to a polypeptide encoded by the polynucleotide.

The integrated system, computer or computer readable medium as defined above, further comprising a link between said one or more sequence strings to a modified plant sugar-sensing characteristics phenotype.

A method of identifying a sequence similar or homologous to one or more polynucleotides as defined above, or one or more polypeptides encoded by the polynucleotides, the method comprising:
(a) providing a sequence database; and,
(b) querying the sequence database with one or more target sequences corresponding to the one or more polynucleotides or to the one or more polypeptides to identify one or more sequence members of the database that display sequence similarity or homology to one or more of the one or more target sequences.

The method as defined above, wherein the querying comprises aligning one or more of the target sequences with one or more of the one or more sequence members in the sequence database.

The method as defined above, wherein the querying comprises identifying one or more of the one or more sequence members of the database that meet a user-selected identity criteria with one or more of the target sequences.

The method as defined above, further comprising linking the one or more of the polynucleotides as defined above, or encoded polypeptides, to a modified plant sugar-sensing characteristics phenotype.

A plant comprising altered expression levels of an isolated or recombinant polynucleotide as defined above.

A plant comprising altered expression levels or the activity of an isolated or recombinant polypeptide as defined above.

## Claims

1. A transgenic plant with modified sugar-sensing characteristics, which plant comprises a recombinant polynucleotide comprising a nucleotide sequence selected from the group consisting of:
(a) a nucleotide sequence encoding a polypeptide comprising the sequence of SEQ ID No : 22, or a complementary nucleotide sequence thereof;
(b) a nucleotide sequence encoding a polypeptide comprising a conservatively substituted variant of a polypeptide of (a);
(c) a nucleotide sequence comprising the sequence of SEQ ID No: 21, or a complementary nucleotide sequence thereof;
(d) a nucleotide sequence comprising silent substitutions in a nucleotide sequence of (c);
(e) a nucleotide sequence which hybridizes under stringent conditions to a nucleotide sequence of one or more of: (a), (b), (c), or (d);
(f) a nucleotide sequence comprising at least 15 consecutive nucleotides of a sequence of any of (a)-(e);
(g) a nucleotide sequence comprising a subsequence or fragment of any of (a)-(f), which subsequence or fragment encodes a polypeptide that modifies a plant's sugar-sensing characteristics;
(h) a nucleotide sequence having at least 34% sequence identity to a nucleotide sequence of any of (a)-(g);
(i) a nucleotide sequence having at least 60% identity sequence identity to a nucleotide sequence of any of (a)-(g);
(j) a nucleotide sequence which encodes a polypeptide having at least 34% identity sequence identity to the polypepitde of SEQ ID No : 22;
(k) a nucleotide sequence which encodes a polypeptide having at least 60% identity sequence identity to the polypeptide of SEQ ID No : 22; and
(l) a nucleotide sequence which encodes a polypeptide having at least 65% sequence identity to a conserved domain of the polypeptide of SEQ ID No : 22.

2. The transgenic plant of claim 1, further comprising a constitutive, inducible, or tissue-active promoter operably linked to said nucleotide sequence.

3. The transgenic plant of claim 1, wherein the plant is selected from :
soybean, wheat, com, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

4. An isolated or recombinant polynucleotide comprising a nucleotide sequence selected from the group consisting of:
(a) a nucleotide sequence encoding a polypeptide comprising the sequence of SEQ ID No : 22, or a complementary nucleotide sequence thereof;
(b) a nucleotide sequence encoding a polypeptide comprising a conservatively substituted variant of a polypeptide of (a);
(c) a nucleotide sequence comprising the sequence of SEQ ID No: 21 , or a complementary nucleotide sequence thereof;
(d) a nucleotide sequence comprising silent substitutions in a nucleotide sequence of (c);
(e) a nucleotide sequence which hybridizes under stringent conditions to a nucleotide sequence of one or more of: (a), (b), (c), or (d);
(f) a nucleotide sequence comprising at least 15 consecutive nucleotides of a sequence of any of (a)-(e);
(g) a nucleotide sequence comprising a subsequence or fragment of any of(a)-(f), which subsequence or fragment encodes a polypeptide that modifies a plant's sugar-sensing characteristics;
(h) a nucleotide sequence having at least 34% sequence identity to a nucleotide sequence of any of (a)-(g);
(i) a nucleotide sequence having at least 60% identity sequence identity to a nucleotide sequence of any of (a)-(g);
(j) a nucleotide sequence which encodes a polypeptide having at least 34% identity sequence identity to the polypeptide of SEQ No : 22 ;
(k) a nucleotide sequence which encodes a polypeptide having at least 60% identity sequence identity to the polypeptide of SEQ ID No : 22 ; and
(l) a nucleotide sequence which encodes a polypeptide having at least 65% sequence identity to a conserved domain of the polypeptide of SEQ ED No : 22.

5. The isolated or recombinant polynucleotide of claim 4, further comprising a constitutive, inducible, or tissue-active promoter operably linked to the nucleotide sequence.

6. A cloning or expression vector comprising the isolated or recombinant polynucleotide of claim 4.

7. A cell comprising the cloning or expression vector of claim 6.

8. A transgenic plant comprising the isolated or recombinant polynucleotide of claim 4.

9. A composition produced by one or more of:
(a) incubating one or more polynucleotide of claim 4 with a nuclease;
(b) incubating one or more polynucleotide of claim 4 with a restriction enzyme;
(c) incubating one or more polynucleotide of claim 4 with a polymerase;
(d) incubating one or more polynucleotide of claim 4 with a polymerase and a primer;
(e) incubating one or more polynucleotide of claim 4 with a cloning vector, or
(f) incubating one or more polynucleotide of claim 4 with a cell.

10. A composition comprising two or more different polynucleotides of claim 4.

11. An isolated or recombinant polypeptide comprising a subsequence of at least about 15 contiguous amino acids encoded by the recombinant or isolated polynucleotide of claim 4.

12. A plant ectopically expressing an isolated polypeptide of claim 11.

13. A method for producing a plant having modified sugar-sensing characteristics, the method comprising altering the expression of the isolated or recombinant polynucleotide of claim 4 or the expression levels or activity of a polypeptide of claim 11 in a plant, thereby producing a modified plant, and selecting the modified plant for improved sugar-sensing characteristics thereby providing the modified plant with a modified sugar-sensing characteristics.

14. The method of claim 13, wherein the polynucleotide is a polynucleotide of claim 4.

15. A method of identifying a factor that is modulated by or interacts with a polypeptide encoded by a polynucleotide of claim 4, the method comprising:
(a) expressing a polypeptide encoded by the polynucleotide in a plant; and
(b) identifying at least one factor that is modulated by or interacts with the polypeptide.

16. The method of claim 15, wherein the identifying is performed by detecting binding by the polypeptide to a promoter sequence, or detecting interactions between an additional protein and the polypeptide in a yeast two hybrid system.

17. The method of claim 15, wherein the identifying is performed by detecting expression of a factor by hybridization to a microarray, subtractive hybridization or differential display.

18. A method of identifying a molecule that modulates activity or expression of a polynucleotide or polypeptide of interest, the method comprising:
(a) placing the molecule in contact with a plant comprising the polynucleotide or polypeptide encoded by the polynucleotide of claim 4; and,
(b) monitoring one or more of:
(i) expression level of the polynucleotide in the plant;
(ii) expression level of the polypeptide in the plant;
(iii) modulation of an activity of the polypeptide in the plant; or
(iv) modulation of an activity of the polynucleotide in the plant.

19. An integrated system, computer or computer readable medium comprising one or more character strings corresponding to a polynucleotide of claim 4, or to a polypeptide encoded by the polynucleotide.

20. The integrated system, computer or computer readable medium of claim 19, further comprising a link between said one or more sequence strings to a modified plant sugar-sensing characteristics phenotype.

21. A method of identifying a sequence similar or homologous to one or more polynucleotides of claim 4, or one or more polypeptides encoded by the polynucleotides, the method comprising:
(a) providing a sequence database; and,
(b) querying the sequence database with one or more target sequences corresponding to the one or more polynucleotides or to the one or more polypeptides to identify one or more sequence members of the database that display sequence similarity or homology to one or more of the one or more target sequences.

22. The method of claim 21, wherein the querying comprises aligning one or more of the target sequences with one or more of the one or more sequence members in the sequence database.

23. The method of claim 21, wherein the querying comprises identifying one or more of the one or more sequence members of the database that meet a user-selected identity criteria with one or more of the target sequences.

24. The method of claim 21, further comprising linking the one or more of the polynucleotides of claim 4, or encoded polypeptides, to a modified plant sugar-sensing characteristics phenotype.

25. A plant comprising altered expression levels of an isolated or recombinant polynucleotide of claim 4.

26. A plant comprising altered expression levels or the activity of an isolated or recombinant polypeptide of claim 11.
